# EUROPEAN PATENT APPLICATION

(11) **EP 3 269 816 A1**
(43) Date of publication of application: **17.01.2018**
(21) Application number: 16178896.3
(22) Date of filing: 11.07.2016
(51) Int. Cl.: C12N 15/82

(54) **DEVELOPMENT OF FUNGAL RESISTANT CROPS BY HIGS (HOST-INDUCED GENE SILENCING) MEDIATED INHIBITION OF GPI-ANCHORED CELL WALL PROTEIN SYNTHESIS**

(71) Applicant: KWS SAAT SE, 37574 Einbeck (DE)
(72) Inventor: Stahl, Dietmar, 37574 Einbeck (DE); Temme, Nora, 37574 Einbeck (DE); Deising, Holger, 06118 Halle (Saale) (DE); Garcia Oliveira, Ely, Manhatttan, KS 66502 (US); Schäfer, Wilhelm, 22589 Hamburg (DE)

(57) **Abstract**

The present invention is directed to a transgenic plant or a part thereof comprising a DNA capable of expressing an inhibitory nucleic acid molecule capable of inhibiting the expression of a gene involved in the glycosylphosphatidylinositol anchor biosynthesis pathway in a fungus, to such DNA, to the inhibitory nucleic acid molecule, to a vector comprising the DNA, to methods of producing the plant, of conferring fungal resistance to the plant, or of inhibiting the expression of a gene involved in the glycosylphosphatidylinositol anchor biosynthesis pathway, to the use of the DNA for inactivating a fungus, for protecting a plant against a fungus, or for inhibiting the expression of a gene involved in the glycosylphosphatidylinositol anchor biosynthesis pathway and to a composition comprising the DNA, vector or a dsRNA capable of inhibiting the expression of a gene involved in the glycosylphosphatidylinositol anchor biosynthesis pathway in a fungus.

## Description

Crops are often infected by plant pathogenic fungi. These infections lead to yield losses that can sometimes reach dramatic proportions. The problem of fungal infection has been addressed by phyto-sanitary measures, fungicide treatment and classical introgression of resistance genes in the sense of good agricultural practice. These measures do not lead to a lasting protection of plants from infection with phyto-pathogenic fungi because phyto-sanitary measures only lead to a certain decrease in the concentrations of infective agents, fungicide treatments often result in the emergence of fungicide resistant pathogens, and new crossed resistance genes are usually be broken after a short time. Recently a genetic method was introduced, which allows the generation of fungal resistant plants by expression of RNA interference (RNAi) against fungal target genes (Nowara et al., 2010, Plant Cell 22, 3130-3141). This method is called host-induced gene silencing (HIGS).

The fungal cell wall has structural and physiological functions and is of vital importance for fungal pathogens. The cell wall is a framework that protects the fungal cell against osmolysis, forms the shape of the hyphae and allows the adhesion and deposition of enzymes and UV protective pigments. The cell wall controls the access of large molecules into the cell and protects against lytic enzymes of other organisms. Some phyto-pathogenic fungi form specific infection structures, appressoria, stabilized by strong cell walls by means of high turgor pressure that allows the pathogens access to the host tissue.

In contrast to fungi infecting mammalian hosts, the vast majority of plant pathogenic fungi differentiate into specialized infection cells in order to invade their host plant and to establish a compatible interaction. For example, *Colletotrichum* species, including the maize anthracnose and stem rot fungus *Colletotrichum graminicola,* form a dome-shaped melanized appressorium on the plant. This infection cell develops a turgor pressure of approx. 5.35 MPa (corresponding to 53.5 bar), which is translated into a force at the appressorial base corresponding to 16.8 µN (Bechinger et al., 1999, Optical measurements of invasive forces exerted by appressoria of a plant pathogenic fungus. Science, 285, 1896-1899). Conceivably, rigid cell walls are required in functional appressoria, as indicated by spontaneously exploding infection cells of RNAi strains with reduced β-glucan contents (Oliveira-Garcia E. and Deising H.B., 2013, Infection Structure-Specific Expression of β-1,3-Glucan Synthase Is Essential for Pathogenicity of *Colletotrichum graminicola* and Evasion of β-Glucan-Triggered Immunity in Maize. Plant Cell 25, 2356-2378). Infection structure-specific expression of β-1,3-glucan synthase is essential for pathogenicity of *Colletotrichum graminicola* and evasion of β-glucan-triggered immunity (Oliveira-Garcia and Deising, 2013). Attenuation of PAMP-triggered immunity in maize requires down-regulation of the key β-1,6-glucan synthesis genes KRE5 and KRE6 in biotrophic hyphae of *Colletotrichum graminicola.* After breaching the plant cell wall, biotrophic infection vesicle, primary hyphae, and necrotrophic secondary hyphae are sequentially differentiated. Interestingly, necrotrophic infection hyphae of the RNAi strains mentioned above exhibit large swellings indicative of severe cell wall defects, and this also holds true for a chitin synthase V deletion mutant (Werner S., Sugui J.A., Steinberg G., Deising H.B., 2007. A chitin synthase with a myosin-like motor domain is essential for hyphal growth, appressorium differentiation, and pathogenicity of the maize anthracnose fungus Colletotrichum graminicola. Mol. Plant Microbe Interact. 20: 1555-1567).

Glycosylphosphatidylinositols (GPIs) are structurally complex glycolipids, which, after translocation into the ER lumen, are post-translationally added to the C-terminus of a series of proteins. Covalent attachment of GPI to the C-terminus is one of the most common post-translational modifications of proteins in eukaryotic cells and important for anchoring of proteins to the cell surface (Yu, S. et al., 2013. Recent progress in synthetic and biological studies of GPI anchors and GPI-anchored proteins, Curr Opin Chem Biol. 17(6), 1006-1013). In baker's yeast, 61 proteins have been estimated to be GPI-anchored (Pittet, M. and Conzelmann, A., 2007. Biosynthesis and function of GPI proteins in the yeast Saccharomyces cerevisiae. Biochimica et Biophysica Acta, 1771, 405-420), with half of these residing in the cell wall by covalent attachment to cell wall glucans. Structural analyses have shown that GPI-linked cell wall proteins are tightly associated with β-1,6-glucan. In addition, a subset of GPI-anchored cell wall proteins such as Cwp1 Cwp2, Tir1 and Tir2 of yeast possesses the DGQΦQ motif (where Φ represents any hydrophobic amino acid residue) that is thought to mediate a direct link to β-1,3-glucan (Levin, D.E., 2011. Regulation of Cell Wall Biogenesis in Saccharomycescerevisiae: The Cell Wall Integrity Signaling Pathway. Genetics, Vol. 189, 1145-1175). Thus, GPI anchors seem to play a role in cross-connecting cell wall β-glucan polymers and GPI-linked cell wall proteins play a major role in cell wall structure and rigidity.

Detailled studies in the model yeast *Saccharomyces cerevisiae* have shown that GPI anchor biosynthesis is involved in cell wall formation, likely due to the fact that several cell wall-remodeling enzymes require GPI-anchoring and targeting to the cell surface (Pittet and Conzelmann, 2007). The importance of selected genes involved in GPI anchor biosynthesis, i.e. genes accessible to gene deletion, has also been investigated in filamentous fungi, including *Neurospora crassa* (Bowman, S.M. et al., 2006. Mutational analysis of the glycosylphosphatidylinositol (GPI) anchor pathway demonstrates that GPI-anchored proteins are required for cell wall biogenesis and normal hyphal growth in *Neurospora crassa*, Eukaryot Cell 5: 587-600. doi:10.1128/EC.5.3.587-600.2006.), the opportunistic human pathogen *Aspergillus fumigatus* (Li, H. et al., 2007. Glycosylphosphatidylinositol (GPI) anchor is required in Aspergillus fumigatus for morphogenesis and virulence, Mol Microbiol 64: 1014-1027. doi:10.1111/j.1365-2958.2007.05709.x.), and the plant pathogenic fungi *Fusarium graminearum* (Rittenour W.R. and Harris S.D. (2013) Glycosylphosphatidylinositol-Anchored Proteins in Fusarium graminearum: Inventory, Variability, and Virulence, PLoS ONE 8(11): e81603. doi:10.1371/journal.pone.0081603). These fungi, however, do not undergo infection-related morphogenesis.

While the biosynthesis of GPI and its subsequent transfer to proteins as well as the role of GPI anchor biosynthesis for cell wall formation have been extensively studied in the model yeast Saccharomyces cerevisiae, as well as in Neurospora crassa, Aspergillus fumigatus, and Fusarium graminearum, these fungi are either not pathogenic for plants or do not undergo infection-related morphogenesis when infecting plants. However, there was to date no data whether genes involved in the GPI anchor biosynthesis would also play a role with respect to pathogenicity of fungi infecting plants, including fungi which undergo infection-related morphogenesis when infecting plants. Moreover, there was to date no data as to the suitability of genes involved in the GPI anchor as target genes in gene silencing approaches, let alone in an HIGS approach, for broad and permanent pathogen resistance in plants.

Thus, there exists a need for appropriate fungal target genes, the inhibition of which is suitable to confer broad and permanent resistance on plants against fungi, including fungi which undergo infection-related morphogenesis. To address this issue, we employed RNAi to characterize the genes involved in the GPI anchor biosynthesis pathway and found that down-regulation of transcript abundance of genes of the GPI anchor biosynthesis pathway causes severe cell wall defects in vegetative hyphae, in appressoria and in *in planta* differentiated infection hyphae, and leads to loss of pathogenicity of the fungus.

In particular, RNAi was employed to characterize four genes involved in the GPI anchor biosynthesis pathway of C. *graminicola* and *F. graminearum,* i.e. GPI12 encoding a N-acetylglucosaminylphosphatidylinositol deacetylase, GAA1 and GPI8 encoding the catalytic subunits of the transglutaminase complex, examined in C. *graminicola,* and GWT1 relevant for the vegetative growth of *F*. *graminearum.*

The data shown here suggest that GPI anchor biosynthesis is required for vegetative hyphal growth, conidiation, differentiation of functional appressoria, and development of invasive pathogenic hyphae and that inhibition of GPI anchor biosynthesis reduces pathogenicity of fungi.

In a first aspect, the present invention relates to a transgenic plant or a part thereof comprising as transgene a DNA capable of expressing an inhibitory nucleic acid molecule capable of inhibiting the expression of a gene involved in the glycosylphosphatidylinositol (GPI) anchor biosynthesis pathway in a fungus.

In an embodiment thereof, the gene comprises the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), preferably the GPI12, GAA1, GPI8 and/or GWT1 gene(s).

In an embodiment of the above, the transgenic plant or the part thereof comprises as transgene an expression cassette comprising the DNA.

In an embodiment of the above, the DNA or the expression cassette is stably integrated into the genome of the plant or present in the plant or the part thereof on a vector.

In an embodiment of the above, the inhibitory nucleic acid molecule is an antisense RNA or dsRNA (double stranded RNA), whereby the dsRNA is preferably hpRNA, siRNA or miRNA.

In an embodiment of the above, the DNA encodes an RNA molecule in sense direction and an RNA molecule in antisense direction, wherein the RNA molecule in sense direction or the RNA molecule in antisense direction are substantially complementary to the gene involved in the GPI anchor biosynthesis pathway or a part thereof, the RNA molecule in antisense direction is substantially reverse complementary to the RNA molecule in sense direction, and the RNA molecule in sense direction and the RNA molecule in antisense direction are able to build a dsRNA.

In an embodiment of the above, the length of the antisense RNA, the dsRNA, the RNA molecule in sense direction or the RNA molecule in antisense direction is at least 15, 16, 17, 18, 19 or 20 contiguous nucleotides, preferably at least 21, 22, 23, 24, 25, 30, 40, 50, 60, 70, 80, 90 or 100 contiguous nucleotides, more preferably at least 150, 200, 250, 300, 350, 450, 500, 600, 700, 800, 900 or 1000 contiguous nucleotides, still more preferably 300-1700, 300-1000, 300-700 contiguous nucleotides for the gene involved in the glycosylphosphatidylinositol (GPI) anchor biosynthesis pathway in a fungus or for the cDNA of the gene involved in the glycosylphosphatidylinositol (GPI) anchor biosynthesis pathway in a fungus, and most preferably 493 contiguous nucleotides for the GPI12 gene, 560 contiguous nucleotides for the GAA1 gene, or 690 contiguous nucleotides for the GPI8 gene.

In an embodiment of the above, the expression of the inhibitory nucleic acid molecule is controlled by a promoter, preferably an inducible promoter, more preferably a pathogen inducible and/or tissue specific promoter.

Each of the embodiments of the first aspect also refers back to each preceding embodiment.

In a second aspect, the present invention relates to a DNA capable of expressing an inhibitory nucleic acid molecule capable of inhibiting the expression of a gene involved in GPI anchor biosynthesis pathway in a fungus, whereby preferably the DNA is as defined in the first aspect of the invention, or an expression cassette comprising the DNA.

In a third aspect, the present invention relates to an inhibitory nucleic acid molecule capable of inhibiting the expression of a gene involved in GPI anchor biosynthesis pathway in a fungus, whereby preferably the inhibitory nucleic acid molecule is as defined in the first aspect of the invention.

In a fourth aspect, the present invention relates to a vector comprising the DNA or the expression cassette as referred to in the second aspect of the present invention.

In a fifth aspect, the present invention relates to method of producing the transgenic plant or the part thereof of the first aspect, comprising the following steps: introducing into at least a cell of the plant the DNA or the expression cassette as referred to in the second aspect of the present invention or the vector as referred to in the fourth aspect of the present invention or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus, and regenerating the transgenic plant from the at least a cell.

In a sixth aspect, the present invention relates to method of conferring fungal resistance on a plant or the part thereof comprising the following steps: introducing into the plant or the part thereof the DNA or the expression cassette of the second aspect or the vector of the fourth aspect or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus, and causing expression of the DNA, the expression cassette, the vector, or the dsRNA.

In a seventh aspect, the present invention relates to a method of inhibiting the expression of a gene involved in GPI anchor biosynthesis pathway, comprising: applying the DNA or the expression cassette of the second aspect or the vector of the fourth aspect or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus to the fungus or to a plant or a part thereof.

In an eighth aspect, the present invention relates to a use of the DNA or the expression cassette of the second aspect or the vector of the fourth aspect or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus for inactivating a fungus, while contacting a plant or a part thereof; for protecting a plant against an infection by a fungus; or for inhibiting the expression of a gene involved in the glycosylphosphatidylinositol (GPI) anchor biosynthesis pathway, preferably the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably the GPI12, GAA1, GPI8 and/or GWT1 gene(s), in a fungus.

In a ninth aspect, the present invention relates to a composition comprising the DNA or the expression cassette of the second aspect or the vector of the fourth aspect or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus.

The invention also relates to a transgenic plant or a part thereof comprising a DNA or an expression cassette comprising the DNA, wherein the DNA encodes an aRNA or dsRNA directed against the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 DNA and/or mRNA, preferably against the GPI12, GAA1, GPI8 and/or GWT1 DNA and/or mRNA, wherein more preferably the dsRNA is hpRNA, siRNA or miRNA, or the aRNA is part of the dsRNA, siRNA or miRNA.

The invention also relates to a transgenic plant or a part thereof comprising a DNA or an expression cassette comprising the DNA, wherein the DNA encodes an aRNA or dsRNA directed against the GPI12 DNA and/or mRNA, wherein preferably the dsRNA is hpRNA, siRNA or miRNA, or the aRNA is part of the dsRNA, siRNA or miRNA.

The invention also relates to a transgenic plant or a part thereof comprising a DNA or an expression cassette comprising the DNA, wherein the DNA encodes an aRNA or dsRNA directed against the GAA1 DNA and/or mRNA, wherein preferably the dsRNA is hpRNA, siRNA or miRNA, or the aRNA is part of the dsRNA, siRNA or miRNA.

The invention also relates to a transgenic plant or a part thereof comprising a DNA or an expression cassette comprising the DNA, wherein the DNA encodes an aRNA or dsRNA directed against the GPI8 DNA and/or mRNA, wherein preferably the dsRNA is hpRNA, siRNA or miRNA, or the aRNA is part of the dsRNA, siRNA or miRNA.

The invention also relates to a transgenic plant or a part thereof comprising a DNA or an expression cassette comprising the DNA, wherein the DNA encodes an aRNA or dsRNA directed against the GWT1DNA and/or mRNA, wherein preferably the dsRNA is hpRNA, siRNA or miRNA, or the aRNA is part of the dsRNA, siRNA or miRNA.

The invention also relates to a transgenic plant or a part thereof comprising (a) DNA(s) or (an) expression cassette(s) comprising the DNA(s), wherein the DNA(s) encode(s) (an) aRNA(s) or dsRNA(s) directed against any combination of at least two of the GPI12, GAA1, GPI8 and/or GWT1 DNAs and/or mRNAs, wherein preferably the dsRNA is hpRNA, siRNA or miRNA, or the aRNA is part of the dsRNA, siRNA or miRNA.

The invention also relates to a transgenic plant or a part thereof comprising a DNA or an expression cassette comprising the DNA, wherein the DNA comprises an antisense and a sense sequence whereby the antisense sequence is capable of hybridizing to fungal GPI12 mRNA.

The invention also relates to a transgenic plant or a part thereof comprising a DNA or an expression cassette comprising the DNA, wherein the DNA comprises an antisense and a sense sequence whereby the antisense sequence is capable of hybridizing to fungal GAA1 mRNA.

The invention also relates to a transgenic plant or a part thereof comprising a DNA or an expression cassette comprising the DNA, wherein the DNA comprises an antisense and a sense sequence whereby the antisense sequence is capable of hybridizing to fungal GPI8 mRNA.

The invention also relates to a transgenic plant or a part thereof comprising a DNA or an expression cassette comprising the DNA, wherein the DNA comprises an antisense and a sense sequence whereby the antisense sequence is capable of hybridizing to fungal GWT1 mRNA.

The invention also relates to a transgenic plant or a part thereof comprising (a) DNA(s) or (an) expression cassette(s) comprising the DNA(s), wherein the DNA(s) comprise(s) (an) antisense and (a) sense sequence(s), whereby the antisense sequence(s) is (are) capable of hybridizing to any combination of at least two of the GPI12, GAA1, GPI8 and/or GWT1 mRNAs.

The invention also relates to a transgenic plant or a part thereof comprising a DNA or an expression cassette comprising the DNA, wherein the DNA encodes an RNA molecule in sense direction and an RNA molecule in antisense direction, wherein the RNA molecule in sense direction or the RNA molecule in antisense direction are present on one RNA molecule.

The invention also relates to a transgenic plant or a part thereof comprising a DNA or an expression cassette comprising the DNA, wherein the DNA encodes an RNA molecule in sense direction and an RNA molecule in antisense direction, wherein the RNA molecule in sense direction or the RNA molecule in antisense direction are present on different RNA molecules.

The invention also relates to a transgenic plant or a part thereof comprising a DNA or an expression cassette comprising the DNA, wherein the DNA encodes an RNA molecule in sense direction and an RNA molecule in antisense direction, wherein the RNA molecule is siRNA or miRNA.

The present inventors have surprisingly demonstrated that inhibition of fungal genes involved in the GPI anchor biosynthesis pathway by a polynucleotide introduced into a plant confers resistance on the plant against fungi via gene silencing. The polynucleotide causes cessation of infection, growth, development, reproduction and/or pathogenicity and eventually results in the death of the fungal organism. This demonstration renders the present invention superior over the prior art, as targeting GPI anchor biosynthesis will disturb the function of a large number of proteins (in yeast 61 proteins are predicted to be GPI anchor proteins (Pittet, M. and Conzelmann, A.,2007), in contrast to the specific inhibition of single polysaccharide biosynthesis genes (e.g. β-1,3-glucan synthase, β-1,6-glucan synthase, chitin synthase) which are the target of inhibition in the prior art. Moreover, all known fungal GPI-anchored proteins end up either at the plasma membrane or in the cell wall. The inhibition of the GPI anchor protein biosynthesis does not only disturb the cell wall architecture but also triggers additional defense reactions of the plant. GPI-cell wall proteins, which are not attached to the GPI anchor, are frequently secreted by the fungal cells (Pittet and Conzelmann, 2007) and might act as pathogen-associated molecular pattern (PAMP). The induction of PAMP mediated resistance by GPI-cell wall protein secretion, which enhance plant resistance, is another difference to the already known approaches. Finally, the GPI anchor protein biosynthesis pathway has never been selected for a HIGS approach until now.

The invention demonstrates for the first time that vegetative fungal growth and asexual spore formation are impaired in a phytopathogenic fungus by inhibiting the expression of GPI12, GAA1 and/or GPI18 gene(s). GPI12-, GAA1- and/or GPI18-RNAi fungal strains had a lower rate of conidiation as compared to wildtype strains and the spores were shorter with increased diameters, resulting from reduced cell wall rigidity of the conidia. Moreover, it has been shown that germination rates as well as appressorium formation rates were reduced. Finally, it was shown that GPI12-, GAA1- and/or GPI18-RNAi fungal strains had pathogenicity or virulence defects, so that the strains were unable to invade into intact plants. Additionally, the invention demonstrates for the first time that fungal growth of a GWT1-RNAi fungal strain is impaired. Tested in plants harboring a GWT1-RNAi construct, the plants showed reduced pathogenicity symptoms.

Thus, by using reverse genetic methods, the inventors were able to show that the GPI12, GAA1, GPI18 and GWT1 genes are excellent HIGS target genes capable of selectively interrupting the GPI anchor biosynthesis pathway. The excellent suitability of these genes as targets for inhibiting pathogenicity of fungi is substantiated by several properties, inter alia, these genes are single-copy genes such that in case of an RNAi approach it can be excluded that a second allele will save the host-induced gene silencing and no complete reduction of transcript concentrations is required in order to induce pathogenicity which is another crucial point.

The present inventors have shown by using exemplary genes involved at different stages in the GPI anchor biosynthesis pathway, being the GPI12, GAA1, GPI8 and GWT1 genes, that inhibition of the GPI anchor biosynthesis pathway causes cessation of infection, growth, development, reproduction and/or pathogenicity of fungi. The skilled person will recognize the also the other genes involved in the GPI anchor biosynthesis pathway represent true targets, the inhibition of which has the same effect, namely cessation of infection, growth, development, reproduction and/or pathogenicity of fungi.

The genes involved in the GPI anchor biosynthesis pathways which may be targeted individually or in combination by an inhibitory nucleic acid molecule are excellent targets. Since these genes have homologs in the genomes of a wide variety of fungi, these genes may be used to confer permanent resistance on a wide variety of plant species including a large variety of economically important crop species against a wide variety of plant-pathogenic fungi.

The term "a gene", "a gene involved in the GPI anchor biosynthesis pathway in a fungus" or "a gene involved in the GPI anchor biosynthesis pathway", as referred to herein, refers to any fungal gene or corresponding cDNA which encodes a protein which is involved in the biosynthesis of GPI, thus including genes which are so far known to be involved in the GPI anchor biosynthesis pathway, as referred to below, and genes which are involved in the GPI anchor biosynthesis pathway, however, will be identified in the future. A gene in a fungus is usually a DNA, as the fungal genome consists of DNA. However, the term "gene", as understood herein, also comprises fungal RNA, preferably fungal mRNA, which is transcribed from the gene and is translated into a protein, as well as cDNA derived from fungal mRNA. The inhibitory nucleic acid molecule capable of inhibiting the expression of a gene as comprised by the present invention is capable of inhibiting fungal DNA and/or RNA such as mRNA which is transcribed from the DNA. The RNA and mRNA are understood as being "involved in the GPI anchor biosynthesis pathway".

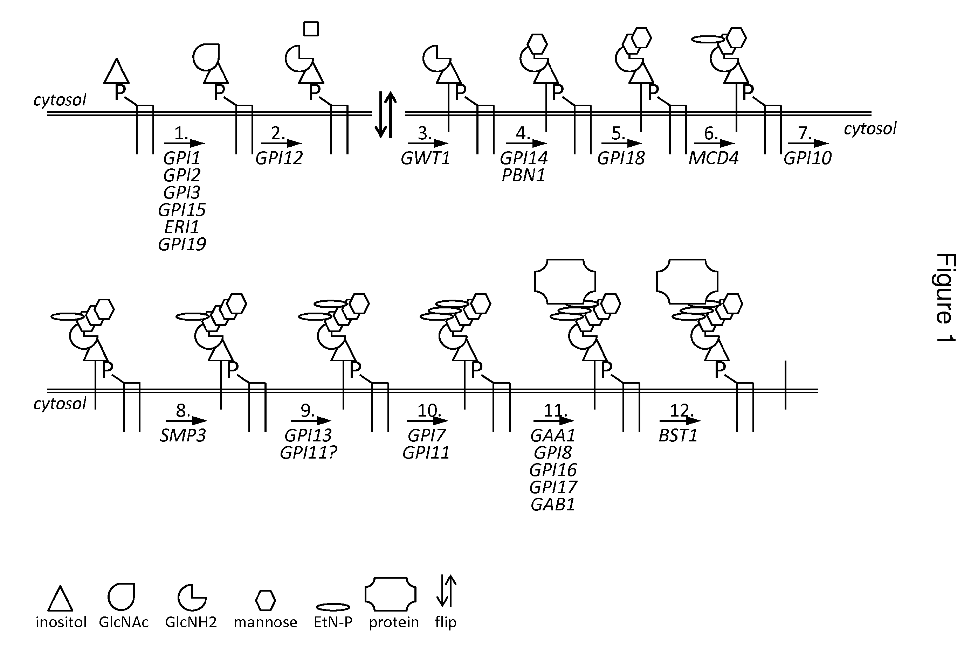

Biosynthesis of GPI and its subsequent transfer to proteins has been extensively studied in baker's yeast. As far as it is known, the different biosynthetic steps are mediated by approx. 25 proteins as shown in Figure 1 and take place successively on the cytoplasmic and on the luminal face of the ER membrane. The GPI anchor biosynthesis pathway is composed of 12 biosynthetic steps, starting with the addition of N-acetyl glucosamine to the membrane anchored phospholipid phosphatidylinositol and terminating with the addition of a protein via its C terminus to the complete GPI. A schematic presentation of the GPI anchor biosynthesis pathway is given in Figure 1. The biosynthesis of GPI starts with the addition of N-acetyl glucosamine to phosphatidylinositol which reaction occurs on the cytoplasmic ER surface. The acetyl group of the *N*-acetyl glucosaminyl residue is then removed and the glucosamine formed is enzymatically 2-*O-*palmitolyated, using acyl-CoA as the acyl donor. The resulting intermediate is translocated to the luminal side of the phospholipid bilayer. In the ER lumen, two mannosyl units are subsequently linked to the glucosamine. Next, a phosphoryl-ethanolamine residue is added to the first mannosyl unit. In yeast, the addition of a fourth onto the third mannosyl residue, previously attached, is required for adding a phosphoethanolamine group to the third mannosyl residue. The last step of GPI biosynthesis before attaching this anchor to a protein is the addition of a third phosphoryl-ethanolamine residue. Then, a protein is attached to GPI.

More specifically, the first step of GPI biosynthesis is the addition of *N*-acetyl glucosamine to phosphatidylinositol. This reaction occurs on the cytoplasmic ER surface and is catalyzed by N-acetyl glucosaminyl transferase, an unusually complex glycosyl transferase consisting of the six core proteins *GPI1, GPI2, GPI3, GPI15, ERI1,* and *GPI19.* The acetyl group of the *N*-acetyl glucosaminyl residue is then removed by the cytoplasmic *N-*acetylglucosaminylphosphatidylinositol deacetylase encoded by *GPI12,* and the glucosamine formed is enzymatically 2-*O*-palmitolyated, using acyl-CoA as the acyl donor. Palmitic acid is added onto the 2-OH group of inositol by GWT1, an acyl-CoA dependent inositol acyltransferase. The resulting intermediate is translocated to the luminal side of the phospholipid bilayer. In the ER lumen, two mannosyl units are subsequently linked to the glucosamine by the two distinct ER-resident dolichol phosphate mannose:glucosaminyl phosphatidylinositol α1-4-mannosyltransferases: GPI14 and GPI18. PBN1, a type I transmembrane glycoprotein, forms a complex with GPI14, thereby stabilizing it. Interestingly, analysis of the substrate specificity of the first dolichol phosphate mannose:glucosaminyl phosphatidylinositol α1-4-mannosyltransferase of the glycosyl-phosphatidylinositol biosynthetic pathway of African trypanosomes showed that N-acetylglucosaminylphosphatidylinositol is more efficiently presented to the enzyme than its deacetylated form, suggesting important substrate channelling via the preceding deacetylase GPI12. Next, the addition of a phosphoryl-ethanolamine residue to the first mannosyl unit, is catalyzed by MCD4. The phosphoryl-ethanolamine group linked to the first mannosyl residue is required for both recognition of the GPI by the mannosyl transferase GPI10 attaching the third mannosyl, as well as by the transamidase complex. In yeast, the addition of a fourth onto the third mannosyl residue by SMP3 is required for adding a phosphoethanolamine group to the third mannosyl residue, as catalyzed by GPI13. The last step of GPI biosynthesis before attaching this anchor to a protein is the addition of a third phosphoryl-ethanolamine residue, which is mediated by ethanolamine phosphotransferase GPI7. The attachment of GPI to a protein is catalyzed by the GPI transamidase complex, a protein complex consisting of the five distinct subunits GPI8, GPI16, GAA1, GAB1, and GPI17, organized into three components. According to a recent model, the central part and the catalytic domain of the GPI transamidase complex is GPI8. GPI8 interacts with GAA1 and with all other subunits of the GPI transamidase complex. GPI8 is a cystein protease cleaving the C-terminal pro-peptide of the substrate protein even in the absence of the GPI anchor (Maxwell, S.E., Ramalingam, S., Gerber, L.D., Brink, L., and Udenfriend S. (1995) An active carbonyl formed during glycosylphosphatidylinositol addition to a protein is evidence of catalysis by a transamidase. J Biol Chem. 270(33):19576-19). Importantly, GAA1 has recently been suggested to function as a metallo-peptide-synthetase catalyzing formation of the peptide bond between the substrate protein's ω-site and the GPI lipid anchor's phosphoethanolamine. The complex reactions involved in biosynthesis of GPI and attachment of the GPI anchor to proteins are conserved from yeast to men. In the last step, the inositol deacylase BST1 removes the palmitic acid moiety from the mature GPI anchored proteins and allows the export of GPI proteins from the ER to the Golgi.

Thus, the term "a gene", "a gene involved in the GPI anchor biosynthesis pathway in a fungus" or "a gene involved in the GPI anchor biosynthesis pathway" refers to any fungal gene which encodes a protein which is involved in the GPI anchor biosynthesis pathway, starting with the addition of N-acetyl glucosamine to phosphatidylinositol and ending with the deacylation of phosphatidylinositol (step 12). Intermediate steps are shown in Figure 1. However, as used herein, the term "a gene", "a gene involved in the GPI anchor biosynthesis pathway in a fungus" or "a gene involved in the GPI anchor biosynthesis pathway" refers to any gene of a GPI anchor biosynthesis pathway, for example a GPI anchor biosynthesis pathway starting with the addition of N-acetyl glucosamine to phosphatidylinositol and ending with step 12, also if the GPI anchor biosynthesis pathway is different from the pathway shown in Figure 1. Furthermore as used herein, the term "gene" comprises DNA involved in the GPI anchor biosynthesis pathway, RNA involved in the GPI anchor biosynthesis pathway and/or mRNA involved in the GPI anchor biosynthesis pathway as well as cDNA derived from mRNA involved in the GPI anchor biosynthesis pathway.

Preferably, the term "a gene", "a gene involved in the GPI anchor biosynthesis pathway in a fungus "or" a gene involved in the GPI anchor biosynthesis pathway", as used herein, comprises GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably GPI12, GAA1, GPI8 and/or GWT1 gene(s).

GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes which are comprised by the present invention as target genes in gene silencing approaches are those which are known in the art as fungal GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 gene, respectively, or are known under a different designation, which is a synonym to the above designations. Exemplary nucleotide and protein sequences of GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, or BST1 of a series of fungi are indicated in Tables 1-5 and in the exemplary part by their accession numbers as available from the NCBI database (National Centre for Biotechnology Information; National Library of Medicine 38A, Bethesda, MD20894, USA; www.ncbi.nih.gov). The specific nucleotide and amino acid sequences are disclosed in the accompanying sequence listing under SEQ ID NOs: 1 to 400.

Moreover, fungal GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes which are comprised by the present invention are those which are homologous to or homologs of known GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes, respectively, such as e.g. those identified for GPI12, GAA1, GWT1 and GPI8 in the sequence listing under odd numbers of SEQ ID Nos: 1 to 350 and 389-394. Homologs are characterized by an identity of at least 30, 40, 50, 60, 70, 80, 85, 90, 95 or 99 % to known genes. In analogy, fungal GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 proteins which are comprised by the present invention are those which are homologous to or homologs of known GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 proteins, respectively, such as e.g. those identified in the sequence listing under even numbers of SEQ ID Nos: 1 to 350, 389-394 and SEQ ID NOs: 395-400. Homologs are characterized by an identity of at least 60, 70, 80, 85, 90, 95 or 99 % to known proteins. Moreover, homologs are characterized by a homology (similarity) of at least 40, 50, 60, 70, 80, 85, 90, 95 or 99 % to known proteins. Homologous amino acids have identical or homologous amino acids. Homologous amino acid residues have similar chemical-physical properties, for example, amino acids belonging to a same group: aromatic (Phe, Trp, Tyr), acid (GIu, Asp), polar (GIn, Asn), basic (Lys, Arg, His), aliphatic (Ala, Leu, He, VaI), with a hydroxyl group (Ser, Thr), or with a short lateral chain (GIy, Ala, Ser, Thr, Met). It is expected that substitutions between such homologous amino acids do not change a protein phenotype (conservative substitutions). A series of algorithms is known in the art for determining homologous sequences. For example, the Smith-Waterman algorithm (Smith and Waterman, "Identification of common molecular subsequences." Journal of molecular Biology 147.1 (1981): 195-197.) is generally known and used in the art if performing pairwise sequence comparisons and the skilled person knows how to apply it. Other algorithms which may be used are the algorithms of Needleman and Wunsch, 1970 ("A general method applicable to the search for similarities in the amino acid sequence of two proteins." Journal of molecular biology 48.3 (1970): 443-453.), the similarity method of Pearson and Lipman, 1988 ("Improved tools for biological sequence comparison." Proceedings of the National Academy of Sciences 85.8 (1988): 2444-2448.), or the algorithm of Karlin and Altschul, 1990 ("Methods for assessing the statistical significance of molecular sequence features by using general scoring schemes." Proceedings of the National Academy of Sciences 87.6 (1990): 2264-2268.), modified by Karlin and Altschul, 1993 ("Applications and statistics for multiple high-scoring segments in molecular sequences." Proceedings of the National Academy of Sciences 90.12 (1993): 5873-5877.), or computerized implementations of these algorithms (GAP, BESTFIT, BLAST, PASTA, and TFASTA in the Wisconsin Genetics Software Package, Genetics Computer Group (GCG), 575 Science Dr., Madison, WI). Typically, the default values of 5.00 for gap weight and 0.30 for gap weight length are used.

Alternatively, fungal genes are comprised by the present invention as target genes if they hybridize under stringent conditions to fungal GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes, respectively, such as those identified by SEQ ID NOs: 1 to 400.

Alternatively, fungal genes are comprised by the present invention as target genes if they encode proteins having the same activity as the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB 1, and BST1 proteins, respectively, such as those identified by SEQ ID NOs: 1 to 400.

Alternatively, identification of GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes and/or proteins may occur via a consensus sequence. Alignment of known GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes or protein sequences may reveal conserved regions. Based on conserved regions, consensus sequences on the nucleic acid and/or amino acid level can be designed which may be used to identify additional GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes, respectively, in other fungi. Accordingly, GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes and proteins which are comprised by the present invention as target genes and proteins, respectively, are those which comprise consensus sequences identified as indicated above.

The term "and/or" within the terms "GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1" and "GPI12, GAA1, GPI8 and/or GWT1" means that either one of the indicated genes or any combination of at least 2, 3, 4, 5, etc. to all genes is or are inhibited in the fungus.

The term "a protein involved in the GPI anchor biosynthesis pathway in a fungus" or "a protein involved in the GPI anchor biosynthesis pathway", as referred to herein, comprises any protein which is encoded by a gene involved in the GPI anchor biosynthesis pathway. Moreover, the term "an RNA involved in the GPI anchor biosynthesis pathway in a fungus" or "a RNA involved in the GPI anchor biosynthesis pathway", as referred to herein, comprises any RNA which is expressed from the gene involved in the GPI anchor biosynthesis pathway. Also, the term "a mRNA involved in the GPI anchor biosynthesis pathway in a fungus" or "a mRNA involved in the GPI anchor biosynthesis pathway", as referred to herein, comprises any mRNA which is expressed from the gene involved in the GPI anchor biosynthesis pathway.

A preferred gene of the present invention is the GPI12, GAA1, GPI8 or GWT1 gene. In analogy, a preferred RNA, mRNA or protein involved in the GPI anchor biosynthesis pathway is the GPI12, GAA1, GPI8 or GWT1 RNA, mRNA or protein, respectively. In the exemplary part of the specification, a series of GPI12, GAA1, GPI8 and GWT1 genes and proteins of various fungi are listed and characterized by their accession numbers. The sequences are comprised in the sequence listing under SEQ ID NOs: 1 to 350 and 389-400. In addition to those genes and proteins, any genes and proteins are comprised by the present invention which fall under the definitions, as presented above.

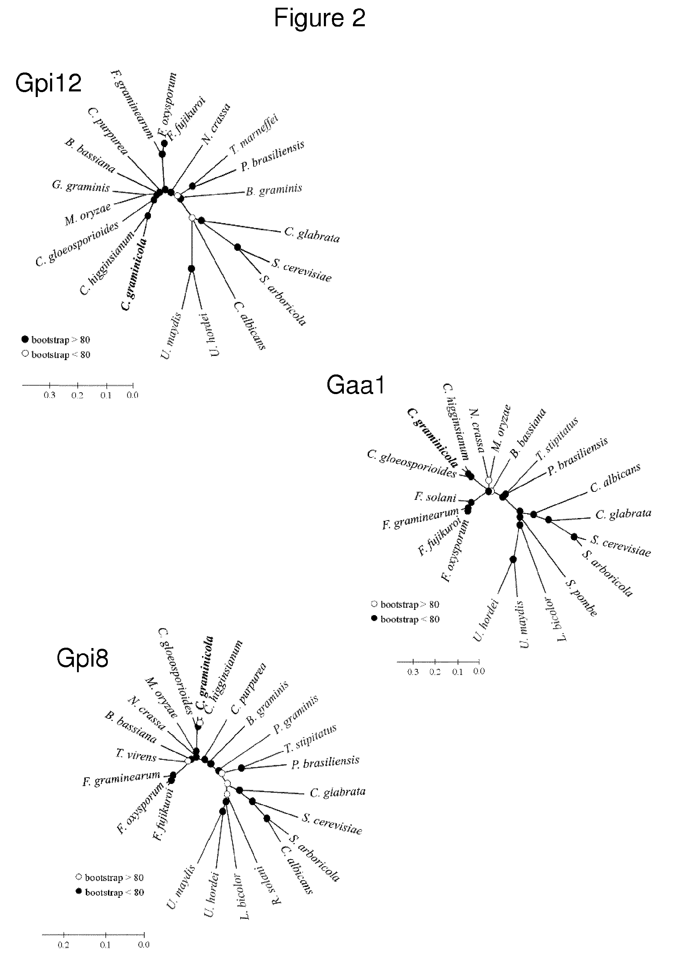

The present invention comprises proteins as fungal target proteins which have the same activity as the GPI12, GAA1, GPI8 and GWT1 proteins identified in the sequence listing as SEQ ID NOs: 1 to 350 and 389 to 400 and/or as indicated directly below. Thereby, GPI12 encodes an N-acetylglucosaminylphosphatidylinositol deacetylase which removes the acetyl group of the *N-*acetyl glucosaminyl residue previously attached to the inositol residue in the second step of the GPI anchor biosynthesis pathway. GAA1 is part of the GPI transamidase complex, which catalyses the attachment of GPI to a protein in the eleventh step of the GPI biosynthesis pathway. GAA1 seems to be a metallo-peptide-synthetase catalyzing formation of the peptide bond between the substrate protein's ω-site and the GPI lipid anchor's phosphoethanolamine. GPI8 is another part of the GPI transamidase complex. It seems to be a cystein protease cleaving the C-terminal pro-peptide of the substrate protein, even in the absence of the GPI anchor (Maxwell, 1995). GAA1 and *GPI8* genes encode two indispensable sub-units of the GPI transamidase complex catalyzing the final GPI anchor transfer to proteins. These genes are single-copy genes in C. *graminicola.* The derived proteins share significant similarity with the corresponding proteins of other filamentous ascomycota, with high homology across proteins of different *Colletotrichum* species (Fig. 2). As expected, sequences of yeasts and of basidiomycota (Fig. 2) are not as closely related to C. *graminicola* GPI12, GAA1 and GPI8. For example, the amino acid identity of C. *graminicola* GPI12, GAA1 and GPI8 with the corresponding C. *higginsianum* proteins is 82, 92, and 94%, respectively. The identity with the *Magnaporthe oryzae* proteins is 61, 65, and 81%, the identity with the S. *cerevisiae* proteins is 30, 31, and 54%, respectively.

Identification of GPI12, GWT1, GAA1, GPI8 genes of other fungal pathogens:
In order to identify conserved amino acid sequences which can be used to identify further GPI12 genes of, e.g. ascomycota and basidiomycota, the GPI12 sequences of several fungal pathogens *(Blumeria graminis f. sp. hordei, Blumeria graminis f. sp. tritici, Bipolaris maydis, Bipolaris victoriae, Bipolaris zeicola, Pyrenophora tritici-repentis, Pyrenophora teres f. teres, Leptosphaeria maculans*, *Mycosphaerella graminicola, Puccinia graminis tritici, Puccinia striiformis, Puccinia triticina, Ustilago hordei, Ustilago maydis, Sclerotinia sclerotiorum, Gaeumannomyces graminis var. tritici, Magnaporthe oryzae, Colletotrichum gloeosporioides, Colletotrichum graminicola, Colletotrichum higginsianum*, *Fusarium fujikuroi, Fusarium oxysporum, Fusarium graminearum, Claviceps purpurea, Verticillium dahliae)* were compared as shown in Figure (15). The amino acid sequences AHPDDEAMFF (SEQ ID NO: 401) and ILCLSSGDADGLG (SEQ ID NO: 402) are highly conserved and suitable to identify further GPI12 proteins of, e.g. ascomycota and basidiomycota. Alternatively, the amino acid sequences AHPDDEXXFF (SEQ ID NO: 411) and LSXGXXXGLG (SEQ ID NO: 412), wherein X is any naturally occurring amino acid, are highly conserved and are suitable consensus sequences to identify further GPI12 proteins of, e.g. ascomycota and basidiomycota. Preferably, X at position 7 of SEQ ID NO: 411 is A (Alanine), C (Cysteine) or S (Serine) and X at position 8 of SEQ ID NO: 411 is M (Methionine) or L (Leucine). Preferably, X at position 3 of SEQ ID NO: 412 is S (Serine) or T (Threonine), X at position 5 of SEQ ID NO: 412 is D (Aspartic Acid) or N (Asparagine), X at position 6 of SEQ ID NO: 412 is A (Alanine) or S and X at position 7 of SEQ ID NO: 412 is D, T, A or E (Glutamic Acid).

In order to identify conserved amino acid sequences which can be used to identify further GWT1 genes of, e.g. ascomycota and basidiomycota, the GWT1 sequences of several fungal pathogens *(Alternaria solani, Pyrenophora teres f. teres, Pyrenophora tritici-repentis, Blumeria graminis f. sp. tritici, Botrytis cinerea, Sclerotinia sclerotiorum, Colletotrichum fioriniae, Colletotrichum graminicola, Colletotrichum higginsianum*, *Fusarium graminearum, Fusarium pseudograminearum*, *Fusarium oxysporum*, *Fusarium verticillioides, Fusarium solani, Magnaporthe oryzae, Cercospora beticola, Sphaerulina musiva, Mycosphaerella fijiensis, Septoria tritici, Zymoseptoria brevis, Mycosphaerella pini, Melampsora populina, Puccinia graminis tritici,*

*Leptosphaeria maculans, Septoria nodorum*, *Setosphaeria turcica, Bipolaris maydis, Bipolaris oryzae, Bipolaris sorokiniana, Bipolaris victoriae, Bipolaris zeicola)* were compared as shown in Figure (16). The amino acid sequences GTSLMDXGVGSFV (SEQ ID NO: 403) and HVXEYGVHWNFFFTL (SEQ ID NO: 404) are highly conserved and suitable to identify further GWT1 proteins of, e.g. ascomycota and basidiomycota. Alternatively, the amino acid sequences GXSLMDXGVGSFV (SEQ ID NO: 413) and HVXEYGXHWNFFFTL (SEQ ID NO: 414) are highly conserved and are suitable consensus sequences to identify further GPI12 proteins of, e.g. ascomycota and basidiomycota. X is any naturally occurring amino acid. Preferably, X at position 2 of SEQ ID NO: 413 is T (Threonine), V (Valine) or I (Isoleucine), X at position 7 of SEQ ID NO: 403 or SEQ ID NO: 413 is M (Methionine), S (Serine), L (Leucine), I or V. Preferably, X at position 3 of SEQ ID NO: 404 or SEQ ID NO: 414 is T (Threonine) or S (Serine), and X at position 7 of SEQ ID NO: 414 is V (Valine) or I (Isoleucine).

In order to identify conserved amino acid sequences which can be used to identify further GAA1 genes of, e.g. ascomycota, several GAA1 sequences of ascomycota *(Cercospora beticola, Mycosphaerella graminicola, Bipolaris oryzae, Bipolaris zeicola*, *Leptosphaeria maculans, Botrytis cinerea*, *Blumeria graminis f. sp. hordei, Blumeria graminis f. sp. tritici, Colletotrichum gloeosporioides, Colletotrichum graminicola, Colletotrichum orbiculare, Verticillium alfalfa, Verticillium dahliae, Fusarium fujukuroi, Fusarium oxysporum*, *Fusarium graminearum, Fusarium solani*, *Gaeumannomyces graminis var. tritici, Magnaporthe oryzae)* were compared as shown in Figure (17). The amino acid sequences TYISENALLPGQVHTYFGGS (SEQ ID NO: 405), RYFKRWSLWSKDI (SEQ ID NO: 406) and NNLLEHLHQSFFFY (SEQ ID NO: 407) are highly conserved and suitable to identify further GAA1 proteins of, e.g. ascomycota. Alternatively, the amino acid sequences TYXSENAXLPGQVHTYFXGS (SEQ ID NO: 415), RYFXRWSXWSKDI (SEQ ID NO: 416) and NNLLEHXHQSFFFY (SEQ ID NO: 417) are highly conserved and suitable to identify further GAA1 proteins of, e.g. ascomycota. X is any naturally occurring amino acid. Preferably, X at position 3 of SEQ ID NO: 415 is I (Isoleucine) or V (Valine), X at position 8 of SEQ ID NO: 415 is L (Leucine) or I, and X at position 18 of SEQ ID NO: 415 is A (Alanine), G (Glycine) or T (Threonine). Preferably, X at position 4 of SEQ ID NO: 416 is K (Lysine), R (Arginine) or N (Asparagine), and X at position 8 of SEQ ID NO: 416 is L (Leucine) or I (Isoleucine). Preferably, X at position 7 of SEQ ID NO: 417 is L (Leucine) or F (Phenylalanine).

In order to identify conserved amino acid sequences which can be used to identify further GPI8 genes of, e.g. ascomycota and basidiomycota, the GPI8 sequences of several fungal pathogens (*Blumeria graminis f. sp. tritici, Botrytis cinerea*, *Bipolaris maydis, Bipolaris sorokiniana*, *Pyrenophora teres f. teres, Pyrenophora tritici-repentis, Leptosphaeria maculans*, *Puccinia graminis, Ustilago hordei, Ustilago maydis, Fusarium graminearum, Fusarium oxysporum, Colletotrichum gloeosporioides, Colletotrichum graminicola, Colletotrichum higginsianum*, *Gaeumannomyces graminis var. tritici, Magnaporthe oryzae, Verticillium dahliae)* were compared as shown in Figure (18). The amino acid sequences NWAVLVCTSRFWFNYRH (SEQ ID NO: 408), VKRLGIPDSQIILMLPDDMACNPRNAFPG (SEQ ID NO: 409) and YMTGHGGNEFLKFQDAEEI (SEQ ID NO: 410) are highly conserved and suitable to identify further GPI8 proteins of, e.g. ascomycota and basidiomycota. The amino acid sequences NWAVLVXTSXFWFNYRH (SEQ ID NO: 418), VKRLGIPDSXIILMLXDDXACNPRNXFPG (SEQ ID NO: 419) and YMTGHGGXEFLKFQDXEEI (SEQ ID NO: 420) are highly conserved and suitable to identify further GPI8 proteins of, e.g. ascomycota and basidiomycota. X is any naturally occurring amino acid. Preferably, X at position 7 of SEQ ID NO: 418 is C (Cysteine), S (Serine) or G (Glycine), and X at position 10 of SEQ ID NO: 418 is R (Arginine) or K (Lysine). Preferably, X at position 10 of SEQ ID NO: 419 is Q (Glutamine) or N (Asparagine), X at position 16 of SEQ ID NO: 419 is P (Proline) or A (Alanine), X at position 19 of SEQ ID NO: 419 is M (Methionine) or A, and X at position 26 of SEQ ID NO: 419 is A, K (Lysine) or M. Preferably, X at position 8 of SEQ ID NO: 420 is N (Asparagine) or D (Aspartic acid), and X at position 16 of SEQ ID NO: 420 is A (Alanine) or Y (Tyrosine).

As used herein, "part" of the gene involved in the GPI anchor biosynthesis pathway is meant any part of the gene to which an aRNA or an RNA molecule in antisense direction hybridizes and inhibits expression thereof. According to the above, the "part" of the gene may be at least 15, 16, 17, 18, 19 or 20 contiguous nucleotides, preferably at least 21, 22, 23, 24, 25, 30, 40, 50, 60, 70, 80, 90 or 100 contiguous nucleotides, and more preferably at least 150, 200, 250, 300, 350, 450, 500, 600, 700, 800, 900 or 1000 contiguous nucleotides in length, but is below the length of gene involved in the GPI anchor biosynthesis pathway. Still more preferably, the length is 100-1700, such as 200-1000, or 300-700 contiguous nucleotides, and most preferably 493 contiguous nucleotides for the GPI12 gene, 560 contiguous nucleotides for the GAA1 gene, or 690 contiguous nucleotides for the GPI8 gene. The "part" of the gene may be in any region of the gene involved in the GPI anchor biosynthesis pathway, so that upon hybridizing of the aRNA or RNA molecule in antisense direction, the transcription of the gene or the translation of the mRNA is inhibited. The "part" may be in a regulatory region such as the promoter region or terminator regulatory sequence, in exon sequences, in intron sequences or along the whole mRNA, as long as the transcription of the gene or the translation of the mRNA is inhibited. The "part" may be one or more than one parts. The "more than one part" may be located on the same gene or on different genes.

As used herein, a "fungus" is any fungus which comprises a GPI anchor biosynthesis pathway. Preferably, the fungus comprises (a) GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably the fungus comprises (a) GPI12, GAA1, GPI8 and/or GWT1 gene(s). Still more preferably, the fungus is selected from the group consisting of ascomycota, basidiomycota, zygomycota, chytridiomycota, blastocladiomycota, neocallimastigomycota, and glomeromycota. Still more preferably, the fungus is a phyto-pathogenic fungus. Still more preferably, the fungus is selected from the genus Alternaria; Aphanomyces; Ashbya; Aspergillus; Beauveria; Bipolaris; Blumeria; Botryotinia; Botrytis; Cercospora; Chaetomium; Colletotrichum; Cladosporium; Claviceps; Coccidioides; Cordyceps; Cryptococcus; Diplocarpon; Diplodia; Drechslera; Endocarpon; Erysiphe; Exophiala; Exserophilum; Fusarium; Gaeumannomyces; Geotrichum; Lachancea; Leptosphaeria; Macrophomina; Magnaporthe; Marssonina; Metarhizium; Microsphaera; Monographella; Myceliophthora; Neurospora; Paracoccidioides; Phaeosphaeria; Phakospora; Puccinia; Pyrenophora; Pyricularia; Ramularia; Rhizoctonia; Saccharomyces; Sclerotium; Trichoderma; Sclerotinia; Ustilago; Verticillium; Zygosaccharomyces; Zymoseptoria; Mycosphaerella; Penicillium; Podospora; Paracoccidioides; Pseudopeziza;; Phaeosphaeria; Sclerotinia; Sordaria; Schizsaccharomyces, Zygosaccharomyces; Talaromyces; Thielavia; Venturia, and Pseudocercospora; and still more preferably the fungus is selected from *Alternaria solani; Aspergillus flavus; Alternaria alternata; Aphanomyces cochlioides; Ashbya gossypii; Beauveria bassiana; Bipolaris maydis; Bipolaris sorokiniana; Botryotinia fuckeliana; Botrytis cinerea; Cercospora beticola, Cercospora kikuchii; Cercospora sojina; Cercospora zea maydis; Colletotrichum graminicola; Colletotrichum gloeosporioides; Colletotrichum orbiculare; Claviceps purpurea; Cordyceps militaris; Diplodia maydis; Drechslera glycines; Drechsiera oryzae; Erysiphe betae; Exserohilum turcicum; Fusarium fujikuroi; Fusarium graminearum; Fusarium moniliforme; Fusarium oxysporum; Fusarium solani; Gaeumannomyces graminis; Leptosphaeria maculans; Magnaporthe oryzae; Microsphaera diffusa; Metarhizium acridum; Metarhizium anisopliae, Penicillium spp.; Pyrenophora teres; Pyrenophora tritici*-*repentis; Puccinia graminis; Trichoderma virens; Sclerotinia sclerotiorum; Ustilago maydis; Ustilago hordei; Verticillium alfalfa; Verticillium dahlia; Zygosaccharomyces bailii; Zygosaccharomyces rouxii; Blumeria graminis; Chaetomium globosum; Colletotrichum higginsianum; Colletotrichum orbiculare; Coccidioides posadasi; Endocarpon posillum; Exophiala dermatitidis; Lachancea thermotoleran; Marssonina brunnea; Myceliophthora thennophile; Metarhizium acridum; Macrophomina phaseolina; Monographella albescens; Paracoccidioides brasiliensis; Phaeoshaeria maydis; Phakopsora pachyrhizi; Pyrenophora tritici*-*repentis; Phaeosphaeria nodorum; Puccinia graminis; Puccinia polysora; Puccinia striiformis; Puccinia triticina; Pyricularia oryzae; Ramularia beticola, Rhizoctonia oryzae; Rhizoctonia solani; Sclerotium rolfsii; Saccharomyces arboricola; Sordaria macrospora; Schizsacchaomyces japonicus, Zygosaccharomyces rouxii; Talaromyces marneffei; Talaromyces stipitatus; Thielavia terrestris; Venturia inaequalis; Verticillium albo-atrum; Verticillium dahlia; Zymoseptoria tritici; and Pseudocercospora fijiensis.* The fungus may also be selected from the genus Candida such as *Candida albicans; Candida glabrata; Candida orthopsilosis; Candida parapsilosis; or Candida tropicalis.* The fungal genes of the present invention may serve in the finding of not yet identified gene(s) of phyto-pathogenic fungi, e.g. via consensus sequences.

Also included within the term "fungus" are fungus-like eukaryotic microorganisms such as the oomycota or oomycetes which include notorious pathogens of plants, causing devastating diseases such as late blight of potato and sudden oak death. Examples of oomycetes which are included by the present invention are Phytophthora infestans, Phytophthora palmivora, Phytophthora sojae.

Oomycetes are included within the present invention and are, for the purposes of the present invention, included within the term "fungal" or "fungus".

As used herein, the term "phyto-pathogenic" or "pathogenic" refers to a fungus which causes a disease in a plant.

The methods as comprised by the present invention which are used to inhibit the expression of a gene involved in the GPI anchor biosynthesis pathway in a fungus are known to the skilled person under the term "gene silencing". "Gene silencing" is a general term used to describe the regulation of gene expression. In particular, this term refers to the ability of a cell to prevent the expression of a gene in the cell. Gene silencing can occur during either transcription or translation of a gene. The application of gene silencing methods in plants is well known in the art. Reference is made to Plant Gene Silencing - Methods and Protocols, Mysore K.S and Senthil-Kumar M. (eds.), Springer Protocols, Humana Press, 2015, which provides the reader with a comprehensive review of various gene silencing methodologies and its applications. The skilled person may apply any methods described in this document or elsewhere which are suitable to achieve gene silencing in a plant or in a fungus.

Generally used gene silencing methodologies in plants include host-induced gene silencing (HIGS) which is a method where a plant controls pathogens by RNA interference (RNAi) and exhibits an enhanced resistance when carrying suitable gene silencing constructs. RNA interference is a natural process used by cells in many eukaryotes to regulate gene expression. RNA interference is a vital part of the immune response to viruses and other foreign genetic material, especially in plants. For inducing RNAi in a cell or organism, dsRNA with a sequence substantially complementary to a gene of interest is synthesized either within a cell or organism or it is synthesized outside the cell or organism and introduced into the cell or organism, where it is recognized as exogenous genetic material and activates the RNAi pathway. The double-stranded molecule is cut into small double-stranded fragments by an enzyme called Dicer. These small fragments, which include small interfering RNAs (siRNA) and microRNA (miRNA), are often 19-40 nucleotides in length. The fragments integrate into a multi-subunit protein called the RNAi induced silencing complex (RISC). One strand of the molecule, the guide strand or antisense strand, binds to RISC, while the other strand, the passenger strand or sense strand, is degraded. The guide strand pairs with a complementary sequence in an mRNA molecule and induces cleavage by Argonaute, the catalytic component of the RISC complex, thereby preventing it from being used as a translation template.

Other gene silencing methodologies in plants use antisense oligonucleotides which are short nucleic acid fragments that bind to substantially complementary target mRNA molecules. These molecules are single-stranded RNA molecules generally 15 to 25 nucleotides long.

The preferred method used in the present invention is the HIGS method. Moreover, any other gene silencing method known in the art may be used for inhibiting the expression of a gene in a fungus while being in contact with a plant.

For the production of fungus resistant plants in the present invention, a DNA capable of expressing an inhibitory nucleic acid molecule capable of inhibiting the expression of a gene involved in the GPI anchor biosynthesis pathway in a fungus is selected and introduced into the plant or a part thereof.

As used herein, "a DNA capable of expressing an inhibitory nucleic acid molecule" is any DNA molecule which results in an inhibitory nucleic acid molecule. Preferably, the DNA comprises an antisense sequence which is substantially complementary to contiguous stretches of the fungal gene involved in the GPI anchor biosynthesis pathway or part thereof. Upon transcription of the DNA, an inhibitory molecule, preferably an RNA molecule, is produced which comprises the antisense sequence which is capable of hybridizing to the fungal gene involved in the GPI anchor biosynthesis pathway or part thereof. The RNA molecule may be an mRNA molecule, a single-stranded antisense RNA, or a dsRNA, for example an hpRNA, siRNA or miRNA. The DNA may comprise one or more than one antisense sequence(s). Thus, the DNA may comprise one or more than one antisense sequence(s) against one of the fungal genes involved in the GPI anchor biosynthesis pathway or more than one antisense sequence(s) against more than one of the fungal genes involved in the GPI anchor biosynthesis pathway. Preferably, the DNA comprises one or more than one antisense sequence(s) against the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, or BST1 gene, more preferably against the GPI12, GAA1, GPI8 or GWT1 gene, or alternatively more than one antisense sequence against any of a combination of at least two of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 genes, more preferably against the GPI12, GAA1, GPI8 and GWT1 genes. The DNA may comprise in a still more preferred embodiment one or more than one sense sequence(s) which is (are) substantially complementary to the antisense sequence(s). Within this latter embodiment, the DNA preferably comprises one or more than one sense sequence(s) against the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, or BST1 gene, more preferably against the GPI12, GAA1, GPI8 or GWT1 gene, or alternatively more than one sense sequence against any of a combination of at least two of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 gene, more preferably against the GPI12, GAA1, GPI8 and GWT1 genes. The antisense and sense sequences may be present on the same or on different DNA strands. Upon transcription, inhibitory molecule(s) comprising the antisense and possibly sense sequences are generated. The DNA may be double-stranded or single-stranded and is preferably double-stranded.

The DNA capable of expressing an inhibitory nucleic acid molecule may be present within an expression cassette. As used herein, an "expression cassette" is a nucleic acid molecule which is composed of one or more genes or genetic sequences and the sequences controlling their expression. An expression cassette may contain a promoter regulatory sequence, also designated promoter, operably linked to an open reading frame or another genetic sequence, and a 3' untranslated region that may contain a polyadenylation site. The promoter directs the machinery of the cell to make RNA and/or protein. As used herein, "operably linked" means that expression of the linked DNA sequences occurs in the plant. An expression cassette may be part of a vector used for cloning and introducing the DNA into a cell.

As used herein, an "antisense sequence" is a sequence which is substantially complementary to any contiguous stretch of (a) fungal mRNA(s) involved in the GPI anchor biosynthesis pathway or part(s) thereof. The antisense sequence is selected such that it hybridizes to a contiguous sequence element of the mRNA(s) involved in the GPI anchor biosynthesis pathway and inhibits the translation thereof. The antisense sequence has a length which allows inhibition of translation of the mRNA(s) involved in the GPI anchor biosynthesis pathway in the fungus. The antisense sequence as comprised by an antisense RNA (aRNA) which inhibits the mRNA(s) involved in the GPI anchor biosynthesis pathway via the antisense mechanism has a length of at least 15 nucleotides and may extend to hundreds of nucleotides or over the whole length of the mRNA(s) involved in the GPI anchor biosynthesis pathway. The preferred length of the antisense sequence as comprised by an aRNA is 15 to 300, 15 to 200, 15 to 100 or 15 to 50 nucleotides and most preferably 15 to 25 nucleotides. The antisense sequence as comprised by dsRNA which induces the RNAi machinery has a length of at least 19 nucleotides and may extend to hundreds of nucleotides or over the whole length of the mRNA(s) involved in the GPI anchor biosynthesis pathway. The preferred length of the antisense sequence as comprised by dsRNA is 19 to 300, 19 to 200, 19 to 100 or 19 to 50 nucleotides and most preferably 19 to 25 nucleotides. The antisense sequence may be directed against the coding part or the regulatory part of the fungal mRNA(s) involved in the GPI anchor biosynthesis pathway.

The selection of a suitable antisense sequence can be performed by methods known in the art. For RNAi constructs, RNAi programs like Emboss siRNA prediction (http://emboss.sourceforge.net/apps/release/6.6/emboss/apps/sirna.html) are available which identify suitable sequence elements of the genes involved in the GPI anchor biosynthesis pathway

As used herein, a "sense sequence" is a sequence which is substantially complementary to an antisense sequence. Due to the substantial complementarity of the sequences, the antisense and sense sequences hybridize with each other.

The term "complementary" includes "complementary" as well as "reverse complementary". "Reverse complementary" means that the nucleotides of a reverse complementary sequence are, as regards their 5' to 3' extension, complementary and ordered in a mirrored fashion with respect to a polynucleotide sequence. Thus, independent of whether antisense and sense sequences are on the same strand or on different strands (DNA or RNA), they need to be reverse complementary allowing them to form a double-strand either by intramolecular hybridization or inter-molecular hybridization, respectively.

The term "capable of expressing" means that the DNA is the starting substance which is transferred within the plant cell into a nucleic acid molecule which is capable of inhibiting the expression of a gene involved in the GPI anchor biosynthesis pathway in a fungus. Preferably, this term comprises the transcription of the DNA into an RNA molecule, which can hybridize to a gene involved in the GPI anchor biosynthesis pathway in a fungus and inhibit the expression thereof.

As used herein, "an inhibitory nucleic acid molecule" or "an inhibitory nucleic acid molecule capable of inhibiting the expression of a gene involved in the GPI anchor biosynthesis pathway" is any nucleic acid molecule which inhibits the transcription and/or translation of a gene involved in the GPI anchor biosynthesis pathway in a fungus. Inhibition may occur in the coding part of the gene involved in the GPI anchor biosynthesis pathway in the fungus or the regulatory part located in the regions 5' and/or 3' to the gene. Preferably, the inhibition is by hybridizing to the gene involved in the GPI anchor biosynthesis pathway in the fungus due to substantial complementarity, so that the DNA involved in the GPI anchor biosynthesis pathway and/or (m)RNA involved in the GPI anchor biosynthesis pathway cannot be transcribed and/or translated, respectively. Inhibition may also be achieved by degrading the hybridized gene involved in the GPI anchor biosynthesis pathway. Preferably, the inhibitory nucleic acid molecule is an RNA molecule which is transcribed from the DNA capable of expressing an inhibitory nucleic acid molecule. More preferably, the inhibitory nucleic acid molecule is a single-stranded antisense RNA (aRNA) which comprises an antisense sequence which is substantially complementary to the fungal gene involved in the GPI anchor biosynthesis pathway and which hybridizes thereto and inhibits the translation thereof. Alternatively, the inhibitory nucleic acid is an RNA molecule in sense direction and an RNA molecule in antisense direction. The RNA molecule in sense direction and the RNA molecule in antisense direction may be one RNA molecule or may be different RNA molecules. Due to their inverse substantial complementarity, the sense and antisense sequences hybridize with each other and form dsRNA. The dsRNA such as siRNA or miRNA may be composed of two separate strands or may be one strand such as hpRNA. In a further alternative, the inhibitory nucleic acid molecule is a dsRNA, namely siRNA or miRNA, which forms within the plant during the RNAi procedure, whereby dsRNA transcribed from the DNA induces the RNAi mechanism and is processed to the siRNA or miRNA. Alternatively, the inhibitory molecule may be a catalytic RNA or ribozyme. In a further alternative, the inhibitory nucleic acid molecule may be or encode a repressor molecule which functions by inhibiting the transcription and/or translation of the gene(s) involved in the GPI anchor biosynthesis pathway and/or RNA(s) involved in the GPI anchor biosynthesis pathway in the fungus. For example, the repressor molecule functions by inhibiting the access of proteins, which are necessary for transcription or translation, to the fungal gene(s) involved in the GPI anchor biosynthesis pathway and/or RNA(s) involved in the GPI anchor biosynthesis pathway.

As used herein, the term "capable of inhibiting the expression of a gene", "capable of inhibiting the expression of a gene involved in the GPI anchor biosynthesis pathway", "inhibiting the expression of a gene", or "inhibiting the expression of a gene involved in the GPI anchor biosynthesis pathway" means that the inhibitory nucleic acid molecule (can) prevent(s) that the fungal gene involved in the GPI anchor biosynthesis pathway is processed further, such as transcribed or translated. Prevention may occur by any kind of inhibition such as hybridization to the gene, cleaving of the gene, repressing the transcription or translation of the gene or catalytic actions as performed by ribozymes. Preferably, the nucleic acid molecule can hybridize or hybridizes to the gene involved in the GPI anchor biosynthesis pathway and prevents the further processing thereof such as transcription of DNA or translation of mRNA. Prevention occurs either by blocking the gene involved in the GPI anchor biosynthesis pathway so that downstream actions cannot be performed or by degrading the gene involved in the GPI anchor biosynthesis pathway by host-specific enzymes. A reduction of expression of at least 40 %, 50 %, 60 %, 70 %, 80 %, 90 %, 95 % or of 100 % for a gene involved in the GPI anchor biosynthesis pathway, as compared to expression in a non-transgenic plant of identical phenotype, allows scoring the transgenic plant as being resistant or as having resistance against a fungus. Preferably, the damages on the plant are reduced by at least 50 %, 60 %, 70 %, 80 %, 90 % or by 100 % in a resistant transgenic plant according to the present invention as compared to a non-transgenic plant of identical phenotype. A reference plant may also be a plant of same species with natural resistance against a specific fungus, such as a plant which has the best fungal resistance in a specific area such as Germany, as compared to other plants of same species.

Each fungus has its characteristic picture of damages which it causes with a host plant. The damages are e.g. visible in the form of leaf spots or root rot (dry texture, concentric rings, discoloration and fruiting structures) or vascular wilt (gradual wilting of the above ground shoots). Other signs are the presence of mycelium and fruiting bodies which range in size from microscopic to easily detectable with the eye. They are found within the leaf spot or stem rot area. The skilled person knows other forms of damages which are caused by fungi on plants. The extent of damages can e.g. be determined by determining the relative area of spots on the plant leaves.

As used herein, the term "a DNA", "an inhibitory nucleic acid molecule" or "a gene" includes the singular and plural forms, particularly incudes "one DNA", "more than one DNA", "one inhibitory nucleic acid molecule", "more than one inhibitory nucleic acid molecule", "one gene", and "more than one gene", such as 2, 3, 4, 5, etc. DNAs expressing 2, 3, 4, 5, etc. inhibitory nucleic acid molecules directed against 2, 3, 4, 5, etc. to all genes involved in the GPI anchor biosynthesis pathway.

The term "a gene" or "a gene involved in the GPI anchor biosynthesis pathway", means either one gene or more than one gene involved in the GPI anchor biosynthesis pathway, such as 2, 3, 4, 5, etc. to all genes involved in the GPI anchor biosynthesis pathway. Consequently, "a DNA capable of expressing an inhibitory nucleic acid molecule" or "an inhibitory nucleic acid molecule" may comprise one or more DNA(s) capable of expressing one or more inhibitory nucleic acid molecule(s) against one gene involved in the GPI anchor biosynthesis pathway or more than one gene such as 2, 3, 4, 5, etc. to all genes involved in the GPI anchor biosynthesis pathway. Consequently, the plant of the present invention may comprise in a cell one DNA molecule expressing one or more inhibitory nucleic acid(s) against one gene involved in the GPI anchor biosynthesis pathway or against more than one gene involved in the GPI anchor biosynthesis pathway. Alternatively, the plant of the present invention may comprise in a cell more than one DNA expressing more than one inhibitory nucleic acid molecules, each DNA or each inhibitory nucleic acid molecule being against one or more than one specific gene(s) involved in the GPI anchor biosynthesis pathway. Also, combinations of DNA against one gene and DNA against more than one gene are comprised by the present invention. In analogy, also combinations of inhibitory nucleic acid molecule against one gene and inhibitory nucleic acid molecule against more than one gene are comprised by the present invention. Moreover, a DNA may express more than one inhibitory nucleic acid molecule against one fungal gene.

The term "expression of a gene" or "expression of a gene involved in the GPI anchor biosynthesis pathway" means (1) the transcription of a gene involved in the GPI anchor biosynthesis pathway into an RNA or mRNA involved in the GPI anchor biosynthesis pathway and/or (2) the translation of a RNA or mRNA involved in the GPI anchor biosynthesis pathway into a protein.

As used herein, an "antisense RNA" or "aRNA" is a single-stranded RNA which comprises an antisense sequence which is substantially complementary to (the) mRNA(s) involved in the GPI anchor biosynthesis pathway which is (are) transcribed in the fungus. The aRNA binds to mRNA(s) involved in the GPI anchor biosynthesis pathway produced by the fungus by base-pairing, thereby obstructing the translation machinery and inhibiting translation and further processing. Consequently, the production of protein(s) involved in the GPI anchor biosynthesis pathway is inhibited.

As used herein, an "RNA molecule in antisense direction" is an RNA molecule which comprises an antisense sequence.

As used herein, an "RNA molecule in sense direction" is an RNA molecule which comprises a sense sequence. An RNA molecule in antisense direction and an RNA molecule in sense direction may be one RNA molecule, i.e. the antisense and sense sequences are present on the same RNA molecule, or may be different RNA molecules, i.e. the antisense and sense sequences are present on different RNA molecules.

As used herein, a "double-stranded RNA" or "dsRNA" is an RNA molecule that is partially or completely double stranded. The dsRNA may be formed by an RNA molecule in antisense direction and an RNA molecule in sense direction. Double-stranded RNA may be formed by a single nucleic acid strand which comprises the sense and antisense sequences. In this case, the RNA molecule in sense direction and the RNA molecule in antisense are on the same RNA molecule. The resulting dsRNA has a hairpin or stem-loop structure, whereby the stem is formed by the sense and anti-sense sequences. The loop may be formed by a sequence which has no substantial complementarity within the strand and lies between the sense and antisense sequences. Such a loop sequence can be an intron sequence of a gene, such as the intron of the RGA2 gene of wheat (Loutre et al., 2009, Two different CC-NBS-LRR genes are required for *Lr10*-mediated leaf rust resistance in tetraploid and hexaploid wheat, Plant Journal 60, 1043-1054) or the second intron of the Cut2 gene of *Magnaporthe oryzae*. Alternatively, the dsRNA may be formed by two different RNA strands, whereby the antisense sequence and the sense sequence are on different strands. In this case, the RNA molecule in antisense direction is different from the RNA molecule in sense direction.

The term "dsRNA" comprises dsRNA which is capable of activating the RNAi pathway in a plant cell. The term "dsRNA" also comprises small or short interfering RNA (siRNA) and micro-RNA (miRNA). The two latter dsRNAs are either expressed in a transgenic plant of the present invention from the DNA capable of expressing an inhibitory nucleic acid molecule or are produced by the RNAi machinery in the plant cell starting from a longer dsRNA expressed from the DNA. The term "dsRNA" also comprises circular interfering RNA (ciRNA), short hairpin RNA (shRNA) and the like. The term "dsRNA" also includes dsRNA, such as hpRNA, siRNA, miRNA or longer dsRNA, comprising antisense and sense sequences, which is not produced within a transgenic plant, but is produced by genetic engineering or synthesizing methods in the laboratory and which is for external application on a plant and/or fungus.

As used herein, "RNAi" or "RNA interference" refers to the process of sequence-specific gene silencing, mediated by double-stranded RNA (dsRNA). In order to function, one strand of the dsRNA comprises an antisense sequence which is substantially complementary to (the) mRNA(s) involved in the GPI anchor biosynthesis pathway or (a) part(s) thereof which is (are) transcribed in the fungus. The other strand comprises a sense sequence which is substantially complementary to the antisense sequence. The dsRNA is capable of activating the RNAi pathway in a plant cell resulting in the formation of a single stranded RNA comprising the antisense strand which binds to (the) mRNA(s) involved in the GPI anchor biosynthesis pathway in the fungus and results in the degradation of the mRNA.

According to the present invention, the sizes of the sense or antisense sequence or the sizes of the RNA molecule in sense direction or the RNA molecule in antisense direction may be the same. Alternatively, the sizes may differ.

The DNA capable of expressing an inhibitory nucleic acid molecule results in one embodiment of the present invention, upon expression in the plant cell in an mRNA molecule which may function as an aRNA and inhibit mRNA(s) involved in the GPI anchor biosynthesis pathway in the fungus. Such DNA molecule comprises on the sense strand an antisense sequence.

In another embodiment, the DNA which is capable of expressing an inhibitory nucleic acid molecule results upon expression in the plant cell in an mRNA molecule or in mRNA molecules which may form a dsRNA which may activate the RNAi machinery. Within this embodiment, the DNA encodes an RNA molecule in sense direction and an RNA molecule in antisense direction. The sense sequence and the antisense sequence may be present on one DNA strand. This may result in an mRNA molecule which comprises the sense sequence and antisense sequence on the same mRNA molecule. Due to the reverse substantial complementarity of the sense sequence and antisense sequence, a hairpin dsRNA will form. Alternatively, if e.g. the sense and antisense sequences being present on the same DNA strand are under the control of different promoters or of one bidirectional promoter, different mRNA molecules are transcribed, one harboring the sense sequence and the other harboring the antisense sequence. Different mRNA molecules, one harboring the sense sequence and the other harboring the antisense sequence, may also form if the sense sequence and the antisense sequence are present on different strands of the DNA. Due to the invers substantial complementarity of the sense and antisense sequences, the mRNAs form a dsRNA. The dsRNA may be recognized as foreign by the plant cell and may activate the RNAi machinery. The dsRNA transcribed from the DNA may be an siRNA or miRNA molecule or may be processed to an siRNA or miRNA molecule. After infection of the plant with a fungus, an exchange of the RNA generated in the plant may occur between the plant and the fungus. The RNA incorporated in the fungus may lead to a sequence-specific gene silencing of a gene involved in the GPI anchor biosynthesis pathway. It is known that the siRNA effect can be continued in plants if the RNA dependent RNA polymerase synthesizes new siRNAs from the degraded mRNA fragments. These secondary or transitive RNAis can enhance the silencing.

As used herein, the term "DNA", "DNA molecule" or DNA polynucleotide" etc. refers to double-stranded DNA, unless otherwise specified.

As used herein, "substantially complementary" refers to polynucleotide strands that are capable of base pairing according to the standard Watson-Crick complementarity rules. It is understood that two polynucleotides hybridize to each other even if they are not completely complementary to each other. Consequently, the term "substantially complementary" means that two polynucleotide sequences are complementary over at least 80% of their nucleotides, preferably over at least 85%, 90%, 95%, 96%, 97%, 98%, 99%. Most preferably, the two polynucleotide sequences are complementary over 100 % of their nucleotides. Alternatively, "substantially complementary" means that two polynucleotide sequences can hybridize under high stringency conditions. It is understood that an antisense sequence which is substantially complementary to (a) fungal gene(s) involved in the GPI anchor biosynthesis pathway or transcript(s) involved in the GPI anchor biosynthesis pathway has a low degree of complementarity to functionally identical or similar genes in plants, preferably below 60 %, and does not hybridize or only to a low degree to endogenous plant genes or transcripts, so that the transcription and/or translation of plant genes is not reduced or reduced to at most 10 %, as compared to a non-transgenic plant of identical phenotype.

As used herein, the term "hybridize(s)(ing)" refers to the formation of a hybrid between two nucleic acid molecules via base-pairing of complementary nucleotides. The term "hybridize(s)(ing) under stringent conditions" means hybridization under specific conditions. An example of such conditions includes conditions under which a substantially complementary strand, namely a strand composed of a nucleotide sequence having at least 80 % complementarity, hybridizes to a given strand, while a less complementary strand does not hybridize. Alternatively, such conditions refer to specific hybridizing conditions of sodium salt concentration, temperature and washing conditions. As an example, highly stringent conditions comprise incubation at 42°C, 50% formamide, 5 x SSC (150 mM NaCl, 15 mM trisodium citrate), 50 mM sodium phosphate, 5 x Denhardt's solution, 10 x dextran sulphate, 20 mg/ml sheared salmon sperm DNA and washing in 0.2 x SSC at about 65°C (SSC stands for 0.15 M sodium chloride and 0.015 M trisodium citrate buffer). Alternatively, highly stringent conditions may mean hybridization at 68°C in 0.25 M sodium phosphate, pH 7.2, 7% SDDS, ImM EDTA and 1% BSA for 16 hours and washing twice with 2 x SSC and 0.1% SDDs at 68°C. Further alternatively, highly stringent hybridisation conditions are, for example: Hybridizing in 4 x SSC at 65°C and then multiple washing in 0.1 x SSC at 65°C for a total of approximately 1 hour, or hybridizing at 68°C in 0.25 M sodium phosphate, pH 7.2, 7% SDS, 1 mM EDTA and 1% BSA for 16 hours and subsequent washing twice with 2 x SSC and 0.1% SDS at 68°C.

For introducing the DNA molecule capable of expressing an inhibitory nucleic acid molecule into a plant or a part thereof, the DNA molecule or the expression cassette harboring the DNA may be inserted into a vector. Vectors which harbor a DNA polynucleotide for effecting inhibition of gene expression in a plant cell are known to those in the art and are also useful for the purposes of the present invention of inhibiting the expression of (a) gene(s) involved in the GPI anchor biosynthesis pathway in a fungus. In addition to the DNA molecule, the vector may comprise heterologous regulatory element(s) in the 5' and optionally in the 3' positions which are able to function in a plant. The vector may comprise a promoter regulatory sequence that is functional in plant cells, operably linked to the DNA molecule, and optionally a terminator regulatory sequence. Such a vector may be a hairpin vector or a double promoter vector, as known in the art. In a double promoter vector, the inserted DNA molecule is transcribed bidirectionally. An inverted double promoter allows the expression of one DNA sequence in the 3' direction and of a second DNA sequence in the 5' direction, whereby the resulting RNAs are substantially complementary to each other and generate dsRNA. Alternatively, two promoters or a bidirectional promoter, e.g. the mannopine synthase promoter (Guevara-García et al., 1993, Tissue-specific and wound-inducible pattern of expression of the mannopine synthase promoter is determined by the interaction between positive and negative cis-regulatory elements, Plant Journal (3):495-505), may be employed such that one promoter regulates the transcription of a DNA sequence comprising a sense sequence and the second promoter regulates the transcription of a DNA sequence comprising an antisense sequence, which, however, is present on the DNA not in complementary location to the sense sequence. Suitable binary vectors for the transformation of plants are the pBINPLUS vector (van Engelen et al., 1995, Transgenic Research 4, 288-290), the pGPTV vector (Becker et al., 1992, New plant binary vectors with selectable markers located proximal to the left T-DNA border, Plant Mol. Biol., 29, 1195-1197), the p6U and p7U vector (www.dna-cloning.com).

According to the invention, the term "promoter regulatory sequence" or "promoter" is intended to mean any promoter of a gene that can be expressed in a plant. Such promoter may be a promoter which is naturally expressed in a plant or is of fungal, bacterial, or viral origin. Alternatively, the promoter may be an inducible promoter. Preferably, the promoter is a tissue specific promoter and/or a pathogen inducible promoter.

Inducible promoters are activated by one or more stimuli such as hormones (for example gibberellin, abscisic acid, jasmonic acid, salicylic acid, ethylene, auxin), chemicals (tetracycline, dexamethasone, estradiol, copper, ethanol, and benzothiadiazol), environmental conditions (light, temperature), abiotic stress (water stress, salt, stress, wounding) and biotic stress (Gurr, S.J., and Rushton, P.J., 2005. Engineering plants with increased disease resistance: How are we going to express it? Trends in Biotechnology. 23 (6), 283-290).

Pathogen-inducible promoters allow the expression of the inhibitory nucleic acid confined to the sites of infection. Native promoters, which are suitable for the pathogen-induced expression of the inhibitory nucleic acid molecule, were isolated from pathogen inducible genes (WO 2000/071732; Strittmatter, G. et al., (1996) Infections with various types of organisms stimulate transcription from a short promoter fragment of the potato gst1 gene, Mol Plant Microbe Interact 9, 68-73).

Synthetic pathogen-inducible promoters are constructed using cis-acting element building blocks and minimal promoters from various sources. The idea behind synthetic promoters is to improve the expression characteristics so as to make promoters that are more suited to the biotechnological aim. The major goal is to reduce unwanted background expression and increase promoter strength. Suited cis-acting elements, minimal promoters and synthetic pathogen-inducible promoters (4xD, 2xS-2xD) were described (WO 2000/29592; WO 2007/147395; WO 2013/091612; Gurr and Rushton, 2005).

The inducible promoter can be a chimeric promoter which is composed of a plurality of elements and does not occur as such in nature. It may contain a minimal promoter and include, upstream from the minimal promoter, at least one cis-regulatory element which serves as a binding site for specific trans-acting factors (e.g. transcription factors). A chimeric promoter can be designed according to the desired requirements and is induced or repressed by different factors. The selection of the cis-regulatory element or a combination of cis-regulatory elements is critical for the specificity or the activity level of a promoter. A cis-regulatory element in a chimeric promoter is either heterologous to the minimal promoter used, i.e. the cis-regulatory element is derived from a different organism or from a different species to that of the minimal promoter used, or a cis-regulatory element in a chimeric promoter is homologous to the minimal promoter used, i.e. the cis-regulatory element and the minimal promoter also occur combined in a natural promoter, however the cis-regulatory element is localized itself or as an additional element within the chimeric promoter in a genetic environment that is different from the natural promoter. A chimeric promoter thus also means a (natural) promoter that was altered by multimerization of at least one cis-regulatory element.

Examples of promoters of plant origin are the histone promoter (EP 0 507 698) or the rice actin promoter (U.S. Pat. No. 5,641,876). Examples of promoters of a plant virus gene are the cauliflower mosaic virus (CaMV 19S or 35S), the cassava vein mosaic virus (CsVMV: WO97/48819) or the circovirus promoter (AU 689 311). Examples for tissue specific promoters are the C1 promoter, which is exclusively active in green plant tissues (Stahl D. J., Kloos, D. U., and Hehl, R. (2004). A sugar beet chlorophyll a/b binding protein void of G-box like elements confer strong and leaf specific reporter gene expression in transgenic sugar beet. BMC Biotechnology 4;31: 12) and the root-specific Mll promoter (Oltmanns H., Kloos D. U., Brieβ W., Pflugmacher M., Stahl D. J., and Hehl, R. (2006). Taproot promoters cause tissue specific gene expression within the storage root of sugar beet. PLANTA 224: 485-495). Further examples of tissue-specific promoters are the napin (EP 0 255 378), phaseolin, glutenin, helianthinin (WO 92/17580), albumin (WO 98/45460) and oleosin (WO 98/45461) promoter. Examples of inducible promoters are the promoters of phenylalanine ammonia lyase (PAL), of HMG-CoA reductase (HMG), of chitinases, of glucanases, of proteinase inhibitors (PI), of genes of the PR1 family, of nopaline synthase (nos) or of the vspB gene (U.S. Pat. No. 5,670,349), the HMG2 promoter (U.S. Pat. No. 5,670,349), the apple beta-galactosidase (ABG1) promoter or the apple amino cyclopropane carboxylate synthase (ACC synthase) promoter (WO 98/45445), or chimeric pathogen inducible promoters (WO 00/29592; WO 2007/147395; WO 2013/091612). An example of a fungal promoter is the constitutive trpC promoter of Aspergillus nidulans (Yelton et al., Transformation of Aspergillus nidulans by using a trpC plasmid, Proceedings of the National Academy of Sciences USA 81 (1984): 1470-1474).

According to the invention, the term "terminator" or "terminator regulatory sequence" is intended to mean any such sequence that is functional in a plant, also comprising polyadenylation sequences. It may be of bacterial origin such as the nos or ocs terminator of *Agrobacterium tumefaciens*, of viral origin such as the CaMV 35S terminator, or of plant origin such as a histone terminator as described in EP 0 633 317.

The selection step for identifying a transformed plant or a part thereof comprising the DNA molecule or a processed construct can be carried out via a selectable gene present in the vector, as referred to above. The selectable gene may comprise an operably linked promoter regulatory sequence and terminator regulatory sequence that are functional in plant cells.

Among the selectable markers that can be used in the present invention, reference is made to genes for resistance against antibiotics, such as the hygromycin phosphotransferase gene, the neomycin phosphotransferase II gene inducing resistance against kanamycin, or the aminoglycoside 3'-adenyltransferase gene, genes for tolerance to herbicides such as the bar gene (White et al., A cassette containing the bar gene of Streptomyces hygroscopicus: a selectable marker for plant transformation, Nucl. Acids Res., 1990, 18: 1062) for tolerance to bialaphos, the EPSPS gene (US 5,188,642) for tolerance to glyphosate or else the HPPD gene (WO 96/38567) for tolerance to isoxazoles, genes encoding identifiable enzymes, such as the GUS enzyme, GFP protein or genes encoding pigments or enzymes regulating pigment production in the transformed cells. Such selectable marker genes are in particular described in patent applications WO 91/02071, WO 95/06128, WO 96/38567, and WO 97/04103.

Marker gene free transformation is another alternative to transfer the expression cassette of interest into the plant.For introducing the DNA molecule into a plant or a part thereof, numerous methods are known in the art. A preferred method applied in the present invention is transformation of the DNA molecule, expression cassette or vector harboring the DNA molecule by the use of bacteria of the Agrobacterium genus, preferably by infection of the cells or tissues of plants with A. *tumefaciens* (Knopf U.C., 1979, Crown-gall and Agrobacterium tumefaciens: survey of a plant cell transformation system of interest to medicine and agriculture, Subcell. Biochem. 6: 143-173; Shaw C.H. et al., 1983, A general method for the transfer of cloned genes to plant cells, Gene 23(3): 315-330) or A. *rhizogenes* (Bevan M.W. and Chilton M., 1982, T-DNA of the Agrobacterium Ti and Ri plasmids, Annu. Rev. Genet. 16: 357-384; Tepfer M. and Casse-Delbart F., 1987, Agrobacterium rhizogenes as a vector for transforming higher plants, Microbiol. Sci. 4(1): 24-28). For example, the transformation of plant cells or tissues with *Agrobacterium tumefaciens* is carried out according to the protocol described by Hiei Y. et al. (1994, Efficient transformation of rice (Oryza sativa L.) mediated by Agrobacterium and sequence analysis of the boundaries of the T-DNA, Plant J. 6(2): 271-282). Another preferred method is the biolistic transformation method, wherein cells or tissues are bombarded with particles onto which the vectors of the invention are adsorbed (Bruce W.B. et al., 1989, Photoregulation of a phytochrome gene promoter from oat transferred into rice by particle bombardment, Proc. Natl. Acad. Sci. USA 86(24): 9692- 9696; Klein T.M.et al., 1992, Transformation of microbes, plants and animals by particle bombardment, Biotechnology 10(3): 286-291; US Patent No. 4,945,050). A further method is the widely used protoplast transformation. Therefor, plant cells are separated by pectinases and subsequently, the cell wall is degraded to generate protoplasts. For transformation, polyethylene glycol is added or electroporation is applied. Other methods are bringing the plant cells or tissues into contact with polyethylene glycol (PEG) and the vectors of the invention (Chang S. and Cohen S.N., 1979, High frequency transformation of Bacillus subtilis protoplasts by plasmid DNA, Mol. Gen. Genet. 168(1): 111-115; Mercenier A. and Chassy B.M., 1988, Strategies for the development of bacterial transformation systems, Biochimie 70(4): 503-517). Electroporation is another method, which consists in subjecting the cells or tissues to be transformed and the vectors of the invention to an electric field (Andreason G.L. and Evans G.A., 1988, Introduction and expression of DNA molecules in eukaryotic cells by electroporation, Biotechniques 6(7): 650-660; Shigekawa K. and Dower W.J., 1989, Electroporation: a general approach to the introduction of macromolecules into prokaryotic and eukaryotic cells, Aust. J. Biotechnol. 3(1): 56-62). Another method consists in directly injecting the vectors into the cells or the tissues by microinjection (Gordon and Ruddle, 1985, DNA-mediated genetic transformation of mouse embryos and bone marrow -- a review, Gene 33(2): 121-136). Those skilled in the art will choose the appropriate method according to the nature of the plant to be transformed and the fungus against which the plant is to be rendered resistant.

Cells or tissues of plants, e.g., root cells grown in culture, can be transformed with the desired gene and grown into mature plants. When transformation is effective, the transgene will be incorporated into the pollen and eggs and passed onto the next generation.

In one embodiment, the DNA molecule or expression cassette is stably integrated into the genome of the transgenic plant, preferably into a chromosome of the plant. Integration can, however, also occur into an extrachromosomal element. By stable integration into the genome of a plant, the DNA sequences can be passed to subsequent generations of the transgenic plant. Alternatively, the DNA molecule or expression cassette is present within the plant cell on the vector used to introduce the DNA molecule and is not stably integrated into the genome of the plant. Therefore, the DNA sequences may not be passed to subsequent generations of the plant.

According to the invention, all plants and all parts of a plant can be treated according to the methods of the present invention. By plants is meant all plants and plant populations such as desirable and undesirable wild plants, and cultivars. Plants can be plants obtained by conventional propagation and breeding methods which can be assisted or supplemented by one or more biotechnological methods such as by use of double haploids, protoplast fusion, random or directed mutagenesis, molecular or genetic markers or by bioengineering or genetic engineering methods.

The term "part of a plant" refers to any parts or organs of a plant such shoot vegetative organs/structures, e.g., leaves, stems or tubers; roots, flowers or floral organs/structures, e.g. bracts, sepals, petals, stamens, carpels, anthers or ovules; seed, including embryo, endosperm or seed coat; fruit or the mature ovary; plant tissue, e.g. vascular tissue or ground tissue; or cells, e.g. guard cells, egg cells or trichomes; or progeny of the same. The term "cell" refers to a cell or cell accumulation within the plant as well as to an isolated cell or isolated cell accumulation. A cell may have a cell wall or may be a protoplast. In particular, the present invention relates to a seed which comprises the DNA, expression cassette or vector as comprised by the present invention. Preferably, the seeds of a transgenic plant retain the DNA, expression cassette or vector as comprised by the invention, so that the new plants generated from a seed continues to comprise the DNA, expression cassette or vector,

Plants that can be protected by the method according to the invention comprise all plants, preferably plants of economic interest, more preferably the plant according to the present invention is selected from the group consisting of barley *(Hordeum vulgare*), sorghum (*Sorghum bicolor*), rye (*Secale cereale),* Triticale, sugar cane (*Saccharum officinarium*), maize *(Zea mays),* foxtail millet (*Setaria italic),* rice (*Oryza sativa), Oryza minuta*, *Oryza australiensis*, *Oryza alta,* wheat (*Triticum aestivum*), *Triticum dorum, Hordeum bolbosum,* purple false brome (*Brachypodium distachyon*), sea barley *(Hordeum marinum),* goat grass *(Aegilops tauschii),* apple *(Malus domestica*), strawberry, sugar beet (*Beta vulgaris),* sunflower (*Helianthus annuus*), Australian carrot (*D*a*ucus glochidiatus),* American wild carrot (*Daucus pusillus*), *Daucus muricatus,* carrot (*Daucus carota*), eucalyptus (*Eucalyptus grandis), Erythranthe guttata*, *Genlisea aurea*, woodland tobacco (*Nicotiana sylvestris*), tobacco (*Nicotiana tabacum), Nicotiana tomentosiformis,* tomato (*Solanum lycopersicum),* potato (*Solanum tuberosum*), coffee (*Coffea canephora),* grape vine *(Vitis vinifera*), cucumber (*Cucumis sativus*), mulberry (*Morus notabilis*), thale cress (*Arabidopsis thaliana), Arabidopsis lyrata*, sand rock-cress *(Arabidopsis arenosa*), *Crucihimalaya himalaica*, *Crucihimalaya wallichii,* wavy bittercress (*Cardmnine flexuosa),* peppergrass (*Lepidium virginicum*), sheperd's-purse (*Capsella bursa*-*pastoris*), *Olmarabidopsis pumila,* hairy rockcress *(Arabis hirsuta*), rape (*Brassica napus*), broccoli *(Brassica oleracea*), *Brassica rapa*, *Brassica juncacea*, black mustard (*Brassica nigra*), radish *(Raphanus sativus*), *Eruca vesicaria sativa,* orange (*Citrus sinensis*), *Jatropha curcas*, cotton (*Gossipium sp.),* soybean (*Glycine max),* and black cottonwood (*Populus trichocarpa).* Particularly preferred, the plant is selected from the group consisting of barley (*Hordeum vulgare*), sorghum (*Sorghum bicolor*), rye (*Secale cereale),* Triticale, sugar cane (*Saccharum officinarium),* maize *(Zea mays*), rice *(Oryza sativa),* wheat *(Triticum aestivum*), *Triticum durum*, *Avena sativa, Hordeum bulbosum*, sunflower (*Helianthus annuus*), carrot *(Daucus carota*), tobacco (*Nicotiana tabacum*), tomato (*Solanum lycopersicum),* potato *(Solanum tuberosum),* coffee *(Coffea canephora),* grape vine *(Vitis vinifera*), cucumber *(Cucumis sativus*), thale cress *(Arabidopsis thaliana*), rape (*Brassica napus*), broccoli *(Brassica oleracea*), *Brassica rapa*, *Brassica juncacea,* black mustard (*Brassica nigra),* radish *(Raphanus sativus*), cotton (*Gossipium sp*.) and soybean (*Glycine max*).

The invention concerns a method of producing a transgenic plant or a part thereof, comprising the steps of introducing into at least a cell of the plant the DNA or the expression cassette or the vector as comprised by the invention, and regenerating the transgenic plant from the at least one cell.

As used herein "regenerating" or "regeneration" means a process of growing an entire plant from a single cell, a group of cells, a part of the plant or a tissue of the plant. The skilled person knows methods of introducing DNA into at least a cell of the plant and growing a plant therefrom. "At least a cell" means a single cell, a group of cells, a part of the plant or a tissue of the plant.

Furthermore, the invention concerns a method of conferring fungal resistance on a plant or a part thereof comprising the steps of introducing into the plant or the part thereof the DNA or the expression cassette or the vector as comprised by the invention, and causing expression of the DNA or the expression cassette.

As used herein, the term "causing expression" means that under the conditions, under which the plant is kept and/or cultivated, transcription of the DNA having been introduced into the plant is induced. For example, if the promoter is an inducible promoter, the activity of such promoter can be induced by the presence or absence of specific biotic or abiotic factors, according to the choice of the user of the present invention. If the promoter is a constitutive promoter, expression continuously occurs.

Furthermore, the invention concerns a method of inhibiting the expression of the gene(s) involved in the GPI anchor biosynthesis pathway in a fungus, comprising applying the DNA or the expression cassette or the vector as comprised by the invention to the fungus or to a plant or a part thereof.

As used herein "applying ... to the fungus or to a plant or a part thereof" or a similar term means that the DNA, expression cassette or vector as comprised by the invention are administered to the fungus, plant or part thereof so that the fungus, plant or part thereof incorporate and express the DNA. The application on the plant may be in the laboratory using any methods of introducing the DNA, expression cassette or vector, as referred to above, into the plant or the part thereof. The application on the plant may also be in the field in order to render the plant which may be infected by a phyto-pathogenic fungus resistant against the fungus. The application may also be directly on the fungus in order to cause cessation of infection, growth, development, reproduction and/or pathogenicity and eventually death of the fungus. "Directly" means that the DNA, expression cassette or vector is applied on the fungus, so that the DNA, expression cassette or vector is introduced into the fungus without plant involvement. The fungus may have already infected the plant or may still be present outside the plant such as in the soil. The application in the field may occur by any method known in the art of applying a fungicide, such as spraying or splashing.

Furthermore, the invention concerns the use of the DNA or the expression cassette or the vector as comprised by the invention for inactivating a fungus, while contacting a plant or a part thereof; for protecting a plant against an infection by a fungus; or for inhibiting the expression of the gene involved in the GPI anchor biosynthesis pathway, preferably the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably the GPI12, GAA1, GPI8 and/or GWT1 gene(s), in a fungus.

As used herein, "inactivating a fungus" means causing cessation of infection, growth, development, reproduction and/or pathogenicity and eventually death of the fungus. Inactivation can occur by RNA molecules directed against the gene or mRNA involved in the GPI anchor biosynthesis pathway of the fungus produced by the transgenic plants. Alternatively, inactivation occurs by applying the DNA, expression cassette or vector as comprised by the present invention onto the fungus outside the plant, such as in the field. Inactivation of the fungus is obtained by reduction of the expression of the gene or mRNA involved in the GPI anchor biosynthesis pathway of the fungus, resulting in the reduction of the damages of the plant.

As used herein, "while contacting a plant" means that the fungus is in touch with the plant. Thus, the fungus is forming or has already formed structures to invade into a cell of the plant. This means that differentiated structures of the fungus such as an appressorium are forming or has already formed and is penetrating or has already penetrated into a cell of the plant.

As used herein, "protecting a plant" means conferring resistance against the fungus on the plant. A resistant plant is not infested and/or damaged by a fungus or is infested and/or damaged by a fungus to a lower extent as compared to a plant of a similar phenotype or the same genotype which is not treated according to the present invention. By introducing a DNA capable of expressing an inhibitory nucleic acid into a plant or a part thereof, resistance against a fungus harboring the gene(s) involved in the GPI anchor biosynthesis pathway, against which the inhibitory molecule is directed, is obtained, so that the plant is protected from the fungus. To determine the resistance, the transgenic plant may be compared with a control plant which has a similar phenotype or the identical genotype, however, does not contain the transgene. Resistance can be determined using an optical score wherein scores from non-resistant, if the symptoms of the transgenic plant correspond to those of the non-transgenic plant, to highly resistant, if no symptoms of fungus infection are seen, may be awarded. Alternatively, resistance can be determined by determining the transcript amount(s) of the RNA(s) involved in the GPI anchor biosynthesis pathway in the fungus during infection of a transgenic plant, as compared to the transcript amounts of the fungus of identical genotype infected on a plant of identical genotype, which does not contain the transgene of the present invention, or in the fungus of same genotype which does not infect a plant. The present inventors have found that, dependent on the amount of transcript reduction, vegetative fungal growth, asexual spore formation, formation of fungal infection structures, and infection rates are impaired, and plant resistance is enhanced. Reduction of the transcript amounts of RNA(s) involved in the GPI anchor biosynthesis pathway by about 40 % was sufficient to see an effect on the parameters, cited above. Thus, transcript reduction of at least 40 %, 50 %, 60 %, 70 %, 80 %, 90 %, 95 % or of 100 % for the transcript(s) involved in the GPI anchor biosynthesis pathway allows scoring the transgenic plant as being resistant or as having resistance against a fungus. Preferably, the damages on the plant are reduced by at least 50 %, 60 %, 70 %, 80 %, 90 % or by 100 % in a resistant transgenic plant according to the present invention as compared to a plant of similar phenotype or the identical genotype, which does not contain the transgene of the present invention.

The invention also concerns a composition comprising the DNA molecule capable of expressing inhibitory nucleic acid molecules capable of inhibiting the expression of a gene involved in the GPI anchor biosynthesis pathway involved in the GPI anchor biosynthesis pathway in a fungus, a composition comprising an expression cassette comprising the DNA molecule, a composition comprising a vector comprising the DNA molecule or a composition comprising dsRNA capable of inhibiting the expression of a gene involved in the GPI anchor biosynthesis pathway in a fungus. The composition may be for external application on a plant and/or a fungus.

Alternatively, the composition may contain dsRNA, wherein one strand of this RNA comprises an antisense sequence which is substantially complementary to the gene involved in the GPI anchor biosynthesis pathway and the other strand comprises a sense sequence which is substantially complementary to the antisense sequence. The dsRNA may be an hpRNA, miRNA or siRNA. The definitions of dsRNA, sense and antisense sequences are given above. When the composition is applied to a plant and/or to a fungus from the outside, such as in the field, the dsRNA may be incorporated into the plant and/or the fungus and may inhibit the expression of a gene involved in the GPI anchor biosynthesis pathway in the fungus via gene silencing such as via the antisense mechanism or the RNAi mechanism.

Double-stranded RNA for the manufacture of the composition in accordance with the invention can be produced in vitro using methods known to the skilled person. Thus, the dsRNA may be synthesized by genetic engineering methods from a DNA or by biosynthesis methods.

The composition in accordance with the invention may be used as a fungicide for a plant or a part thereof which is already infected by a fungus or will be potentially infected. The composition may also be used against the fungus, which has already infected the plant or which has not infected the plant, for example when the fungus is still outside the plant, for example, in the soil. In this regard, the composition is used to control the growth of the pathogenic fungus either by the treatment of a plant or by the application on the fungus. The skilled person knows fungicide compositions and knows which further ingredients such as carrier substrates, whereby the carrier substrate has, for example, an RNA-stabilizing effect, to include into the composition and what methods to use in order to prepare and apply the composition to a plant and/or fungus in the field. The composition in accordance with the invention may furthermore be used as a pre-treatment for seed.

The invention is further explained in the following figures and examples which are included for illustration purposes and are not intended to limit the invention.

### Figures

Figure 1: The main pathway of GPI biosynthesis in yeast. The pathway is composed of 12 biosynthetic steps. The involved yeast genes are shown (inspired by Pittet and Conzelmann, 2007).
Figure 2: Similarity of GPI12, GAA1 and GPI8 genes from different fungal origin ascomycota include *F. fujikuroi, F. oxysporum*, *F. graminearum, F. solani*, *T. virens*, *B. bassiana, N. crassa*, *M. oryzae, C. gloeosporioides, C. graminicola, C. higginsianum*, *C. purpurea, G. graminis, N. crassa*, yeasts include *P. brasiliensis*, *C. glabrata*, *S. cerevisiae, S. arboricola*, *S. pombe*, *C. albicans*, basidiomycota include *B. graminis*, *P. graminis*, *T. stipitatus*, *R. solani, L. bicolor*, *T. marneffei*, *U. hordei*, *U. maydis.*

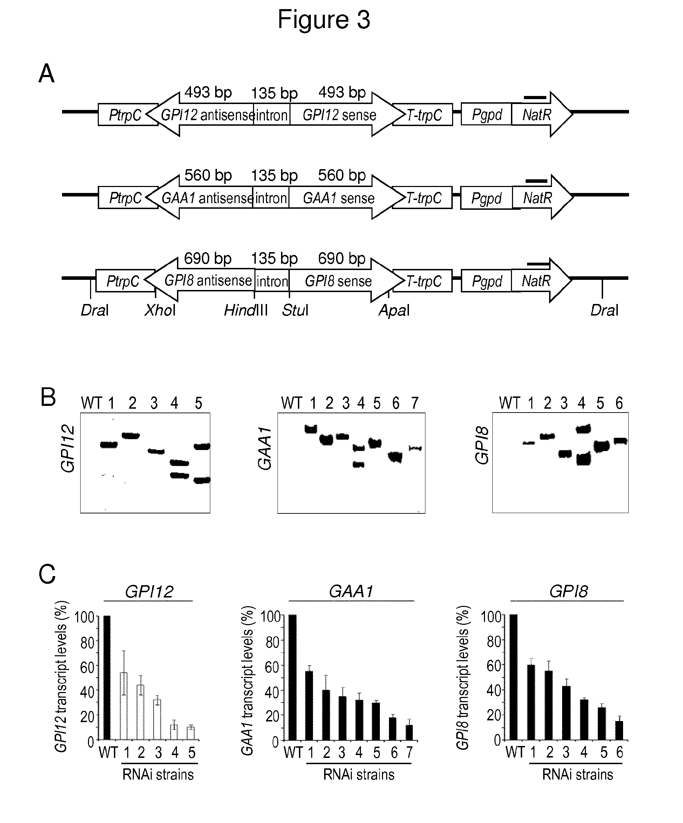

Figure 3: Reduction of GPI12, GAA1 and GPI8 transcript accumulation in the fungal pathogen *Colletotrichum graminicola* by RNAi; A: RNAi constructs designed to reduce GPI12, GAA1 and GPI8 transcripts in *C. graminicola;* B: Determination of the copy number of transformed constructs in transformants of *C. graminicola.* WT = wild type; C: Reduced GPI12, GAA1 and GPI8 transcript accumulation in the RNAi strains as shown by qRT-PCR.

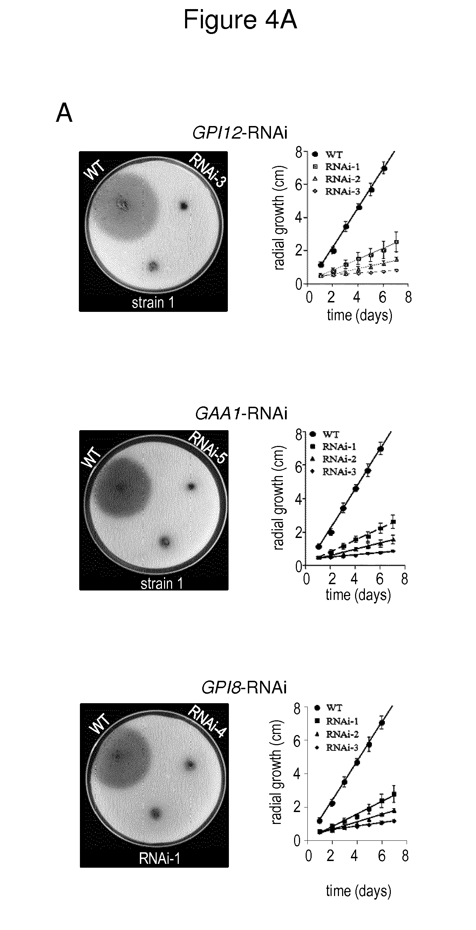

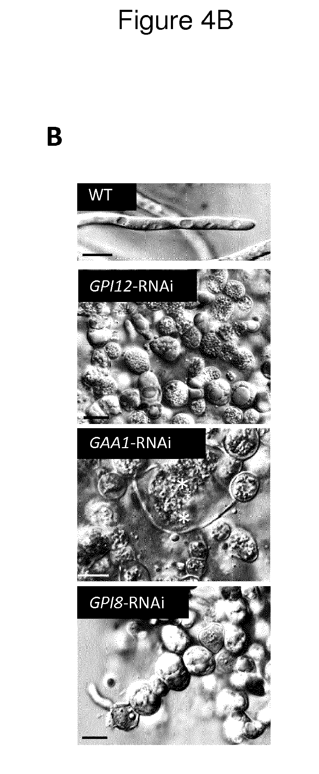

Figure 4: Reduced transcript levels of GPI12, GAA1 and GPI8 impair the vegetative growth of the fungal pathogen Colletotrichum graminicola; A: Reduction of vegetative growth of GPI12, GAA1 and GPI8 RNAi strains in a vegetative growth assay dependent from the transcript abundance (see Fig. 3); B: Malformation of fungal hyphae in GPI12, GAA1 and GPI8 RNAi strains as shown by microscopical analysis. WT = wild type.

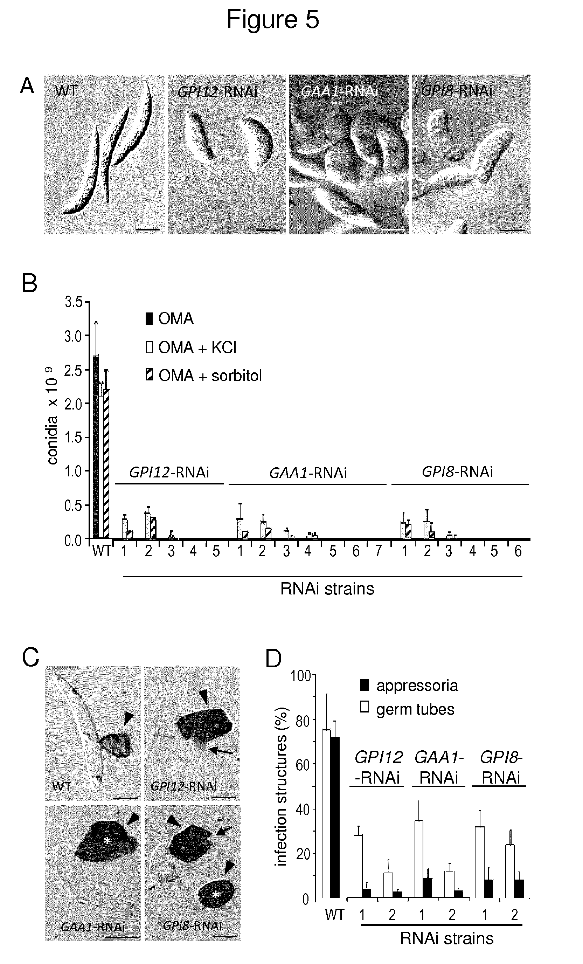

Figure 5: Reduced transcript levels of GPI12, GAA1 and GPI8 impair asexual spore formation and spore germination; A: Malformation of conidia from GPI12, GAA1 and GPI8 RNAi strains on oat meal agar (OMA) supplemented with 1 M sorbitol. WT = wild type; B: Absence of conidia (non-stabilized medium) or strongly reduced conidia formation (osmotically stabilized medium, 0.5 M KCl or 1 M sorbitol) of GPI12, GAA1 and GPI8 RNAi strains in comparison to the wild type (WT); C: Appressoria of GPI12, GAA1 and GPI8 RNAi strains explode on an artificial surface; D: Reduced germination and infection structure (appressoria) formation of GPI12, GAA1 and GPI8 RNAi strains compared to the wild type (WT).

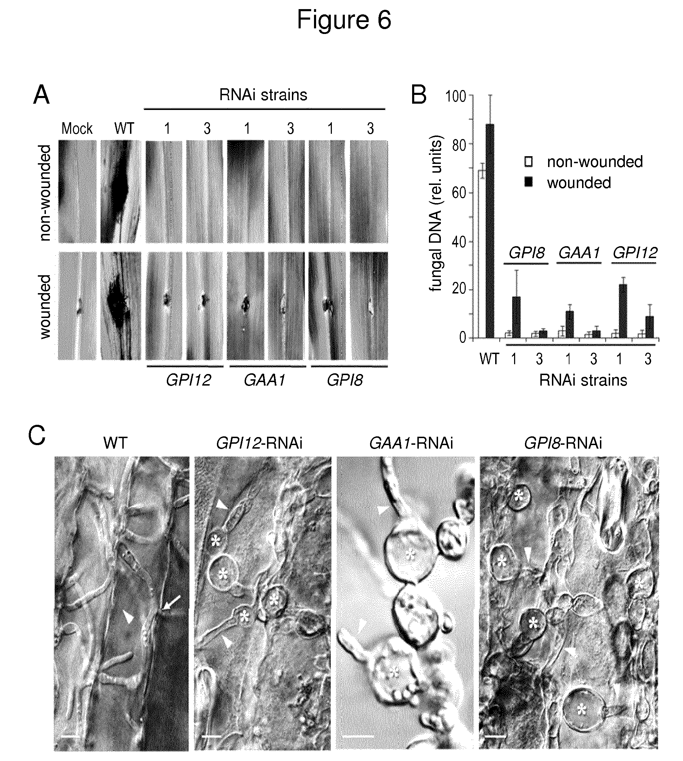

Figure 6: Reduced transcript levels of GPI12, GAA1 and GPI8 result in reduced infection rates on corn leaves; A: Loss of pathogenicity of GPI12, GAA1 and GPI8 RNAi strains on intact corn leaves and strong reduction of virulence on wounded leaves; B: Strong reduction of RNAi strain fungal biomass in infected corn leaves as shown by qPCR; C: Severely distorted infections hyphae of the GPI12, GAA1 and GPI8 RNAi strains at the inoculation site on wounded corn leaves.

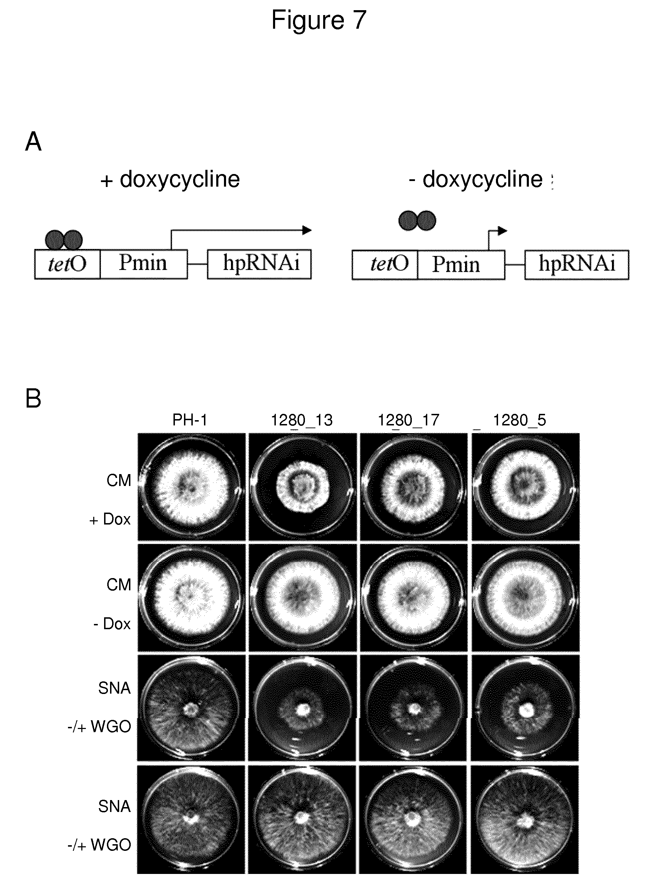

Figure 7: The GWT1 gene is important for the growth of Fusarium graminearum as shown by the chemical induction of an RNAi GWT1 construct; A: Design of the chemical inducible fungal promoter: The tetracyclin dependent transactivator rtTA binds in the presence of doxyzyclin (Dox) at the tet0 promoter and allows the expression of the coding region. The promoter is silent in the absence of doxycycline; B: Growth retardation of the Fusarium transformants 1280_13, 1280_17 and 1280_5 after Dox induction of a transformed RNAi GWT1 construct.

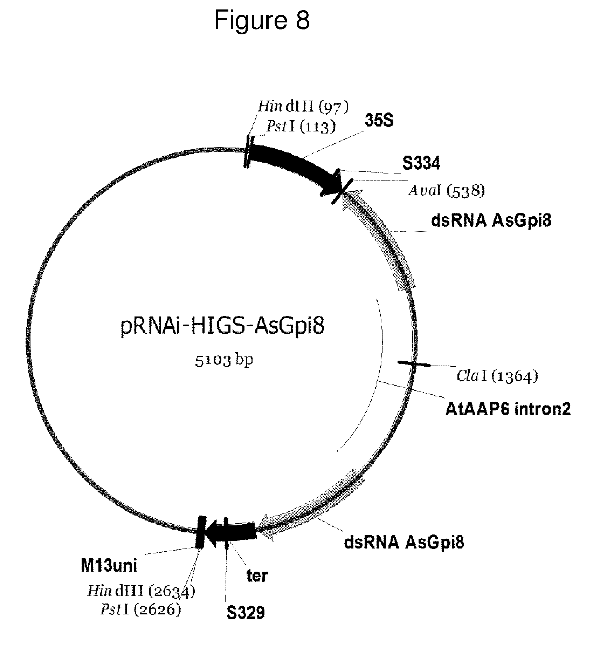

Figure 8: Hairpin RNAi vector pRNAi-HIGS-AsGpi8 designed for the generation of HIGS GPI8 potato lines. The GPI8 sequence of *Alternaria solani* was inserted into a hairpin RNAi-construct. The 2 identical fragments of the GPI gene (dsRNA AsGPI8) are orientated in a forward and a reverse orientation and are separated by the intron 2 of the AtAAP2 gene of *Arabidopsis thaliana.* 35S = promoter of the 35S CaMV gene, A, ter = terminator of the CaMV 35S gene, AtAAP2 intron = intron of the AAP2 gene.

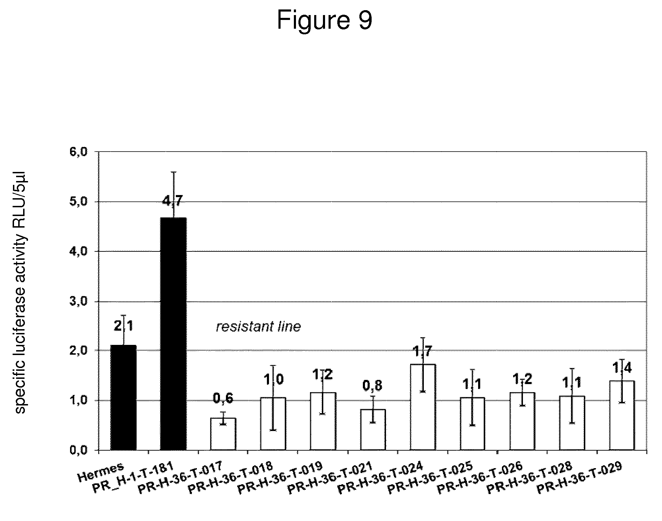

Figure 9: Detection of GPI8 transcriptional silencing activity in HIGS_{GPI8} lines. The transcriptional silencing activity of line PR-H-36-T-017 was the highest in comparison to other HIGS _{GPI8} potato lines.

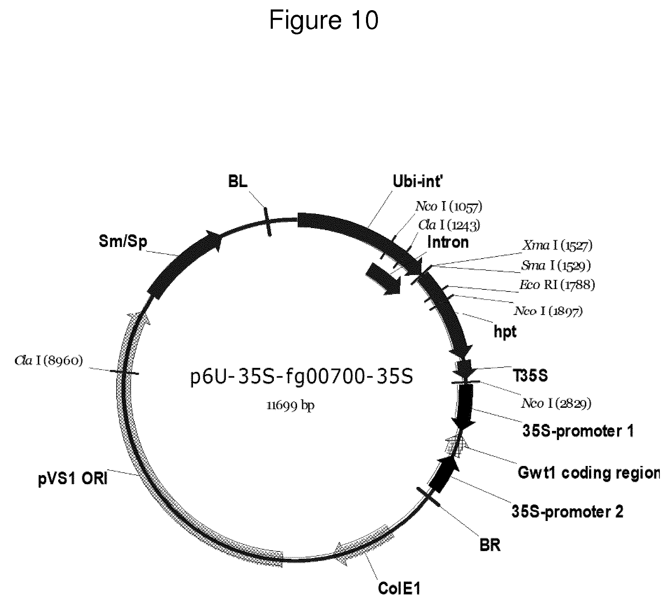

Figure 10: Double promoter RNAi binary vector p6U-35S-fg00700-35S designed for the generation of HIGS _{GWT1} wheat lines. The GWT1 sequence of *Fusarium graminearum* was inserted into a double promoter RNAi-construct. In the double promoter construct the HIGS target gene is flanked by two 35S promotors in a forward and a reverse orientation which will result in the biosynthesis of a sense and an antisense transcript. Ubi-int = ubiquitin promoter, hpt = Hygromycin resistance gene, T35S = terminator of the CaMV 35S gene, BR = right border of T-DNA BL = left border of T-DNA, Sm/Sp = streptomycin selection marker.

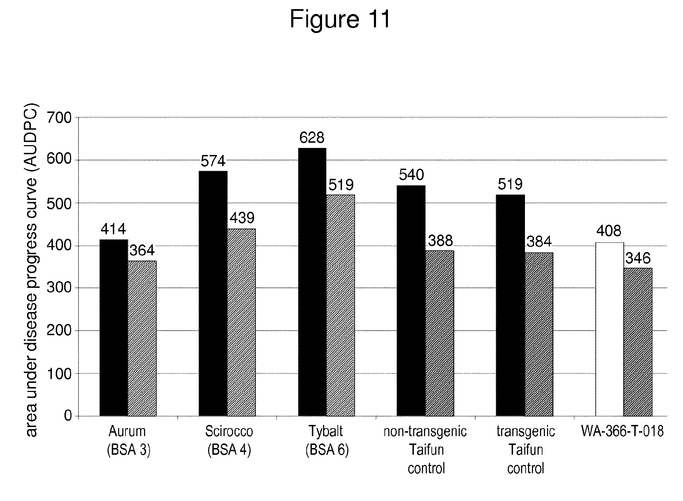

Figure 11: Enhanced Fusarium resistance and reduced DON content of the HIGS _{GWT1} wheat line. The HIGS _{GWT1} wheat line WA336-T-018 showed a reduced AUDPC (18 d.p.i) value (filled columns) in comparison to the transformation genotype Taifun and a transgenic Taifun control. The transgenic control was transformed with a reporter gene construct which should not influence resistance. The disease score was calculated as mean from 26 or 30 infected heads. The concentration of the mycotoxin DON (striped columns) was also reduced in the infected heads of the HIGS line in comparison to Taifun lines as shown by ELISA. The reference lines, Aurum, Scirocco and Tybalt, are also shown. Aurum was the wheat cultivar with the best Fusarium resistance available in Germany. BSA (Bundessortenamt, Germany) scores describe the resistance of these lines in the field (1-9).

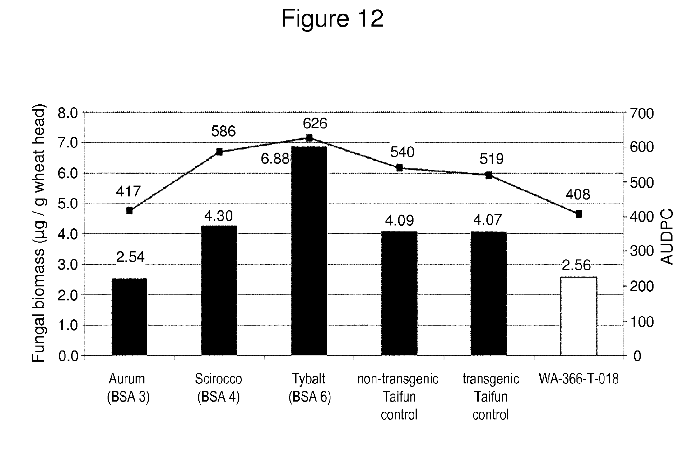

Figure 12: Enhanced Fusarium resistance and reduced fungal biomass of the HIGS _{GWT1} wheat line. The HIGS _{GWT1} wheat line WA336-T-018 showed a reduced AUDPC (18 d.p.i) value (line) in comparison to the transformation genotype Taifun and a transgenic Taifun control. The transgenic control was transformed with a reporter gene construct which should not influence resistance. The AUDPC value was calculated as mean from the infected heads used for the determination of the fungal biomass. The fungal biomass (columns) was also reduced in the infected heads of the HIGS line in comparison to Taifun lines as shown by qPCR. The reference lines, Aurum, Scirocco and Tybalt, are also shown. Aurum was the wheat cultivar with the best Fusarium resistance available in Germany. Since Aurum has some negative agronomical properties the cultivation is very limited. BSA scores describe the resistance of these lines in the field (1-9).

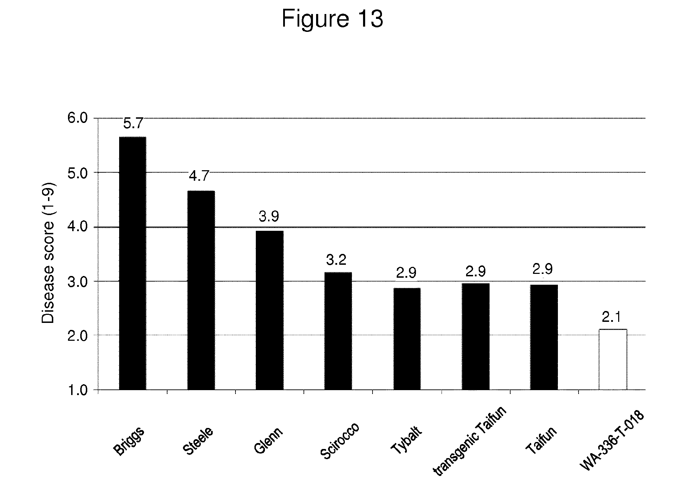

Figure 13: Enhanced Fusarium head scab resistance of the HIGS line WA-336-T-018 at field trials in the USA. Shown is the mean of disease severity (1-9) at two locations. 1 = healthy heads, 9 = 100% bleached heads.

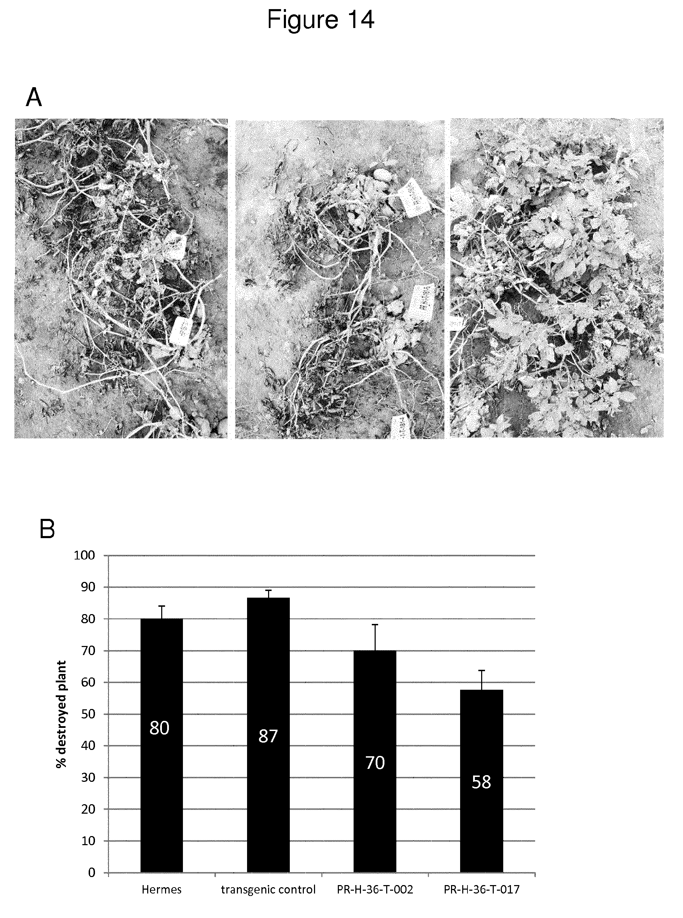

Figure 14: Enhanced early blight resistance of the HIGS potato line PR-H-36-T-017 as shown under near field conditions at field trials in Germany: A: Shown are plants of the plots at the end of the trial. Hermes = genotype used for transformation (left), transgenic control HIGS_{GFP} used as negative control (central), PR-H-36-T-017 = HIGS_{GPI8} line showing enhanced *Alternaria solani* (early blight) resistance (right); B: Disease scores of early blight assay 50 days after inoculation (mean of 3 plots with 6 plants each). The transformation genotype Hermes and the transgenic control, which was transformed with an RNAi construct against the green fluorescence protein (GFP) showed a destruction of the plants by 80 and 87%, respectively. The HIGS_{GPI8} lines PR-H-36-T-002 and PR-H-36-T-017 showed only a plant damage of 70 and 58%.

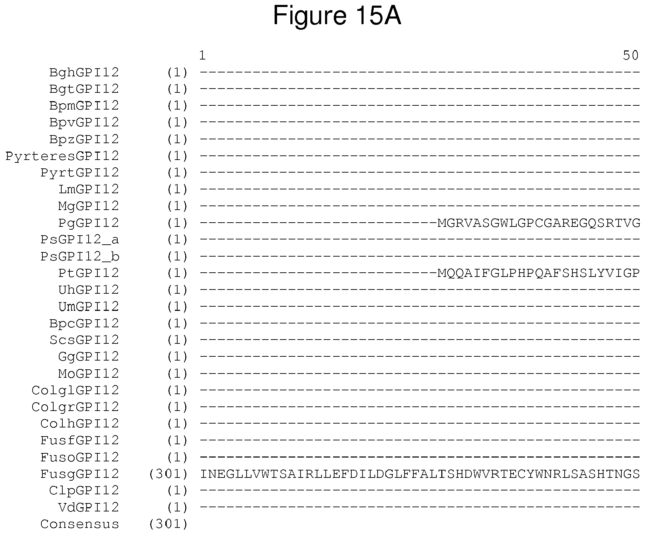

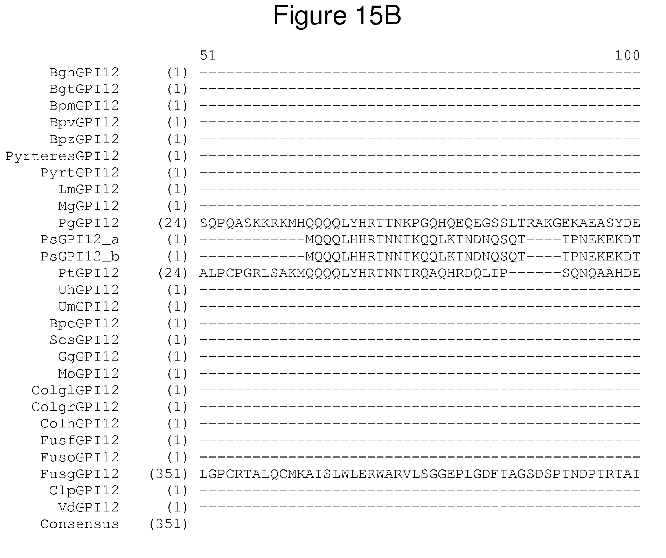

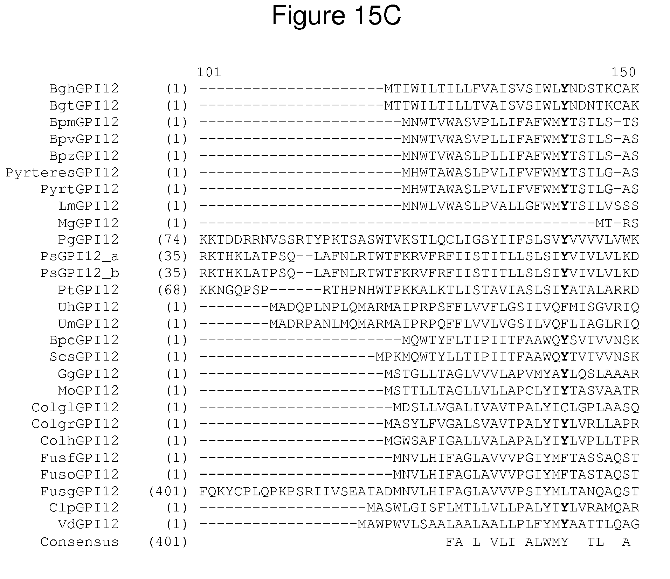

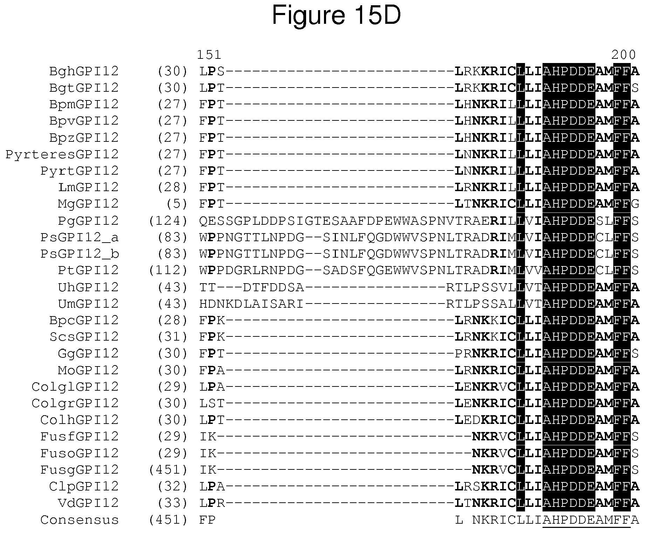

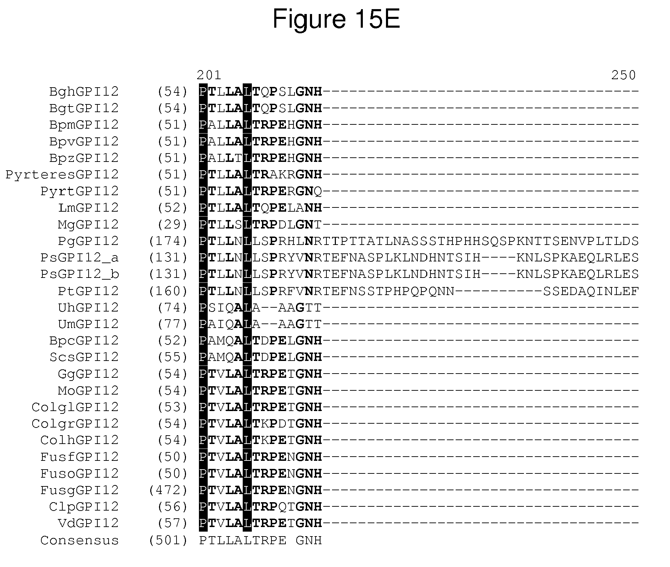

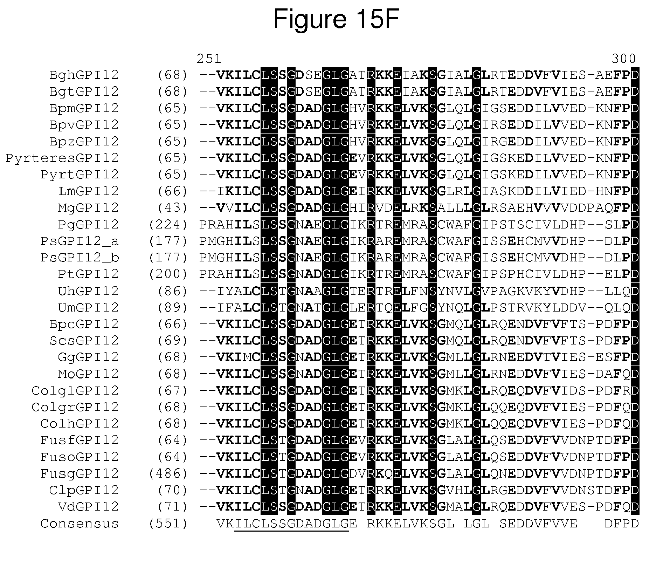

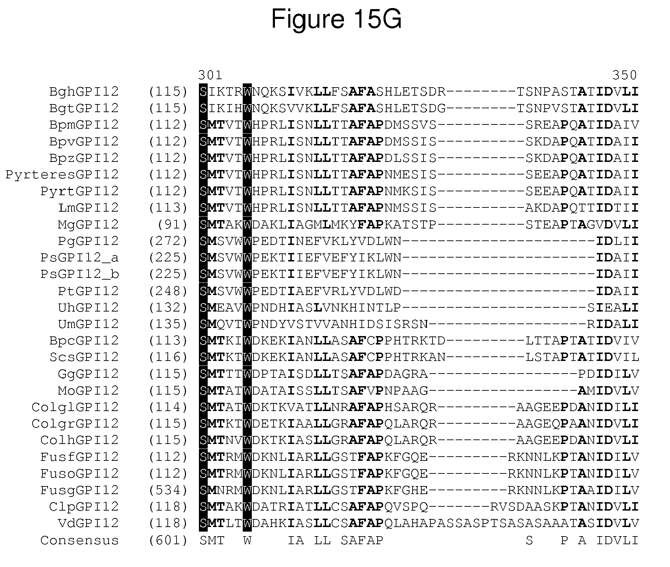

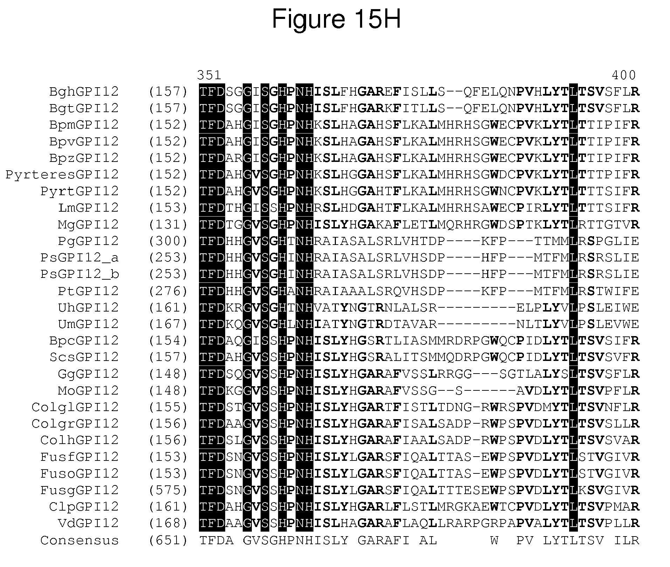

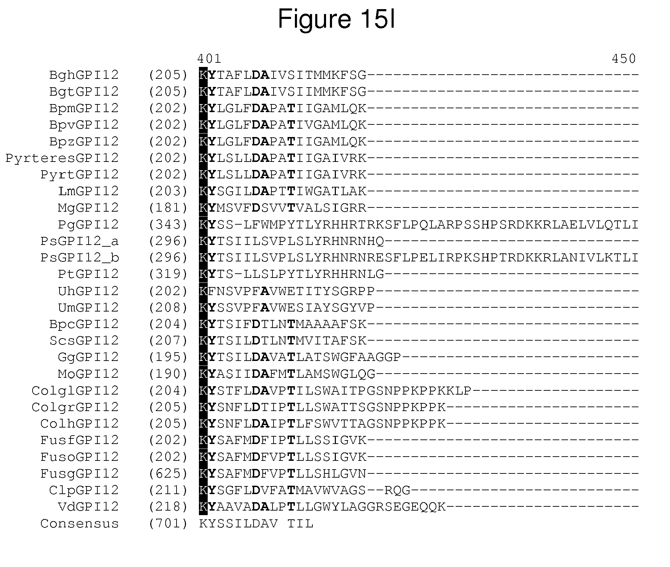

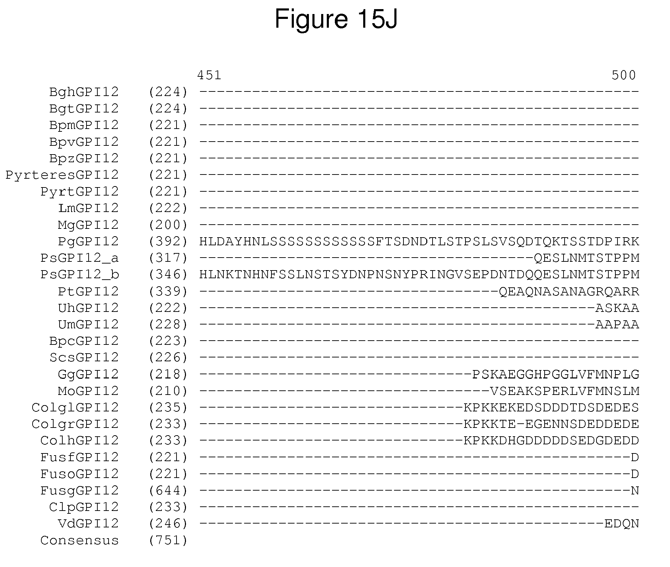

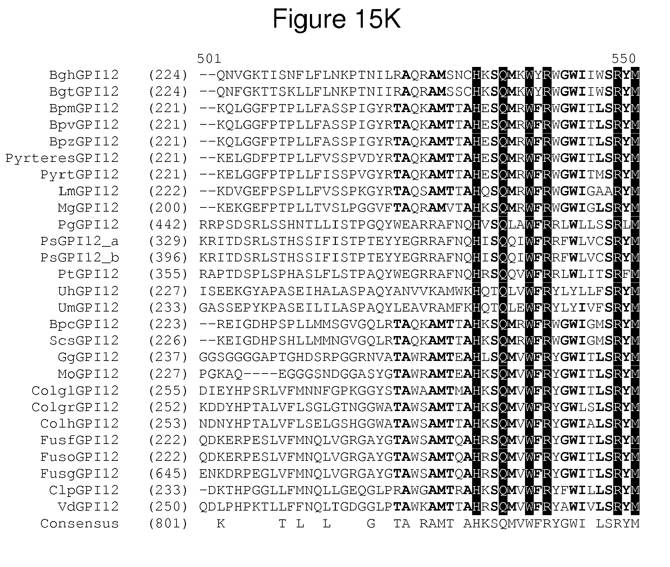

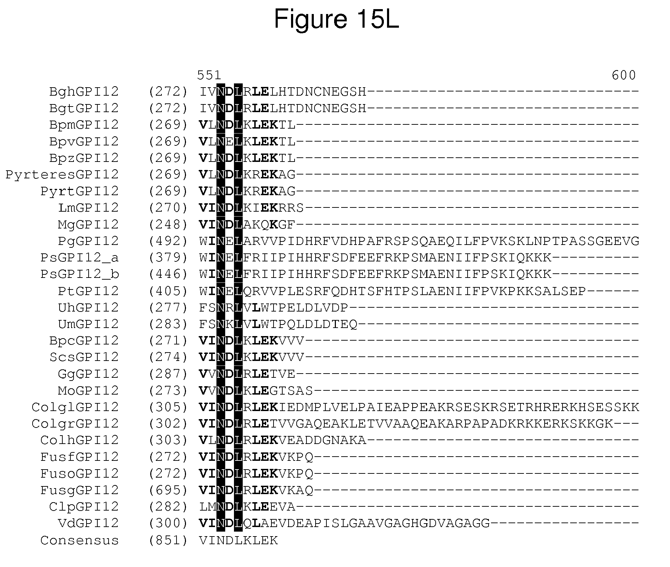

Figure 15 A-L: GPI12 protein alignment: Identification of conserved amino acid sequences in the GPI12 proteins of several fungal pathogens *(Blumeria graminis f. sp. hordei* (Bgh), *Blumeria graminis f. sp. tritici* (Bgt), *Bipolaris maydis* (Bpm), *Bipolaris victoriae* (Bpv), *Bipolaris zeicola* (Bpz), *Pyrenophora tritici-repentis* (Pyrteres), *Pyrenophora teres f. teres* (Pyrt), *Leptosphaeria maculans* (Lm), *Mycosphaerella graminicola* (Mg), *Puccinia graminis tritici* (Pg), *Puccinia striiformis* (Ps), *Puccinia triticina* (Pt), *Ustilago hordei* (Uh), *Ustilago maydis* (Um), *Sclerotinia sclerotiorum* (Scs), *Gaeumannomyces graminis var. tritici* (Gg), *Magnaporthe oryzae* (Mo), *Colletotrichum gloeosporioides* (Colgl), *Colletotrichum graminicola* (Colgr), *Colletotrichum higginsianum* (Colh), *Fusarium fujikuroi* (Fusf), *Fusarium oxysporum* (Fuso), *Fusarium graminearum* (Fusg), *Claviceps purpurea* (Clp), *Verticillium dahliae* (Vd)). Selected conserved regions are underlined.

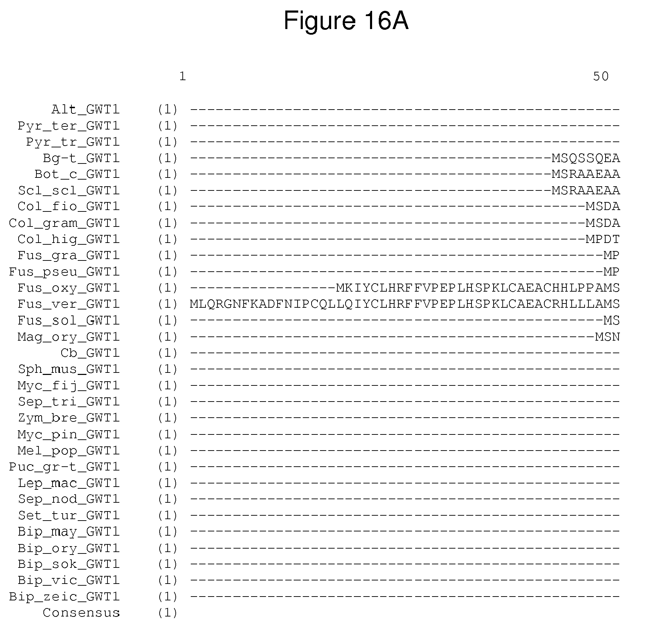

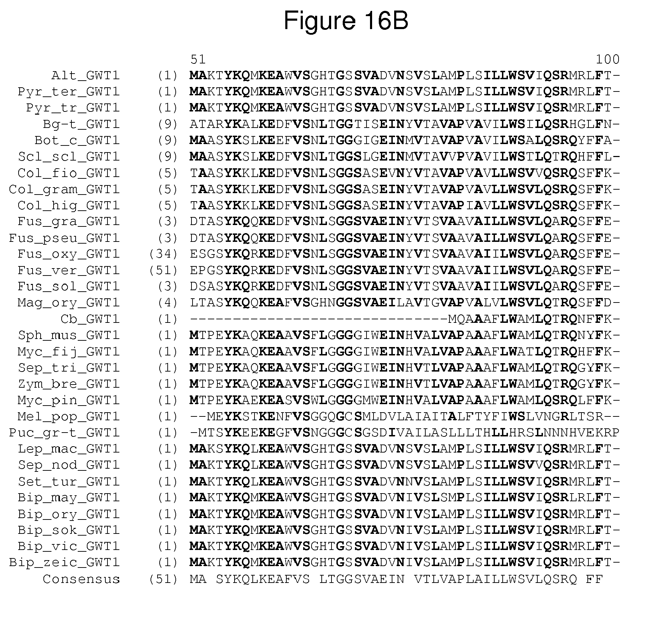

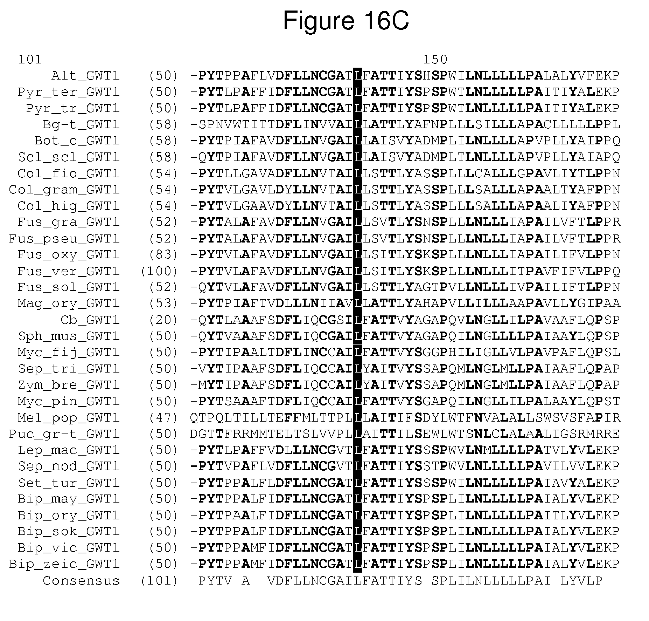

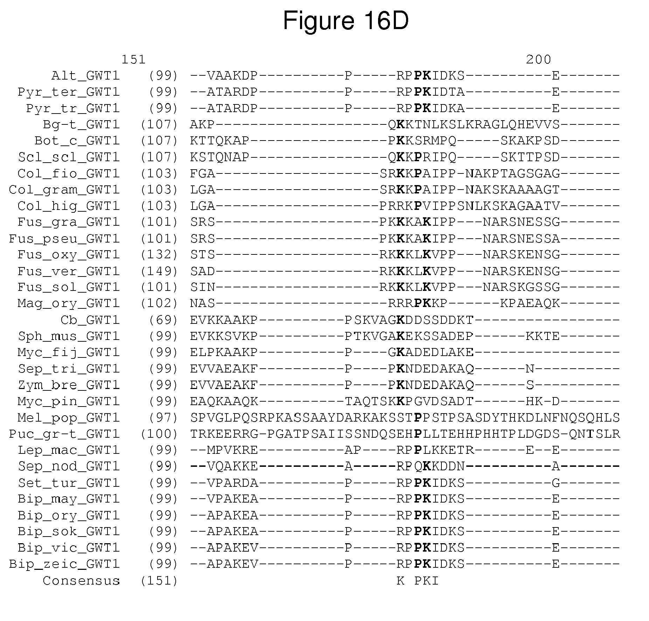

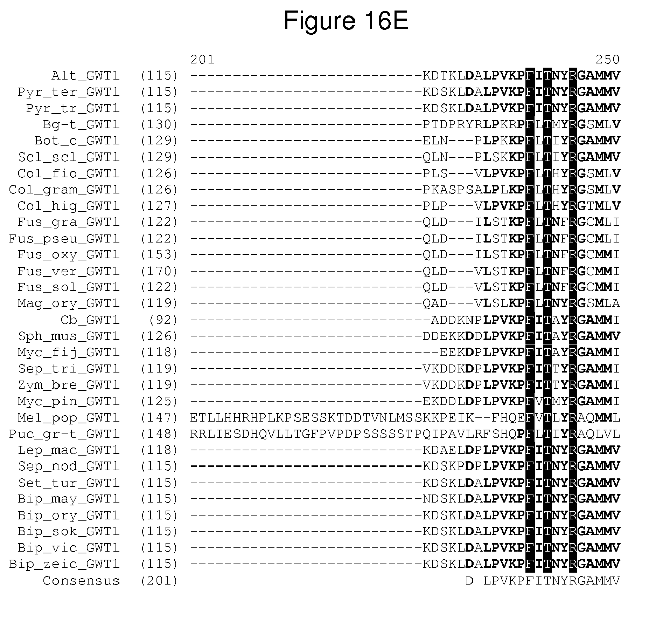

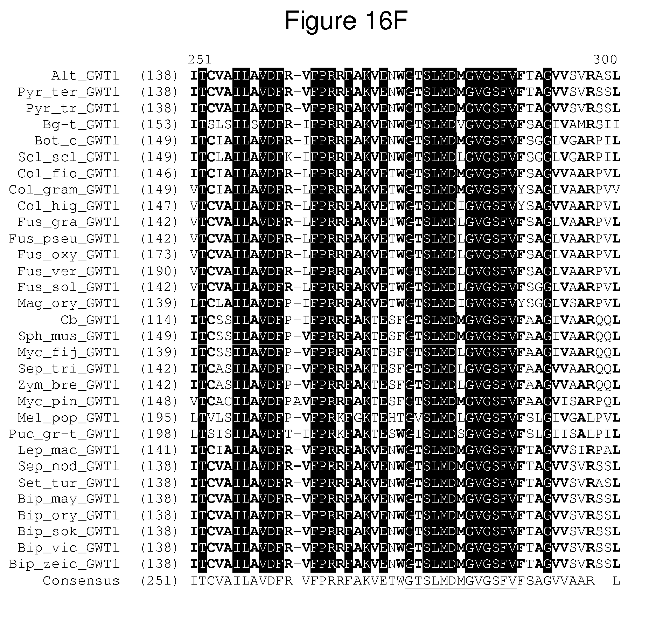

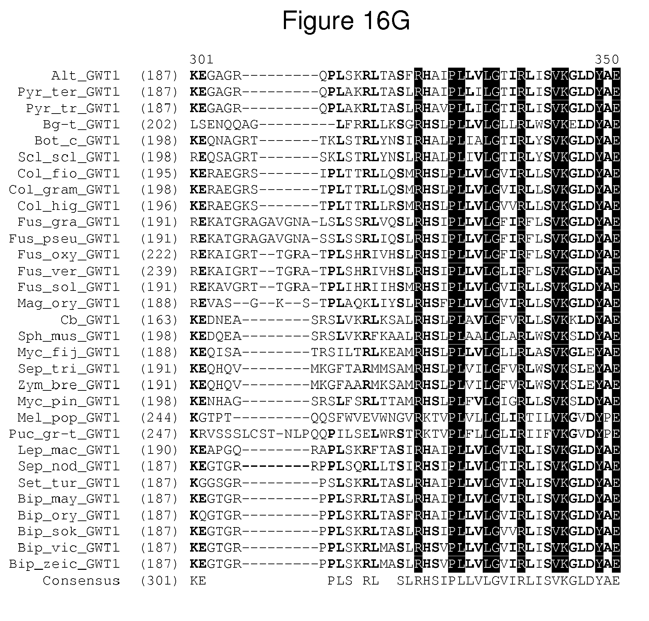

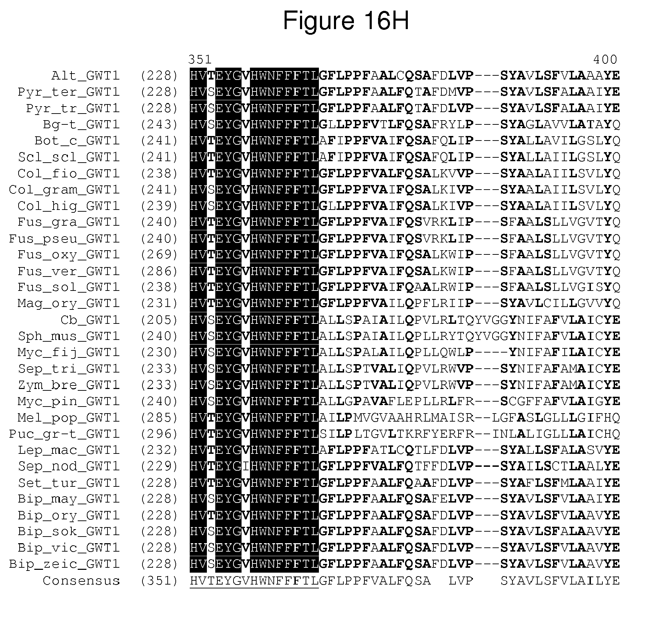

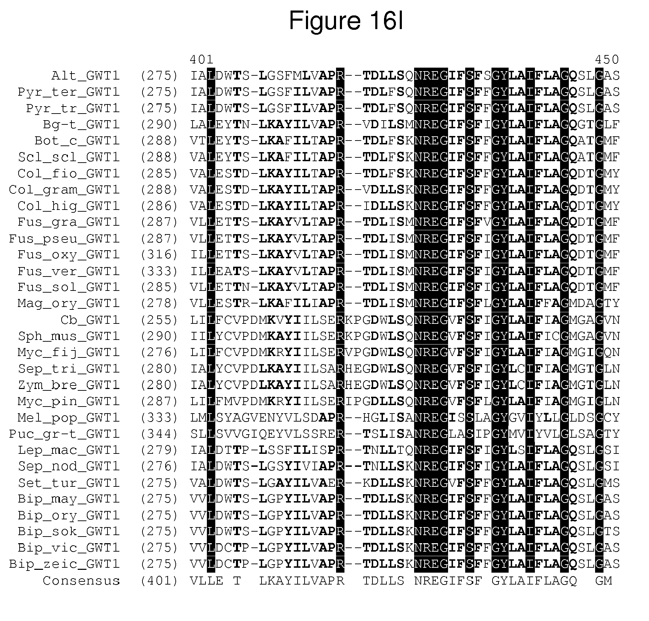

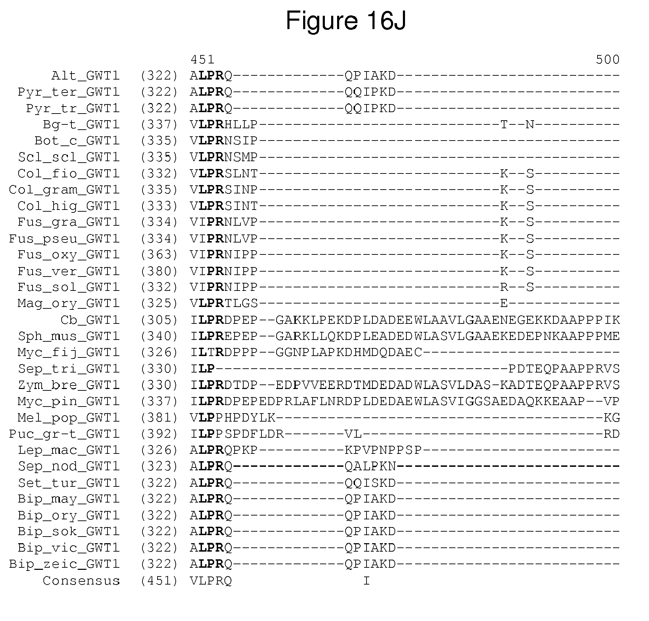

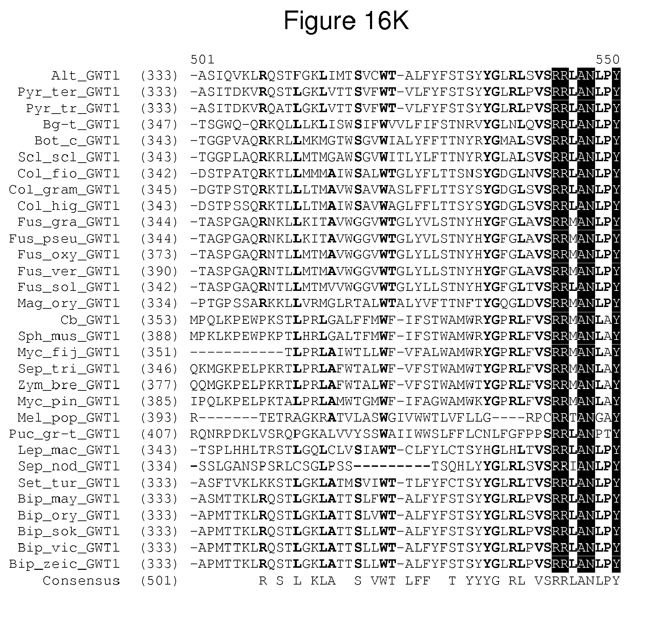

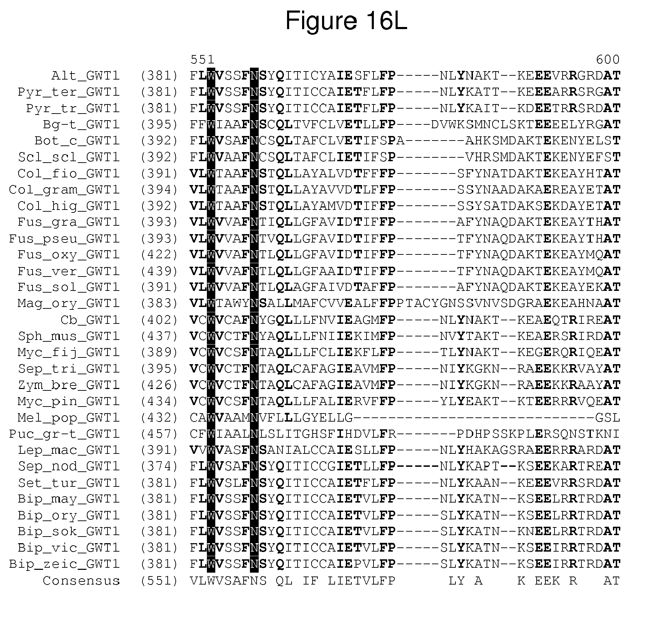

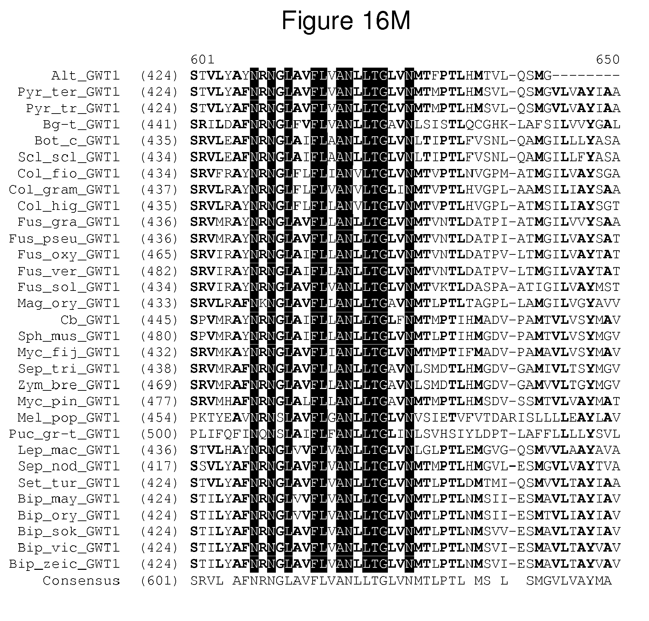

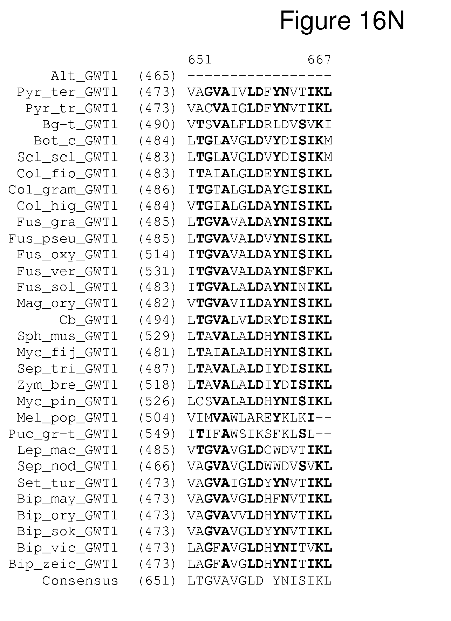

Figure 16 A-N: GWT1 protein alignment: Identification of conserved amino acid sequences in the GWT1 proteins of ascomycota and basidiomycota. The GWT1 sequences of several fungal pathogens *(Alternaria solani* (Alt), *Pyrenophora teres f. teres* (Pyr_ter), *Pyrenophora triticirepentis* (Pyr_tr), *Blumeria graminis f. sp. tritici* (Bg_t), *Botrytis cinerea* (Bot_c), *Sclerotinia sclerotiorum* (Scl_scl), *Colletotrichum fioriniae* (Col_fio), *Colletotrichum graminicola* (Col_gram), *Colletotrichum higginsianum* (Col_hig), *Fusarium graminearum* (Fus_gra), *Fusarium pseudograminearum* (Fus_pseu), *Fusarium oxysporum* (Fus_oxy), *Fusarium verticillioides* (Fus_ver), *Fusarium solani* (Fus_sol), *Magnaporthe oryzae* (Mag_ory), *Cercospora beticola* (Cb), *Sphaerulina musiva* (Sph_mus), *Mycosphaerella fijiensis* (Myc_fij), *Septoria tritici* (Sep_tri), *Zymoseptoria brevis* (Zym_bre), *Mycosphaerella pini* (Myc_pin), *Melampsora populina* (Mel_pop), *Puccinia graminis tritici* (Puc_gr-t), *Leptosphaeria maculans* (Lep_mac), *Septoria nodorum* (Sep_nod), *Setosphaeria turcica* (Set_tur), *Bipolaris maydis* (Bip_may), *Bipolaris oryzae* (Bip_ory), *Bipolaris sorokiniana* (Bip_sok), *Bipolaris victoria* (Bip_vic), *Bipolaris zeicola* (Bip_zeic)). Selected conserved regions are underlined.

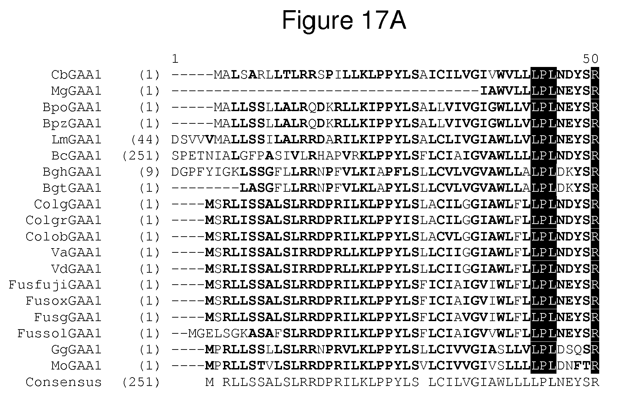

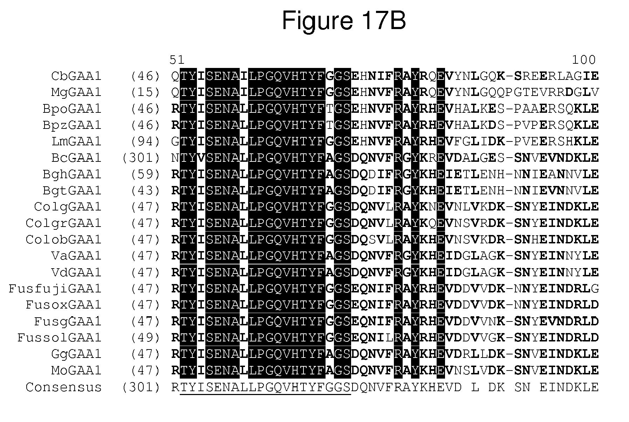

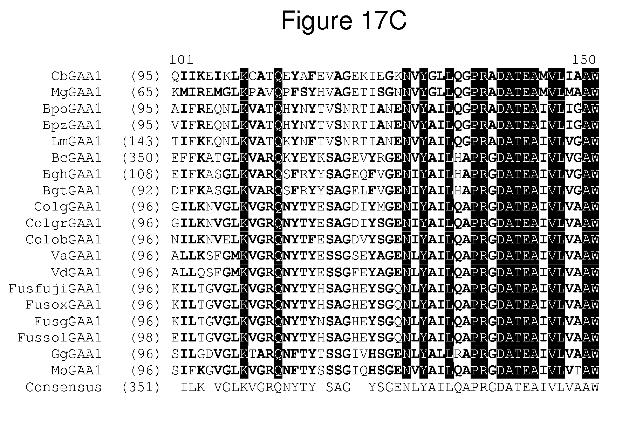

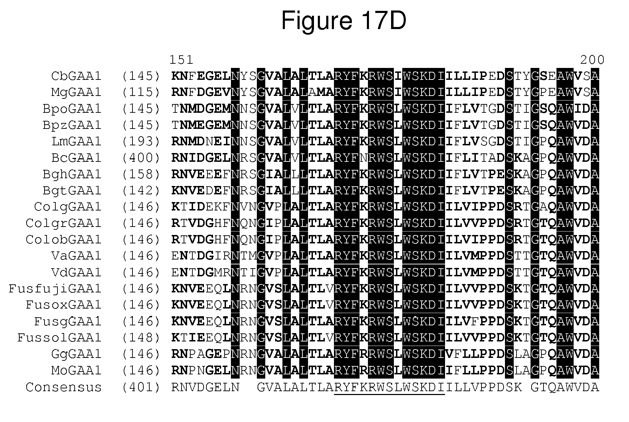

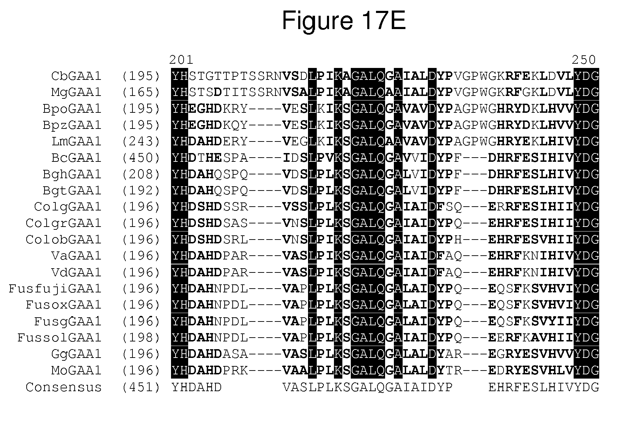

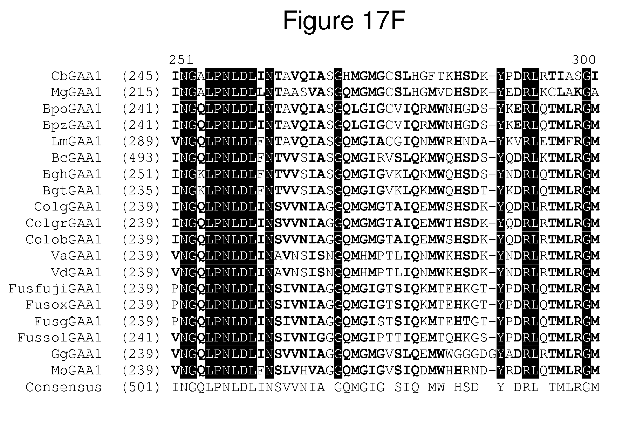

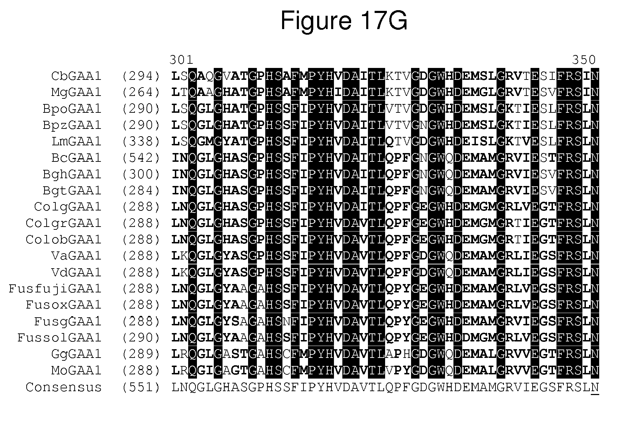

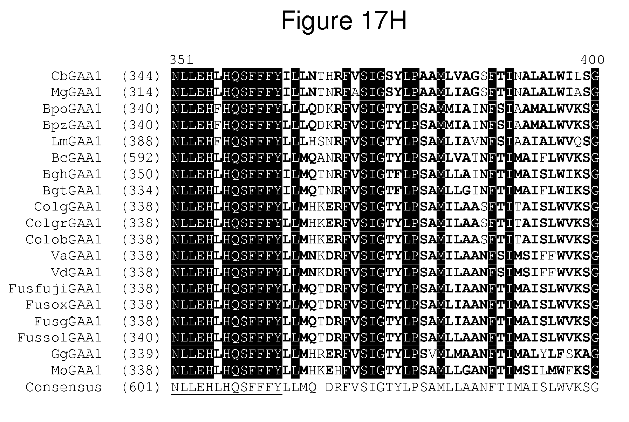

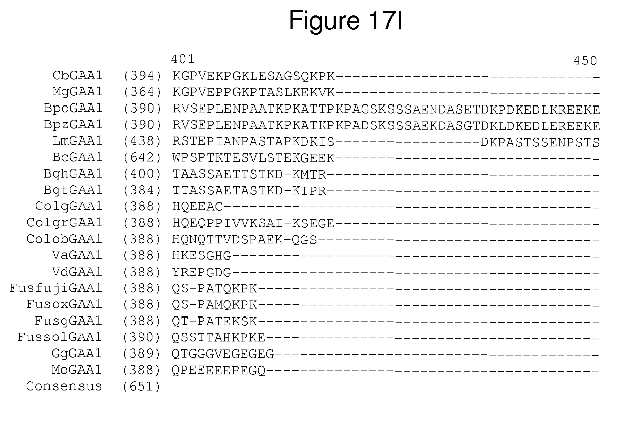

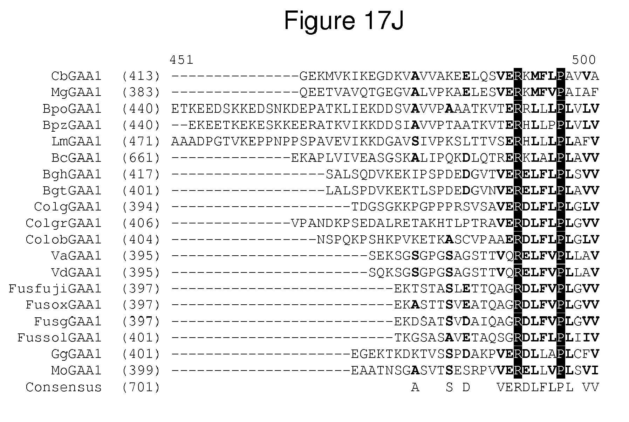

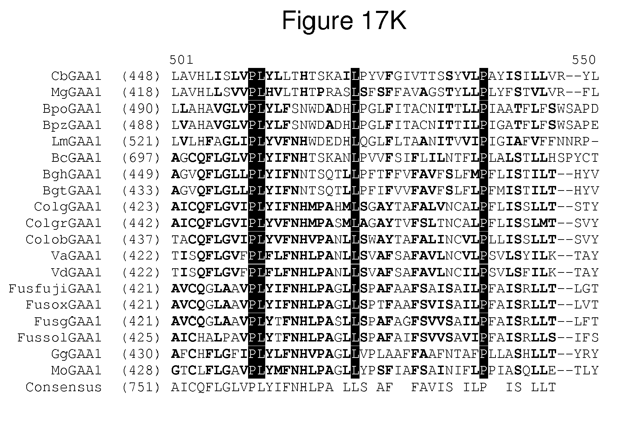

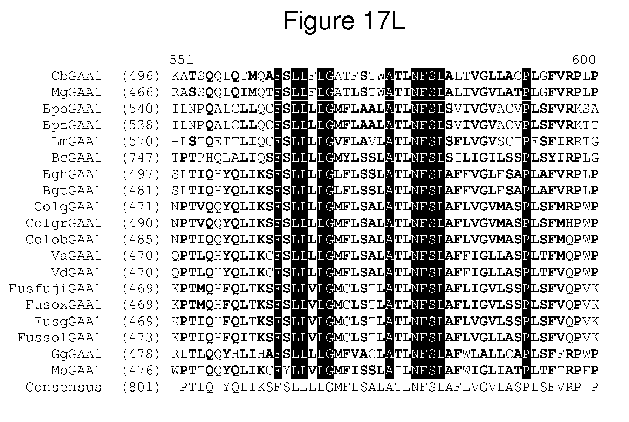

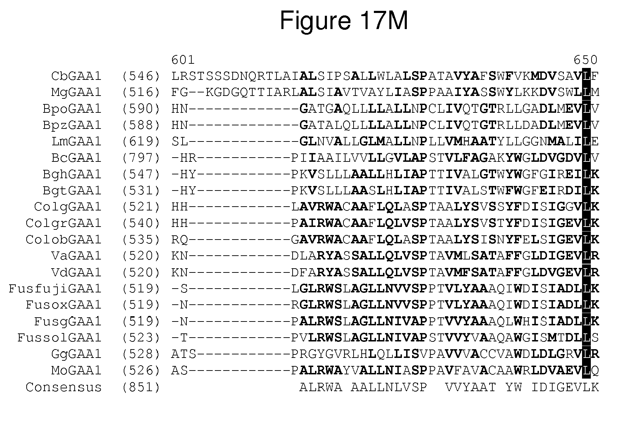

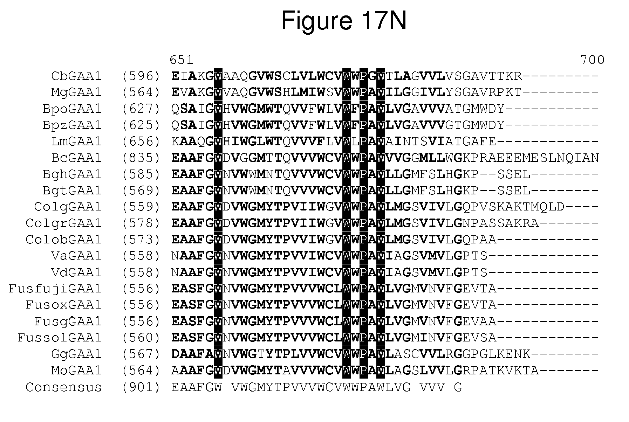

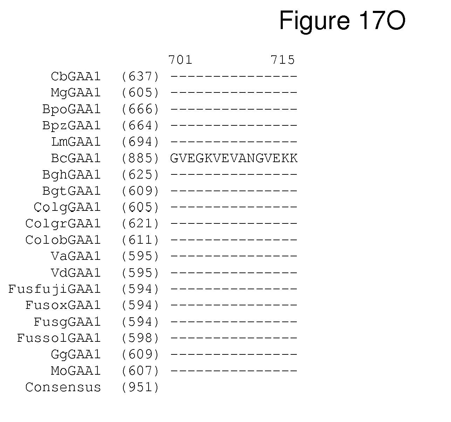

Figure 17 A-O: GAA1 protein alignment: Identification of conserved amino acid sequences in the GAA1 proteins of ascomycota *(Cercospora beticola* (Cb), *Mycosphaerella graminicola* (Mg), *Bipolaris oryzae* (Bpo), *Bipolaris zeicola* (Bpz), *Leptosphaeria maculans* (Lm), *Botrytis cinerea* (Bc), *Blumeria graminis f. sp. hordei* (Bgh), *Blumeria graminis f. sp. tritici* (Bgt), *Colletotrichum gloeosporioides* (Colg), *Colletotrichum graminicola* (Colgr), *Colletotrichum orbiculare* (Colob), *Verticillium alfalfa* (Va), *Verticillium dahliae* (Vd), *Fusarium fujikuroi* (Fusfuji), *Fusarium oxysporum* (Fusox), *Fusarium graminearum* (Fusg), *Fusarium solani* (Fussol), *Gaeumannomyces graminis var. tritici* (Gg), *Magnaporthe oryzae* (Mo)). Selected conserved regions are underlined.

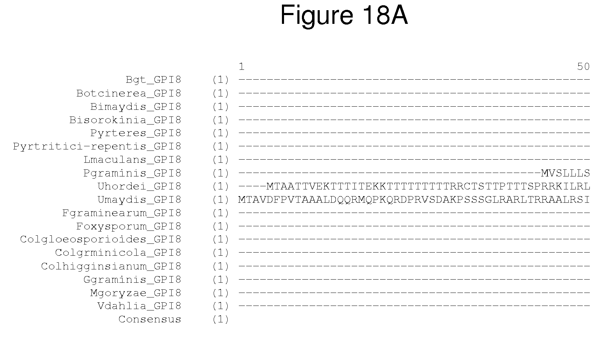

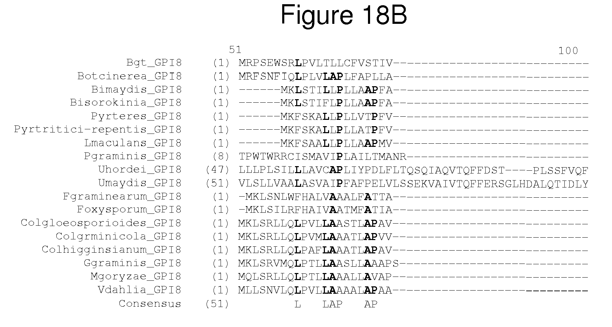

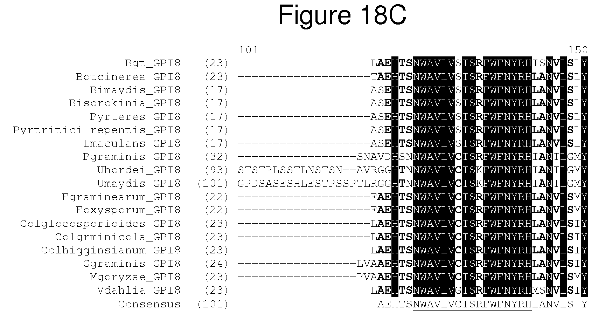

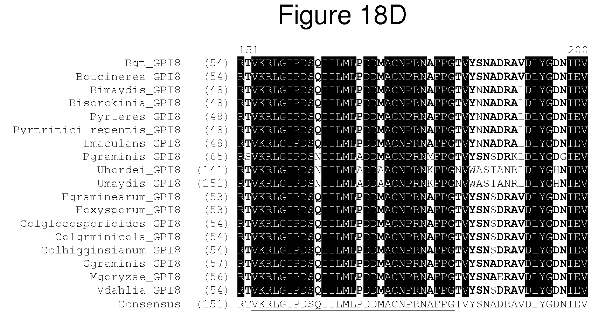

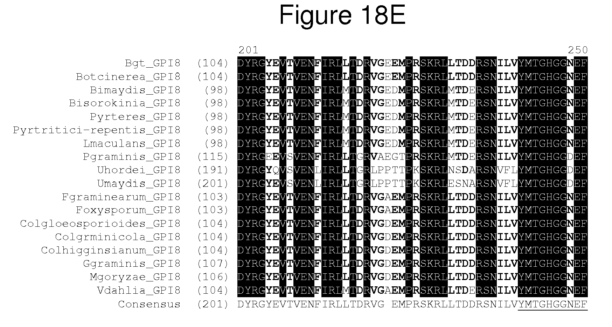

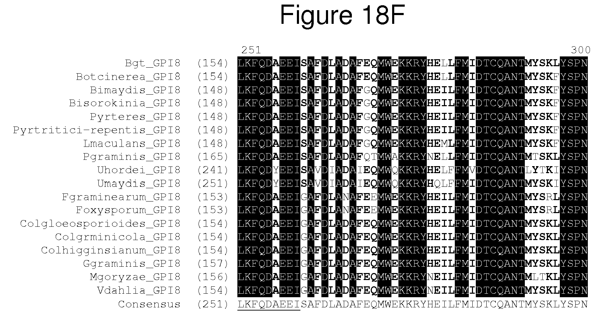

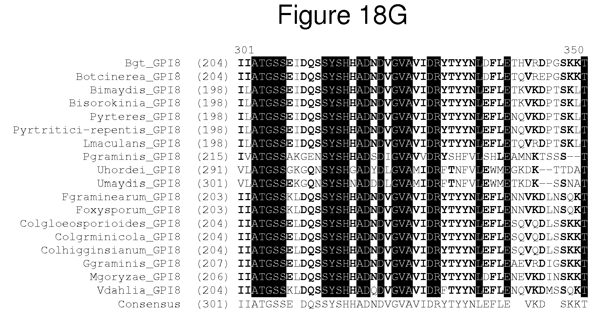

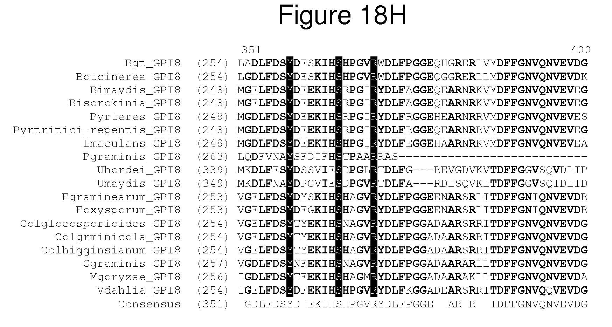

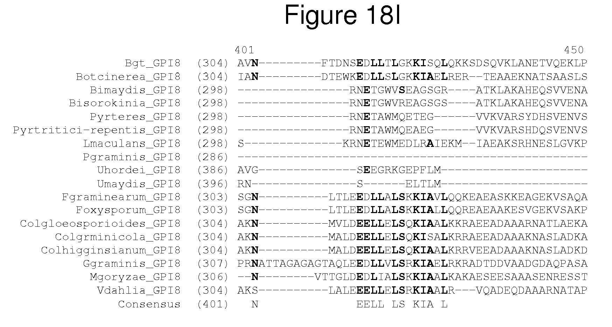

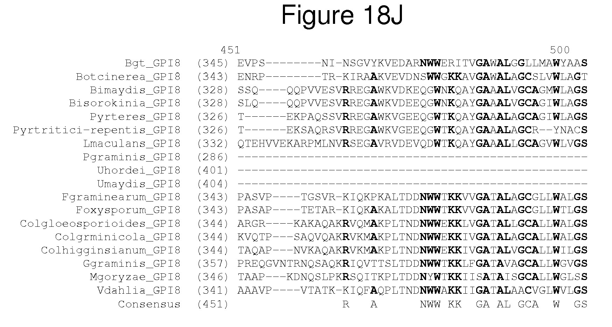

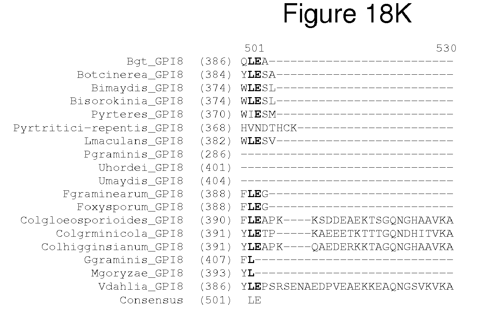

Figure 18 A-K: GPI8 protein alignment: Identification of conserved amino acid sequences of GPI8 proteins of ascomycota and basidiomycota *(Blumeria graminis f*. *sp. tritici* (Bgt), *Botrytis cinerea* (Botcinerea), *Bipolaris maydis* (Bimaydis), *Bipolaris sorokiniana* (Bisorokinia), *Pyrenophora teres f. teres* (Pyrteres), *Pyrenophora tritici-repentis* (Pyrtritici-repentis), *Leptosphaeria maculans* (Lmaculans), *Puccinia graminis* (Pgraminis), *Ustilago hordei* (Uhordei), *Ustilago maydis* (Umaydis), *Fusarium graminearum* (Fgraminearum), *Fusarium oxysporum* (Foxysporum), *Colletotrichum gloeosporioides* (Colgloeosporioides), *Colletotrichum graminicola* (Colgrminicola), *Colletotrichum higginsianum* (Colhigginsianum), *Gaeumannomyces graminis var. tritici* (Ggraminis), *Magnaporthe oryzae* (Mgoryzae), *Verticillium dahliae* (Vdahlia)). Selected conserved regions are underlined.

### Examples

### Results

### Identification of target proteins and genes

The assembly of the GPI complex and its covalent attachment to the C-terminus of proteins is a ubiquitous process in eukaryotic cells which serves to post-translationally modify proteins to anchor them to the cell surface. Thereby, a substantial amount of the estimated 61 GPI-anchored proteins identified in baker's yeast play a major role in cell wall structure and rigidity. They serve enzymatic functions required for the biosynthesis and the continuous shape adaptations of the cell wall, others seem to be structural elements of the cell wall and yet others mediate cell adhesion. The assembly of the GPI anchor and its attachment to proteins in *Saccharomyces cerevisiae* is presented in Figure 1 where in a series of steps the GPI complex is build and attached to a protein. The different biosynthetic steps are mediated by approx. 25 proteins and take place successively on the cytoplasmic and on the luminal face of the ER membrane. The proteins which are involved comprise GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1. The first step of GPI biosynthesis is the addition of *N*-acetyl glucosamine to phosphatidylinositol. This reaction occurs on the cytoplasmic ER surface and is catalyzed by *N*-acetyl glucosaminyl transferase, an unusually complex glycosyl transferase consisting of the six core proteins GPI1, GPI2, GPI3, GPI15, ERI1, and GPI19. The acetyl group of the N-acetyl glucosaminyl residue is then removed by the cytoplasmic N-acetylglucosaminylphosphatidylinositol deacetylase encoded by GPI12, and the glucosamine formed is enzymatically 2-O-palmitolyated, using acyl-CoA as the acyl donor. The resulting intermediate is translocated to the luminal side of the phospholipid bilayer. In the ER lumen, two mannosyl units are subsequently linked to the glucosamine by the two distinct ER-resident mannosyl transferases GPI14 and GPI18. Interestingly, analysis of the substrate specificity of the first dolichol phosphate mannose:glucosaminyl phosphatidylinositol α1-4-mannosyltransferase of the glycosyl-phosphatidylinositol biosynthetic pathway of African trypanosomes showed that *N*-acetylglucosaminylphosphatidylinositol is more efficiently presented to the enzyme than its deacetylated form, suggesting important substrate channelling via the preceding deacetylase GPI12 (Smith, 1996). Next, the addition of a phosphoryl-ethanolamine residue to the first mannosyl unit, is catalyzed by Mcd4. The phosphoryl-ethanolamine group linked to the first mannosyl residue is required for both recognition of the GPI by the third mannosyl transferase GPI10, as well as by the transamidase complex. In yeast, the addition of a fourth onto the third mannosyl residue by SMP3 is required for adding a phosphoethanolamine group to the third mannosyl residue, as catalyzed by GPI13. The last step of GPI biosynthesis before attaching this anchor to a protein is the addition of a third phosphoryl-ethanolamine residue, which is mediated by ethanolamine phosphotransferase GPI7. The attachment of GPI to a protein is catalyzed by the GPI transamidase complex, a protein complex consisting of the five distinct subunits GPI8, GPI16, GAA1, GAB1, and GPI17, organized into three components. According to a recent model, the central part and the catalytic domain of the GPI transamidase complex is GPI8. GPI8 interacts with Gaa1 and with all other subunits of the GPI transamidase complex. The work by Udenfriend and colleagues suggested that GPI8 is a cystein protease cleaving the C-terminal pro-peptide of the substrate protein even in the absence of the GPI anchor (Maxwell, 1995). Importantly, GAA1 has recently been suggested to function as a metallo-peptide-synthetase catalyzing formation of the peptide bond between the substrate protein's ω-site and the GPI lipid anchor's phosphoethanolamine. The complex reactions involved in biosynthesis of GPI and attachment of the GPI anchor to proteins are conserved from yeast to men.

Enzymes and proteins involved in protein-anchoring via GPI are conserved in fungi. Reference is made to Figure 2, where the similarity of three selected proteins, GPI12, GAA1 and GPI8, of various species is shown in dendrograms. Protein and nucleic acid sequences of GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and BST1 of selected fungal species are state of the art. Exemplary proteins are referred to herein by their accession numbers (see also below) and are disclosed, in the order of appearance, in the sequence listing under SEQ ID NOs: 1 to 400. The indicated sequences may serve as reference sequences for identifying homologous proteins and genes in other fungal species, characterized by a degree of identity of at least 60, 70, 80, 85, 90, 95 or 99% or by a degree of homology of at least 40, 50, 60, 70, 80, 85, 90, 95 or 99% on the amino acid level or by a degree of identity of at least 30, 40, 50, 60, 70, 80, 85, 90, 95 or 99% on the nucleic acid level or by hybridization under stringent hybridization conditions with respect to the reference sequence.

Preferred target genes and proteins of the present invention are GPI12, GAA1, GPI8, and GWT1. Exemplary organisms and the accession numbers under which amino acid and nucleotide sequences are to be found are listed in Tables 1 to 4.

**Table 1: The following list indicates the organisms and the accession numbers under which amino acid and nucleotide sequences of GPI12 are to be found. These amino acid and nucleotide sequences are disclosed, in the order of appearance, in the sequence listing under SEQ ID NOs: 1-86 and 393-396 (odd numbers are nucleotide sequences and subsequent even numbers are corresponding amino acid sequence, exceptions are SEQ ID NOs: 395 and 396 which are amino acid sequences without corresponding nucleotide sequences).**

| Fungal pathogen | amino acid sequence GPI12; uniprot or NCBI reference sequence | sequence identifier of nucleotide sequence | sequence identifier of amino acid sequence |
|---|---|---|---|
| *Alternaria solani* | no reference | SEQ ID NO: 393 | SEQ ID NO: 394 |
| *Blumeria graminis f. sp. tritici 96224* | EPQ62736.1 | SEQ ID NO: 1 | SEQ ID NO: 2 |
| *Blumeria graminis f. sp. hordei DH14* | CCU76388.1 | SEQ ID NO: 3 | SEQ ID NO: 4 |
| *Bipolaris maydis C5* | EMD87943.1 | SEQ ID NO: 5 | SEQ ID NO: 6 |
| *Bipolaris oryzae ATCC 44560* | W6ZBJ1-COCM | SEQ ID NO: 11 | SEQ ID NO: 12 |
| *Bipolaris victoriae FI3* | EUN24454.1 | SEQ ID NO: 7 | SEQ ID NO: 8 |
| *Bipolaris zeicola 26-R-13* | XP_007713568.1 | SEQ ID NO: 9 | SEQ ID NO: 10 |
| *Botryosphaeria parva* | R1E9V7 _BOTPV | SEQ ID NO: 15 | SEQ ID NO: 16 |
| *Botrytis cinerea B05.10* | XP_001550141.1 | SEQ ID NO: 13 | SEQ ID NO: 14 |
| *Cercospora beticola* | no reference | SEQ ID NO: 17 | SEQ ID NO: 18 |
| *Cochliobolus sativus* | M2T701_COCSN | SEQ ID NO: 19 | SEQ ID NO: 20 |
| *Colletotrichum graminicola M1.001* | EFQ25317.1 | SEQ ID NO: 21 | SEQ ID NO: 22 |
| *Colletotrichum gloeosporioides Cg-14* | EQB47646.1 | SEQ ID NO: 23 | SEQ ID NO: 24 |
| *Colletotrichum higginsianum* | CCF33957.1 | SEQ ID NO: 25 | SEQ ID NO: 26 |
| *Claviceps purpurea 20.1* | CCE27514.1 | SEQ ID NO: 27 | SEQ ID NO: 28 |
| *Diplodia seriata* | A0A0G2GVF3_9PEZI | SEQ ID NO: 29 | SEQ ID NO: 30 |
| *Fusarium fujikuroi IMI 58289* | CCT73649.1 | SEQ ID NO: 31 | SEQ ID NO: 32 |
| *Fusarium graminearum PH-1* | XP_384272.1 | SEQ ID NO: 33 | SEQ ID NO: 34 |
| *Fusarium oxysporum Fo5176* | EGU74772.1 | SEQ ID NO: 35 | SEQ ID NO: 36 |
| *Fusarium pseudograminearum (strain CS3096)* | K3V2Z5_FUSPC | SEQ ID NO: 37 | SEQ ID NO: 38 |
| *Gaeumannomyces graminis var. tritici R3-111a-1* | EJT81184.1 | SEQ ID NO: 39 | SEQ ID NO: 40 |
| *Leptosphaeria maculans JN3* | XP_003840011.1 | SEQ ID NO: 41 | SEQ ID NO: 42 |
| *Macrophomina phaseolina (strain MS6)* | K2SFE7_MACPH | SEQ ID NO: 43 | SEQ ID NO: 44 |
| *Magnaporthe oryzae 70-15* | XP_003712526.1 | SEQ ID NO: 45 | SEQ ID NO: 46 |
| *Marssonina brunnea f. sp. multigermtubi MB_m1* | K1WYF0_MARBU | SEQ ID NO: 47 | SEQ ID NO: 48 |
| *Mycosphaerella fijiensis* | M3B8L0_PSEFD | SEQ ID NO: 49 | SEQ ID NO: 50 |
| *Mycosphaerella pini* | N1PWT0_DOTSN | SEQ ID NO: 51 | SEQ ID NO: 52 |
| *Phytophthora infestans* | no reference | - | SEQ ID NO: 395 |
| *Phytophthora sojae* | no reference | - | SEQ ID NO: 396 |
| *Puccinia graminis tritici* | XP_003329745.2 | SEQ ID NO: 53 | SEQ ID NO: 54 |
| *Puccinia striiformis a* | KNE92355.1 | SEQ ID NO: 55 | SEQ ID NO: 56 |
| *Puccinia striiformis b* | KNE92354.1 | SEQ ID NO: 57 | SEQ ID NO: 58 |
| *Puccinia triticina* | OAV93070.1 | SEQ ID NO: 59 | SEQ ID NO: 60 |
| *Pyrenophora tritici-repentis Pt-1C-BFP* | XP_001935903.1 | SEQ ID NO: 61 | SEQ ID NO: 62 |
| *Pyrenophora teres f. teres 0-1* | XP_003303045.1 | SEQ ID NO: 63 | SEQ ID NO: 64 |
| *Sclerotinia borealis* | W9C6D0_9HELO | SEQ ID NO: 65 | SEQ ID NO: 66 |
| *Sclerotinia sclerotiorum 1980* | XP_001587613.1 | SEQ ID NO: 67 | SEQ ID NO: 68 |
| *Septoria nodorum* | Q0UE33_PHANO | SEQ ID NO: 69 | SEQ ID NO: 70 |
| *Septoria tritici* | XP_003855355.1 | SEQ ID NO: 71 | SEQ ID NO: 72 |
| *Setosphaeria turcica* | no reference | SEQ ID NO: 73 | SEQ ID NO: 74 |
| *Sphaerulina musiva* | M3DBL1_SPHMS | SEQ ID NO: 75 | SEQ ID NO: 76 |
| *Ustilago maydis 521* | XP_756449.1 | SEQ ID NO: 77 | SEQ ID NO: 78 |
| *Ustilago hordei* | CCF54030.1 | SEQ ID NO: 79 | SEQ ID NO: 80 |
| *Verticillium dahliae VdLs.17* | XP_009656893.1 | SEQ ID NO: 81 | SEQ ID NO: 82 |
| *Zymoseptoria brevis* | A0A0F4GCJ5_9PEZI | SEQ ID NO: 83 | SEQ ID NO: 84 |
| *Zymoseptoria tritici (Mycosphaerella graminicola)* | A7F332_SCLS1 | SEQ ID NO: 85 | SEQ ID NO: 86 |

**Table 2: The following list indicates the organisms and the accession numbers under which amino acid and nucleotide sequences of GAA1 are to be found. These amino acid and nucleotide sequences are disclosed, in the order of appearance, in the sequence listing under SEQ ID NOs: 87 to 190, 389-390 and 397-398 (odd numbers are nucleotide sequences and subsequent even numbers are corresponding amino acid sequence, exceptions are SEQ ID NOs: 397 and 398 which are amino acid sequences without corresponding nucleotide sequences).**

| Fungal pathogen | amino acid sequence GAA1 uniprot or NCBI reference sequence | sequence identifier of nucleotide sequence | sequence identifier of amino acid sequence |
|---|---|---|---|
| *Alternaria solani* | kws data | SEQ ID NO: 87 | SEQ ID NO: 88 |
| *Bipolaris maydis* | N4WYU8_COCH4 | SEQ ID NO: 89 | SEQ ID NO: 90 |
| *Bipolaris oryzae* | W6Y6U9_COCCA | SEQ ID NO: 91 | SEQ ID NO: 92 |
| *Bipolaris sorokiniana* | M2T337_COCSN | SEQ ID NO: 93 | SEQ ID NO: 94 |
| *Bipolaris victoriae FI3* | W7ECX7_COCVI | SEQ ID NO: 95 | SEQ ID NO: 96 |
| *Bipolaris zeicola 26-R-13* | XP_007710354.1 | SEQ ID NO: 97 | SEQ ID NO: 98 |
| *Blumeria graminis f. sp. hordei DH14* | CCU76546.1 | SEQ ID NO: 99 | SEQ ID NO: 100 |
| *Blumeria graminis f. sp. tritici 96224* | EPQ65222.1 | SEQ ID NO: 101 | SEQ ID NO: 102 |
| *Botryosphaeria parva (strain UCR-NP2)* | R1EK29_BOTPV | SEQ ID NO: 103 | SEQ ID NO: 104 |
| *Botrytis cinerea BcDW1* | M7TNH6_BOTF1 | SEQ ID NO: 105 | SEQ ID NO: 106 |
| *Cercospora beticola* | no reference | SEQ ID NO: 107 | SEQ ID NO: 108 |
| *Colletotrichum fioriniae PJ7* | A0A010QKM2_9PEZI | SEQ ID NO: 109 | SEQ ID NO: 110 |
| *Colletotrichum graminicola M1.001]* | E3QI05_COLGM | SEQ ID NO: 111 | SEQ ID NO: 112 |
| *Colletotrichum gloeosporioides Cg-14* | T0M079_COLGC | SEQ ID NO: 113 | SEQ ID NO: 114 |
| *Colletotrichum higginsianum* | CCF39866.1 | SEQ ID NO: 115 | SEQ ID NO: 116 |
| *Colletotrichum orbiculare MAFF 240422* | N4VRZ7_COLOR | SEQ ID NO: 117 | SEQ ID NO: 118 |
| *Colletotrichum sublineola* | A0A066X1M2_COLSU | SEQ ID NO: 119 | SEQ ID NO: 120 |
| *Diaporthe ampelina* | A0A0G2HNS3_9PEZI | SEQ ID NO: 121 | SEQ ID NO: 122 |
| *Diplodia maydis* | no reference | SEQ ID NO: 123 | SEQ ID NO: 124 |
| *Diplodia seriata* | A0A0G2ED10_9PEZI | SEQ ID NO: 125 | SEQ ID NO: 126 |
| *Fusarium fujikuroi IMI 58289* | CCT67544.1 | SEQ ID NO: 127 | SEQ ID NO: 128 |
| *Fusarium graminearum PH-1* | I1RUQ4_GIBZE | SEQ ID NO: 129 | SEQ ID NO: 130 |
| *Fusarium oxysporum f. sp. cubense race 4* | F9FCF7_FUSOF | SEQ ID NO: 131 | SEQ ID NO: 132 |
| *Fusarium pseudograminearum (strain CS3096)* | K3U8J3_FUSPC | SEQ ID NO: 133 | SEQ ID NO: 134 |
| *Gaeumannomyces graminis var. tritici R3-111a-1* | J3NV59_GAGT3 | SEQ ID NO: 135 | SEQ ID NO: 136 |
| *Leptosphaeria maculans JN3* | E5A3J3_LEPMJ | SEQ ID NO: 137 | SEQ ID NO: 138 |
| *Macrophomina phaseolina (strain MS6)* | K2QLD8_MACPH | SEQ ID NO: 139 | SEQ ID NO: 140 |
| *Magnaporthe oryzae 70*-*15]* | G4N7S2_MAGO7 | SEQ ID NO: 141 | SEQ ID NO: 142 |
| *Marssonina brunnea f*. *sp. multigermtubi* | K1WPF4_MARBU | SEQ ID NO: 143 | SEQ ID NO: 144 |
| *Melampsora larici*-*populina* | F4RWS7_MELLP | SEQ ID NO: 145 | SEQ ID NO: 146 |
| *Mycosphaerella fijiensis* | M3AL08_PSEFD | SEQ ID NO: 147 | SEQ ID NO: 148 |
| *Mycosphaerella pini* | N1PCN8_DOTSN | SEQ ID NO: 149 | SEQ ID NO: 150 |
| *Nectria haematococca (Fusarium solani)* | C7ZLS4_NECH7 | SEQ ID NO: 151 | SEQ ID NO: 152 |
| *Phaeosphaeria nodorum* | XP_001802233.1 | SEQ ID NO: 155 | SEQ ID NO: 156 |
| *Phytophthora infestans T30-4* | XP_002898680.1 | SEQ ID NO: 389 | SEQ ID NO: 390 |
| *Phytophthora ramorum* | no reference | - | SEQ ID NO: 397 |
| *Phytophthora sojae* | no reference | - | SEQ ID NO: 398 |
| *Puccinia graminis tritici (black rust)* | no reference | SEQ ID NO: 157 | SEQ ID NO: 158 |
| *Puccinia striiformis* (*yellow rust*) | KNF03903.1 | SEQ ID NO: 159 | SEQ ID NO: 160 |
| *Puccinia triticina* (*brown rust*) | kws data | SEQ ID NO: 161 | SEQ ID NO: 162 |
| *Pyrenophora teres f. teres (strain 0*-*1)* | E3S4J2_PYRTT | SEQ ID NO: 163 | SEQ ID NO: 164 |
| *Pyrenophora tritici-repentis (strain Pt-1C-BFP)* | B2VXP3_PYRTR | SEQ ID NO: 165 | SEQ ID NO: 166 |
| *Sclerotinia borealis F-4157* | W9CQ82_9HELO | SEQ ID NO: 167 | SEQ ID NO: 168 |
| *Sclerotinia sclerotiorum 1980 UF-70* | A7F0D2_SCLS1 | SEQ ID NO: 169 | SEQ ID NO: 170 |
| *Septoria nodorum* | Q0U8B2_PHANO | SEQ ID NO: 171 | SEQ ID NO: 172 |
| *Septoria tritici* / *Mycosphaerella graminicola* | XP_003848386.1 | SEQ ID NO: 153 | SEQ ID NO: 154 |
| *Setosphaeria turcica (strain NY001)* | no reference | SEQ ID NO: 173 | SEQ ID NO: 174 |
| *Setosphaeria turcica (strain 28A)* | no reference | SEQ ID NO: 175 | SEQ ID NO: 176 |
| *Sphaerulina musiva (strain SO2202)* | M3CYA3_SPHMS | SEQ ID NO: 177 | SEQ ID NO: 178 |
| *Ustilago maydis 521* | XP_762352.1 | SEQ ID NO: 179 | SEQ ID NO: 180 |
| *Ustilago hordei* | CCF48763.1 | SEQ ID NO: 181 | SEQ ID NO: 182 |
| *Verticillium dahliae VdLs.17* | G2WVI9_VERDV | SEQ ID NO: 183 | SEQ ID NO: 184 |
| *Verticillium alfalfae VaMs.102* | C9SRB1_VERA1 | SEQ ID NO: 185 | SEQ ID NO: 186 |
| *Zymoseptoria brevis* | A0A0F4GNF8_9PEZI | SEQ ID NO: 187 | SEQ ID NO: 188 |
| *Zymoseptoria tritici (strain CBS 115943* / *IPO323)* | no reference | SEQ ID NO: 189 | SEQ ID NO: 190 |

**Table 3: The following list indicates the organisms and the accession numbers under which amino acid and nucleotide sequences of GPI8 are to be found. These amino acid and nucleotide sequences are disclosed, in the order of appearance, in the sequence listing under SEQ ID NOs: 191-278, 391-392, and 399 (odd numbers are nucleotide sequences and subsequent even numbers are corresponding amino acid sequence, exception is SEQ ID NO: 399 which is an amino acid sequence without corresponding nucleotide sequence).**

| Fungal pathogen | amino acid sequence GPI8 uniprot or NCBI reference sequence | sequence identifier of nucleotide sequence | sequence identifier of amino acid sequence |
|---|---|---|---|
| *Alternaria solani* | no reference | SEQ ID NO: 191 | SEQ ID NO: 192 |
| *Blumeria graminis f. sp. tritici 96224* | EPQ66664.1 | SEQ ID NO: 193 | SEQ ID NO: 194 |
| *Bipolaris maydis* | EMD93277.1 | SEQ ID NO: 195 | SEQ ID NO: 196 |
| *Bipolaris sorokiniana* | XP_007695404.1 | SEQ ID NO: 197 | SEQ ID NO: 198 |
| *Bipolaris oryzae* | W6ZCQ9_COCMI | SEQ ID NO: 199 | SEQ ID NO: 200 |
| *Bipolaris victoriae FI3* | W7ELV8_COCVI | SEQ ID NO: 201 | SEQ ID NO: 202 |
| *Bipolaris zeicola 26-R-13* | W6Y7X6_COCCA | SEQ ID NO: 203 | SEQ ID NO: 204 |
| *Botryosphaeria parva* | R1G6E1_BOTPV | SEQ ID NO: 205 | SEQ ID NO: 206 |
| *Botrytis cinerea* | EMR90910.1 | SEQ ID NO: 207 | SEQ ID NO: 208 |
| *Cercospora beticola* | no reference | SEQ ID NO: 209 | SEQ ID NO: 210 |
| *Claviceps purpurea* | CCE31202.1 | SEQ ID NO: 211 | SEQ ID NO: 212 |
| *Colletotrichum gloeosporioides* | ELA35472.1 | SEQ ID NO: 213 | SEQ ID NO: 214 |
| *Colletotrichum graminicola M1.001* | EFQ31045.1 | SEQ ID NO: 215 | SEQ ID NO: 216 |
| *Colletotrichum higginsianum* | CCF33935.1 | SEQ ID NO: 217 | SEQ ID NO: 218 |
| *Colletotrichum orbiculare* | ENH84689.1 | SEQ ID NO: 219 | SEQ ID NO: 220 |
| *Colletotrichum sublineola* | A0A066X3R0_COLSU | SEQ ID NO: 221 | SEQ ID NO: 222 |
| *Diplodia seriata.* | A0A0G2EU52_9PEZI | SEQ ID NO: 223 | SEQ ID NO: 224 |
| *Fusarium fujikuroi* | CCT64293.1 | SEQ ID NO: 225 | SEQ ID NO: 226 |
| *Fusarium graminearum* | XP_389811.1 | SEQ ID NO: 227 | SEQ ID NO: 228 |
| *Fusarium oxysporum f. sp. cubense* | ENH73817.1 | SEQ ID NO: 229 | SEQ ID NO: 230 |
| *Fusarium pseudograminearum* | K3VI57_FUSPC | SEQ ID NO: 231 | SEQ ID NO: 232 |
| *Gaeumannomyces graminis var. tritici* | XP_009218379.1 | SEQ ID NO: 233 | SEQ ID NO: 234 |
| *Leptosphaeria maculans* | XP_003837876.1 | SEQ ID NO: 235 | SEQ ID NO: 236 |
| *Macrophomina phaseolina* | K2SFV8_MACPH | SEQ ID NO: 237 | SEQ ID NO: 238 |
| *Magnaporthe oryzae* | XP_003713394.1 | SEQ ID NO: 239 | SEQ ID NO: 240 |
| *Marssonina brunnea f. sp. multigermtubi* | K1XD14_MARBU | SEQ ID NO: 241 | SEQ ID NO: 242 |
| *Melampsora larici-populina* | F4RLF1_MELLP | SEQ ID NO: 243 | SEQ ID NO: 244 |
| *Microbotryum lychnidis-dioicae* | U5HEY6_USTV1 | SEQ ID NO: 245 | SEQ ID NO: 246 |
| *Mycosphaerella fijiensis* | M3B0T1_PSEFD | SEQ ID NO: 247 | SEQ ID NO: 248 |
| *Nectria haematococca* | C7Z4S9_NECH7 | SEQ ID NO: 249 | SEQ ID NO: 250 |
| *Phytophthora infestans* | no reference | SEQ ID NO: 391 | SEQ ID NO: 392 |
| *Phytophthora sojae* | no reference | - | SEQ ID NO: 399 |
| *Puccinia graminis f. sp. tritici* | E3JXE4_PUCGT | SEQ ID NO: 251 | SEQ ID NO: 252 |
| *Puccinia striiformis* | no reference | SEQ ID NO: 253 | SEQ ID NO: 254 |
| *Puccinia triticina* | no reference | SEQ ID NO: 255 | SEQ ID NO: 256 |
| *Pyrenophora teres* | XP_003296516.1 | SEQ ID NO: 257 | SEQ ID NO: 258 |
| *Pyrenophora tritici*-*repentis* | XP_001932828.1 | SEQ ID NO: 259 | SEQ ID NO: 260 |
| *Sclerotinia borealis* | W9C4T5_9HELO | SEQ ID NO: 261 | SEQ ID NO: 262 |
| *Septoria nodorum* | QOUPJ4_PHANO | SEQ ID NO: 263 | SEQ ID NO: 264 |
| *Setosphaeria turcica (strain 28A)* | no reference | SEQ ID NO: 265 | SEQ ID NO: 266 |
| *Sphaerulina musiva* | M3CJM0_SPHMS | SEQ ID NO: 267 | SEQ ID NO: 268 |
| *Ustilago hordei* | CCF49395.1 | SEQ ID NO: 269 | SEQ ID NO: 270 |
| *Ustilago maydis* | XP_761856.1 | SEQ ID NO: 271 | SEQ ID NO: 272 |
| *Verticillium dahliae* | XP_009650278.1 | SEQ ID NO: 273 | SEQ ID NO: 274 |
| *Zymoseptoria brevis* | A0A0F4GM06_9PEZI | SEQ ID NO: 275 | SEQ ID NO: 276 |
| *Zymoseptoria tritici (Mycosphaerella graminicola)* | KJX97235.1 | SEQ ID NO: 277 | SEQ ID NO: 278 |

**Table 4: The following list indicates the organisms and the accession numbers under which amino acid and nucleotide sequences of GWT1 are to be found. These amino acid and nucleotide sequences are disclosed, in the order of appearance, in the sequence listing under SEQ ID NOs: 279 to 350, and 400 (odd numbers are nucleotide sequences and subsequent even numbers are corresponding amino acid sequence, exception is SEQ ID NO: 400 which is an amino acid sequence without corresponding nucleotide sequence).**

| Fungal pathogen | amino acid sequence GWT1 uniprot or NCBI reference sequence | sequence identifier of nucleotide sequence | sequence identifier of amino acid sequence |
|---|---|---|---|
| *Alternaria solani* | no reference | SEQ ID NO: 279 | SEQ ID NO: 280 |
| *Bipolaris maydis* | N4X697_COCH4 | SEQ ID NO: 285 | SEQ ID NO: 286 |
| *Bipolaris oryzae ATCC 44560* | W6ZVI8_COCMI | SEQ ID NO: 287 | SEQ ID NO: 288 |
| *Bipolaris victoriae FI3* | W7EBJ8_COCVI | SEQ ID NO: 289 | SEQ ID NO: 290 |
| *Bipolaris zeicola 26-R-13* | W6XXE1_COCCA | SEQ ID NO: 291 | SEQ ID NO: 292 |
| *Blumeria graminis f. sp. tritici.* | E7DZJ9_BLUGR | SEQ ID NO: 293 | SEQ ID NO: 294 |
| *Botryotinia fuckeliana (Botrytis cinerea)* | G2Y8N6_BOTF4 | SEQ ID NO: 295 | SEQ ID NO: 296 |
| *Cercospora beticola* | no reference | SEQ ID NO: 281 | SEQ ID NO: 282 |
| *Cochliobolus sativus (Bipolaris sorokiniana)* | M2SB78_COCSN | SEQ ID NO: 297 | SEQ ID NO: 298 |
| *Colletotrichum fioriniae PJ7* | A0A010QVS2_9PEZI | SEQ ID NO: 299 | SEQ ID NO: 300 |
| *Colletotrichum graminicola* | E3Q3K0_COLGM | SEQ ID NO: 301 | SEQ ID NO: 302 |
| *Colletotrichum higginsianum* | H1VZH3_COLHI | SEQ ID NO: 303 | SEQ ID NO: 304 |
| *Diplodia maydis* | no reference | SEQ ID NO: 283 | SEQ ID NO: 284 |
| *Gaeumannomyces graminis var. tritici* | J3NRY1_GAGT3 | SEQ ID NO: 305 | SEQ ID NO: 306 |
| *Fusarium graminearum* | GWT1_GIBZE | SEQ ID NO: 307 | SEQ ID NO: 308 |
| *Fusarium pseudograminearum* | K3V2I8_FUSPC | SEQ ID NO: 309 | SEQ ID NO: 310 |
| *Fusarium oxysporum* | F9FXK6_FUSOF | SEQ ID NO: 311 | SEQ ID NO: 312 |
| *Fusarium verticillioides* | W7LLV3_GIBM7 | SEQ ID NO: 313 | SEQ ID NO: 314 |
| *Leptosphaeria maculans* | E5A261_LEPMJ | SEQ ID NO: 315 | SEQ ID NO: 316 |
| *Magnaporthe oryzae* | G4MWY7_MAGO7 | SEQ ID NO: 317 | SEQ ID NO: 318 |
| *Melampsora larici*-*populina* | F4RV06_MELLP | SEQ ID NO: 319 | SEQ ID NO: 320 |
| *Mycosphaerella fijiensis* | M2Z0D7_PSEFD | SEQ ID NO: 321 | SEQ ID NO: 322 |
| *Mycosphaerella pini* | N1PRQ8_DOTSN | SEQ ID NO: 323 | SEQ ID NO: 324 |
| *Nectria haematococca (Fusarium solani spp. pisi)* | C7YII9_NECH7 | SEQ ID NO: 325 | SEQ ID NO: 326 |
| *Phytophthora infestans* | no reference | SEQ ID NO: 327 | SEQ ID NO: 328 |
| *Phytophthora ramorum* | no reference | - | SEQ ID NO: 400 |
| *Phytophthora sojae* | no reference | SEQ ID NO: 329 | SEQ ID NO: 330 |
| *Puccinia graminis f. sp*. *tritici* | E3K5G3_PUCGT | SEQ ID NO: 331 | SEQ ID NO: 332 |
| *Pyrenophora teres f. teres* | E3S5G3_PYRTT | SEQ ID NO: 333 | SEQ ID NO: 334 |
| *Pyrenophora tritici*-*repentis* | B2W5A6_PYRTR | SEQ ID NO: 335 | SEQ ID NO: 336 |
| *Sclerotinia sclerotiorum* | XP_001587300.1 | SEQ ID NO: 337 | SEQ ID NO: 338 |
| *Septoria nodorum* | QOU1Z7_PHANO | SEQ ID NO: 339 | SEQ ID NO: 340 |
| *Setosphaeria turcica (strain 28A)* | R0J317_SETT2 | SEQ ID NO: 341 | SEQ ID NO: 342 |
| *Sphaerulina musiva* | M3B4C9_SPHMS | SEQ ID NO: 343 | SEQ ID NO: 344 |
| *Sporisorium reilianum* | E6ZZ44_SPORE | SEQ ID NO: 345 | SEQ ID NO: 346 |
| *Zymoseptoria brevis* | A0A0F4GYQ8_9PEZI | SEQ ID NO: 347 | SEQ ID NO: 348 |
| *Zymoseptoria tritici* | no reference | SEQ ID NO: 349 | SEQ ID NO: 350 |

**Table 5: The following list indicates the organisms and the accession numbers under which amino acid and nucleotide sequences of GPI1, GPI2, GPI3, GPI15, ERI1, GPI19, GPI14, PBN1, GPI18, MCD4, GPI10, SMP3, GPI13, GPI11, GPI7, GPI16, GPI17, GAB1, and BST1 are to be found. These amino acid and nucleotide sequences are disclosed, in the order of appearance, in the sequence listing under SEQ ID NOs: 351 to 388.**

| Fungal pathogen | Gene | Genbank or NCBI reference sequence | sequence identifier of nucleotide sequence | sequence identifier of amino acid sequence |
|---|---|---|---|---|
| *Colletotrichum graminicola* | GPI1 | EFQ27535.1 | SEQ ID NO: 351 | SEQ ID NO: 352 |
| *Bipolaris maydis* | GPI2 | XP_014083615.1 | SEQ ID NO: 353 | SEQ ID NO: 354 |
| *Magnaporthe oryzae* | GPI3 | XP_003719931.1 | SEQ ID NO: 355 | SEQ ID NO: 356 |
| *Leptosphaeria maculans* | GPI15 | XP_003844705.1 | SEQ ID NO: 357 | SEQ ID NO: 358 |
| *Saccharomyces cerevisiae* | ERI1 | NP_690846.1 | SEQ ID NO: 359 | SEQ ID NO: 360 |
| *Pyrenophora tritici-repentis* | GPI19 | XP_001930950.1 | SEQ ID NO: 361 | SEQ ID NO: 362 |
| *Setosphaeria turcica* | GPI14 | XP_008030968.1 | SEQ ID NO: 363 | SEQ ID NO: 364 |
| *Colletotrichum graminicola* | PBN1 | X_008089048.1 | SEQ ID NO: 365 | SEQ ID NO: 366 |
| *Leptosphaeria maculans* | GPI18 | XP_003837812.1 | SEQ ID NO: 367 | SEQ ID NO: 368 |
| *Zymoseptoria tritici* | MCD4 | XP_003852356.1 | SEQ ID NO: 369 | SEQ ID NO: 370 |
| *Fusarium graminearum* | GPI10 | Q4IB63.1 | SEQ ID NO: 371 | SEQ ID NO: 372 |
| *Bipolaris maydis* | SMP3 | XP_014076431.1 | SEQ ID NO: 373 | SEQ ID NO: 374 |
| *Fusarium oxysporum f. sp. cubense* | GPI13 | EMT70116.1 | SEQ ID NO: 375 | SEQ ID NO: 376 |
| *Puccinia graminis f. sp. tritici* | GPI11 | XP_003322129.2 | SEQ ID NO: 377 | SEQ ID NO: 378 |
| *Colletotrichum gloeosporioides* | GPI7 | XP_007279636.1 | SEQ ID NO: 379 | SEQ ID NO: 380 |
| *Rhizoctonia solani* | GPI16 | EUC66655.1 | SEQ ID NO: 381 | SEQ ID NO: 382 |
| *Rhizoctonia solani* | GPI17 | ELU43284.1 | SEQ ID NO: 383 | SEQ ID NO: 384 |
| *Phytophthora parasitica* | GAB1 | ETP33465.1 | SEQ ID NO: 385 | SEQ ID NO: 386 |
| *Puccinia striiformis f. sp. tritici* | BST1 | KNE97721.1 | SEQ ID NO: 387 | SEQ ID NO: 388 |

Especially preferred, the inhibitory nucleic acid molecule capable of inhibiting the expression of (a) gene(s) involved in the GPI anchor biosynthesis pathway in a fungus inhibits the expression of the gene(s) of the following species: *Alternaria solani*, *Blumeria graminis*, *Botryotinia fuckeliana*, *Cercospora beticola*, *Claviceps purpurea*, *Colletotrichum graminicola, Colletotrichum higginsianum*, *Colletotrichum orbiculare*, *Fusarium fujikuroi*, *Fusarium solani*, *Fusarium oxysporum*, *Gaeumannomyces graminis*, *Magnaporthe oryzae, Puccinia graminis*, *Puccinia triticiana*, *Pyrenophora tritici-repentis, Phaeosphaeria nodorum*, *Sclerotinia sclerotiorum*, *Ustilago maydis*, *Verticillium alfalfae*, and *Verticillium dahlia.*

### Selection of GPI12, GAA1 and GPI8 of Colletotrichum graminicola

The complex GPI anchor biosynthesis pathway is closely associated with the endoplasmic reticulum (ER), with the first steps being catalyzed on the cytoplasmic surface of the ER membrane and the latter, leading to transfer of the final GPI anchor to the C-terminus of GPI-anchored proteins, occurring in the ER lumen. In order to investigate the importance of GPI biosynthesis in the infection process of a plant pathogenic fungus we decided to disrupt this complex pathway of the maize pathogen C. *graminicola* at two distinct early and late steps and attempted to delete the second gene of this pathway, *GPI12* (gene annotation number EFQ25317.1 (SEQ ID NO: 21); (http://www.broadinstitute.org/annotation/genome/colletotrichum_group/MultiHome.html;O'Conne 11, 2012. Lifestyle transitions in plant pathogenic Colletotrichum fungi deciphered by genome and transcriptome analyses. Nat Genet. 2012 Sep; 44(9):1060-5), encoding an N-acetylglucosaminylphosphatidylinositol deacetylase (SEQ ID NO: 22), as well as the *GAA1* (E3QI05_COLGM; SEQ ID NO: 111) and *GPI8* (EFQ31045.1; SEQ ID NO: 215) genes encoding two indispensable sub-units of the GPI transamidase complex catalyzing the final GPI anchor transfer to proteins (SEQ ID NOs: 112 and 216). These genes are single-copy genes in C. *graminicola*, and the derived proteins share significant similarity with the corresponding proteins of other filamentous ascomycota, and the similarity is particularly true across proteins of different *Colletotrichum* species (Fig. 2). As expected, sequences of yeasts and of basidiomycota (Fig. 2) are not as closely related to C. *graminicola* GPI12, GAA1 and GPI8. For example, the amino acid identity of C. *graminicola* GPI12, GAA1 and GPI8 with the corresponding C. *higginsianum* proteins is 82, 92, and 94%. The identity with the *Magnaporthe oryzae* proteins is 61, 65, and 81%, the identity with the S. *cerevisiae* proteins is 30, 31, and 54%, respectively.

In order to delete *GPI12, GAA1,* and *GPI8,* we performed targeted gene deletion experiments, using the hygromycin resistance gene of *Escherichia coli* (Punt P.J. et al.,1987, Transformation of Aspergillus based on the hygromycin B resistance marker from Escherichia coli. Gene.56(1):117-24), flanked by approx 1 Kbp 5'- and 3'-flanking DNA of the gene to be deleted. Among 200 transformants screened for each of the three single-copy genes, no transformants carrying homologous integration of the respective deletion cassette was found, strongly suggesting that *GPI12, GAA1* and *GPI8* are involved genes in C. *graminicola.* Therefore we employed a RNAi strategy previously established to characterize the cell wall β-1,3-glucan biosynthesis gene *GLS1* (Oliveira-Garcia, 2013) (Fig. 3). The sense and corresponding antisense sequences used in RNAi constructs were 493, 560, and 690 bp for *GPI12, GAA1*, and *GPI8*, respectively, and the strong constitutive *trpC* promoter of *Aspergillus nidulans* (Yelton, 1984) was used to control transcription of the RNAi constructs (Fig. 3A) (Oliveira-Garcia, 2013). Transformants harbored one or two copies of the *GPI12*-, *GAA1-* or *GPI8*-RNAi construct (Fig. 3B). Importantly, all transformants tested showed strongly decreases of transcript abundances, ranging from approximately 50 - 90%, as indicated by RT-qPCR analyses (Fig. 3C).

### Reduction of GPI12, GAA1 and GPI8 transcript levels impair vegetative fungal growth

In order to analyze the role of *GPI12, GAA1* and *GPI8* in vegetative development, growth assays were performed with three independent RNAi strains on oat meal agar (OMA) and hyphal morphology was microscopically investigated (Fig. 4A). Interestingly, even the strain with the weakest reduction of transcript abundance (*GPI12*-, *GAA1-* and *GPI8*-RNAi strain 1), showing between 40 and 50%, had a growth reduction by 71, 68 and 64%, respectively (compare Fig. 3C and Fig. 4A). The strains with more pronounced reduction of transcript abundance, i.e. RNAi strains 2 and 3, showed negligible growth, clearly indicating the importance of *GPI12, GAA1* and *GPI8* for hyphal growth. Interestingly, in contrast to vegetative hyphae of the WT strain, which developed long filaments, all RNAi strains tested formed hyperpigmented dark swellings (Fig. 4B). Only occasionally very short filaments growing out of a swelling were observed (Fig. 4B, *GPI8-*RNAi), suggesting that cell walls of filaments were not rigid enough to maintain the hyphal shape and formed swellings when turgor pressure increased. Occasionally large swellings containing intrahyphal hyphae were seen, but hyphae formed within other hyphae were also misshapen (Fig. 4B, *GAA1*-RNAi, asterisk). Interestingly, hyperpigmented swellings were also seen in *GLS1-*RNAi strains. This striking similarity of the phenotypes strongly suggests that GPI anchors play an involved role in β-glucan-dependent cell wall structure.

### Reduction of GPI12, GAA1 and GPI8 transcript levels impair asexual spore formation

Formation of asexual spores is a critical step in fungal propagation. In *Aspergillus nidulans* RNA sequencing revealed altered regulation of approx. 20% of all predicted genes at the shift from vegetative hyphal growth to conidiation, corresponding to more than 2,000 genes. Indeed, previous studies have shown that mutations affecting high-affinity iron uptake caused reduce rates of sporulation and, moreover, affect the conidial shape (Oliveira-Garcia, 2013). As sporulation defects were significant in mutants affecting cell wall rigidity, and as GPI anchored proteins play important roles in cell walls, we studied formation of conidia in *GPI12*-, *GAA1-* and *GPI8*-RNAi strains. Conidiation of *GPI12*-, *GAA1-* and *GPI8*-RNAi strains on OMA required osmotic stabilization (Oliveira-Garcia, 2013). On non-stabilized medium, only the WT strain conidiated (Fig. 5B). On OMA complemented with 0.5 M KCl or 1 M sorbitol, conidia were produced by *GPI12-, GAA1-* and *GPI8*-RNAi strains, but the rate of conidiation was significantly lower than that of the WT strain. Importantly, RNAi strains showing strongly reduced transcript abundance, i.e. *GPI12*-RNAi strains 4 and 5, *GAA1*-RNAi strains 5, 6 and 7, and *GPI8*-RNAi strains 4, 5 and 6 failed to sporulate completely (compare Figs. 3C and 5B). Apparently, sporulation rates and transcript abundance correlate. Quantification of transcript abundance and rates of conidiation suggests that suppression of transcript abundance to more than 90% in the case of *N-*acetylglucosaminylphosphatidylinositol deacetylase gene *GPI12* and 70% in the case of the GPI transamidase complex genes *GAA1* and *GPI8* is required for elimination of asexual sporulation, even when media are osmotically stabilized.

On oat meal agar supplemented with 1 M sorbitol the WT strain formed falcate conidia (Fig. 5A, WT). In comparison, the *GPI12*-, *GAA1-* and *GPI8*-RNAi strain formed shorter spores with increased diameters, leading to an oval to bean-like phenotype (Fig. 5A, *GPI12*-, *GAA1-* and *GPI8-*RNAi, compare with WT). This bean-like swollen phenotype may be well explained by reduced cell wall rigidity of conidia formed by *GPI12-, GAA1-* and *GPI8*-RNAi strains.

Reduction of GPI12, GAA1 and GPI8 transcript levels impair formation of fungal infection

### structures

As GPI anchors are thought to play a role in cross-connecting cell wall β-glucan polymers and thus presumably also in cell wall rigidity, we investigated rates of appressorium differentiation and appressorial integrity in two independent *GPI12*-, *GAA1-* and *GPI8*-RNAi strains (Fig. 5). In these experiments we used strains with moderately reduced transcript abundance, corresponding to approx. 40 to 60%. In all RNAi strains reduced transcript abundance correlated with reduced germination rates, and while the vast majority of the germ tubes formed by the WT strain differentiated an appressorium, appressorium formation rates were severely reduced in *GPI12*-, *GAA1-* and *GPI8*-RNAi strains (Fig. 5D). Appressoria of these strains, like those formed by the WT, were strongly melanized (Fig. 5C, arrowheads). Importantly, infection cells of all RNAi strains, when allowed to form on artificial polypropylene surfaces, exploded spontaneously at high frequency (Fig. 6C, arrows). Again, these observations highlight the importance of GPI anchor biosynthesis in cell wall rigidity and infection cell functionality.

### Reduction of GPI12, GAA1 and GPI8 transcript levels results in reduced infection rates

In order to analyze pathogenicity or virulence defects resulting from appressorial penetration defects/failure, we inoculated conidia of *GPI12*-, *GAA1-* and *GPI8*-RNAi strains onto intact maize leaf surfaces. As appressoria of these strains exploded on artificial surfaces, we anticipated that the *GPI12*-, *GAA1-* and *GPI8*-RNAi strains would have problems to invade the host tissue. Indeed, independent RNAi strains were unable to invade the plant and to cause anthracnose disease symptoms. The WT strain had full penetration competence and severe anthracnose disease symptoms were seen 5 days after inoculation (Fig. 6A, non-wounded). On wounded leaves, minor necrotization of the tissue was observed at the margins of the wounds, but as compared with the disease symptoms caused by the WT strain, the symptoms caused by the mutants were minor (Fig. 6A, wounded). Quantification of fungal DNA in the inoculated tissue by qPCR fully supported the macroscopical observations and clearly indicated that the RNAi strains were unable to invade intact plants (Fig. 6B). Furthermore, qPCR analyses showed that reduction of transcript abundance of all three genes investigated correlated with the ability of C. *graminicola* mutants to develop on wounded leaves and thus with virulence (compare Figs. 3C and 7B). Microscopy showed that the WT strain inoculated onto wounded leaves extensively colonized its host leave and formed thin, fast growing secondary hyphae indicative of a necrotrophic lifestyle (Fig. 6C, WT, arrowhead), and these hyphae frequently breached plant cell walls (Fig. 6C, WT, arrow). In contrast, all RNAi strains tested showed severely distorted infectious hyphae, exhibiting large swellings (Fig. 6C, RNAi strains, asterisks) connected by short stretches of non-swollen hyphae ((Fig. 6C, RNAi strains, arrowhead). Randomly occurring swellings suggest that the cell wall rigidity of all RNAi strains is insufficient to maintain the hyphal shape in pathogenic hyphae.

### Enhanced early blight resistance of HIGS _{GPI8} potato lines

In order to test the suitability of GPI anchor biosynthesis genes for the improvement of fungal resistance of crops a fragment of the GPI8 gene of the potato pathogen *Alternaria solani* was selected. Based on homology search with the yeast GPI8 gene the GPI8 gene was identified in a proprietary KWS data bank of the *Alternaria solani* genomic sequence.

The GPI8 fragment was used for the construction of an RNAi-hairpin construct pRNAi-HIGS-AsGpi8 (Fig. 8). The HindIII-HindIII fragment of this construct was cloned into the binary vector p95N which was transformed by Agrobacterium timefaciens mediated transformation into the potato genotype Hermes. As a control an RNAi-hairpin construct against the GFP protein was generated and also transformed into potato.

The transgenic HIGS_{GPI8} potato lines were in-vitro multiplied and tested under near-field like conditions for A. *solani* (early blight) resistance. The line PR-H-36-T-017 showed enhanced early blight resistance compared to the non-transgenic Hermes and the HIGS _{GFP} potato line. The transcriptional silencing activity of the HIGS _{GPI8} potato lines were determined by biolistic transfer of a reporter gene construct which contains a fusion between the HIGS target gene and the luciferase gene. The transcriptional silencing activity of line PR-H-36-T-017 was the highest in comparison to other HIGS _{GPI8} potato lines (Fig. 9). This result showed that the silencing activity correlates with the early blight resistance and might be used to screen for plants with enhanced fungal resistance.

Under near field conditions at field trials in Germany an enhanced early blight resistance of the HIGS potato line PR-H-36-T-017 has been shown (Fig. 14). The transformation genotype Hermes and the transgenic control, which was transformed with an RNAi construct against the green fluorescence protein (GFP) showed a destruction of the plants by 80 and 87%, respectively. The HIGS_{GPI8} lines PR-H-36-T-002 and PR-H-36-T-017 showed only a plant damage of 70 and 58%.

### The GWT1 gene is important for the vegetative growth of Fusarium graminearum as shown by the chemical induction of a RNAi _{GWT1}

The doxycycline (Dox) inducible tetO promoter was applied to analyze the relevance of the GWT1 gene for the vegetative growth of Fusarium graminearum (Fig. 7A). An RNAi _{GWT1} construct under the control of the tetO promoter was transformed into *F. graminearum.* In the absence of Dox the growth of the transformants 1280_13, 1280_17 and 1280_5 was identical to the growth of the wild type PH-1. In the presence of Dox the transformants 1280_13, 1280_17 and 1280_5 showed growth retardation (Fig. 7B) which showed that the reduction of GWT1 expression impairs the fungal growth.

### Enhanced Fusarium head scab resistance of HIGS_{GWT1} wheat lines

In order to show the suitability of another GPI anchor protein biosynthesis gene for the improvement of Fusarium resistance of wheat a fragment of the GWT1 gene of the wheat and corn pathogen *Fusarium graminearum* was selected. Based on homology search the GWT1 gene was identified in a public data bank of *Fusarium graminearum* genomic sequences. The GWT1 gene fragment was used for the construction of a double promoter RNAi-construct which was transformed by Agrobacterium mediated transformation into the wheat genotype Taifun. In the double promoter construct the HIGS target gene is flanked by two 35S promotors in a forward and a reverse orientation which will result in the biosynthesis of a sense and an antisense transcript (Fig. 10). Sense and antisense transcript will hybridize to a double stranded RNA molecule which is the target for the siRNA generating enzymes.

Homozygous and heterozygous plants of the segregating T1 generation were identified by PCR and the transgenic plants were tested for Fusarium graminearum resistance in the greenhouse. The heads of the whole plants were inoculated with Fusarium spores (25.000 spores/ml). The spread of disease was rated six times at an interval of 2-4 days, using a percentage scale of 0-100 with increments of 1, where 0 indicates a healthy head and 100 indicates a completely bleached head.

The six disease score values were used for the calculation of an "area under disease progress curve" (AUDPC). The HIGS _{GWT1} wheat line WA336-T-018 showed reduced visual Fusarium head scab symptoms in comparison to the Taifun genotype as shown by a reduced AUDPC value (Fig. 11). The determination of the mycotoxin DON by ELISA revealed also a reduction of the toxin in the heads of the HIGS line. The determination of the fungal biomass in the infected heads by qPCR revealed a 40 % reduction of Fusarium biomass in the heads of the HIGS_{GWT1} wheat line WA336-T-018 in comparison to Taifun (Fig.12). In summary the HIGS _{GWT1} wheat line showed a reduction of visual symptoms, fungal biomass and DON mycotoxin comparable or even better as to the cultivar Aurum. Aurum was the wheat cultivar with the best Fusarium resistance available in Germany. Since Aurum has some negative agronomical properties (yellow flour), the cultivation is very limited.

A field trial with a random plot design was performed at two locations in Minnesota (USA) to determine the fungal resistance under field conditions. Plots of wheat plants were spray inoculated with a mixture of 8 Fusarium isolates from the USA and a visual disease scoring was done (1-9, 1=healthy, 9 =100% bleached heads). The HIGS_{GWT1} wheat line WA336-T-018 showed a disease score of 2.1 in comparison to a value of 2.9 for the genotype Taifun and the mean of transgenic controls in the Taifun background. Conventional reference lines from the USA (Briggs, Steele, Glenn) and Germany (Scirocco, Tybalt) were included into the trials. The transgenic HIGS line performed superior compared to the reference lines and showed especially a significant higher resistance as Glenn, which is recommended to the US farmers as partial Fusarium tolerant variety (Fig. 13).

Taken together, the data shown here suggest that GPI anchor biosynthesis is required for vegetative hyphal growth, conidiation, differentiation of functional appressoria, and development of invasive pathogenic hyphae.

## Claims

1. A transgenic plant or a part thereof comprising as transgene a DNA capable of expressing an inhibitory nucleic acid molecule capable of inhibiting the expression of a gene involved in the glycosylphosphatidylinositol (GPI) anchor biosynthesis pathway in a fungus.

2. The transgenic plant or the part thereof of claim 1, wherein the gene comprises the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), preferably the GPI12, GAA1, GPI8 and/or GWT1 gene(s).

3. The transgenic plant or the part thereof of claim 1 or 2 comprising as transgene an expression cassette comprising the DNA.

4. The transgenic plant or the part thereof of any one of claims 1 to 3, wherein the DNA or the expression cassette is stably integrated into the genome of the plant or present in the plant or the part thereof on a vector.

5. The transgenic plant or the part thereof of any one of claims 1 to 4, wherein the inhibitory nucleic acid molecule is an antisense RNA or dsRNA, whereby the dsRNA is preferably hpRNA, siRNA or miRNA.

6. The transgenic plant or the part thereof of any one of claims 1 to 5, wherein the DNA encodes an RNA molecule in sense direction and an RNA molecule in antisense direction, wherein the RNA molecule in sense direction or the RNA molecule in antisense direction are substantially complementary to the gene involved in the GPI anchor biosynthesis pathway or a part thereof, the RNA molecule in antisense direction is substantially reverse complementary to the RNA molecule in sense direction, and the RNA molecule in sense direction and the RNA molecule in antisense direction are able to build a dsRNA.

7. The transgenic plant or the part thereof of claim 5 or 6, wherein the length of the antisense RNA, the dsRNA, the RNA molecule in sense direction or the RNA molecule in antisense direction is at least 15, 16, 17, 18, 19 or 20 contiguous nucleotides, preferably at least 21, 22, 23, 24, 25, 30, 40, 50, 60, 70, 80, 90 or 100 contiguous nucleotides, more preferably at least 150, 200, 250, 300, 350, 450, 500, 600, 700, 800, 900 or 1000 contiguous nucleotides, still more preferably 300-1700, 300-1000, or 300-700, and most preferably 493 contiguous nucleotides for the GPI12 gene, 560 contiguous nucleotides for the GAA1 gene, or 690 contiguous nucleotides for the GPI8 gene.

8. The transgenic plant or the part thereof of any one of claims 1 to 7, wherein the expression of the inhibitory nucleic acid molecule is controlled by a promoter, preferably an inducible promoter, more preferably a pathogen inducible and/or tissue specific promoter.

9. A DNA capable of expressing an inhibitory nucleic acid molecule capable of inhibiting the expression of a gene involved in the GPI anchor biosynthesis pathway in a fungus, preferably as defined in any one of claims 2 to 8, or an expression cassette comprising the DNA.

10. An inhibitory nucleic acid molecule capable of inhibiting the expression of a gene involved in the GPI anchor biosynthesis pathway in a fungus, preferably as defined in any one of claims 5 to 7.

11. A vector comprising the DNA or the expression cassette of claim 9.

12. A method of producing the transgenic plant or the part thereof of any one of claims 1 to 8, comprising the following steps:
introducing into at least a cell of the plant the DNA or the expression cassette of claim 9 or the vector of claim 11 or a dsRNA capable of inhibiting the expression of a gene involved in the GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus, and
regenerating the transgenic plant from the at least a cell.

13. A method of conferring fungal resistance to a plant or the part thereof comprising the following steps:
introducing into the plant or the part thereof the DNA or the expression cassette of claim 9 or the vector of claim 11 or a dsRNA capable of inhibiting the expression of a gene involved in the GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus, and
causing expression of the DNA, the expression cassette, the vector, or the dsRNA.

14. A method of inhibiting the expression of a gene involved in the GPI anchor biosynthesis pathway, comprising:
applying the DNA or the expression cassette of claim 9 or the vector of claim 11 or a dsRNA capable of inhibiting the expression of a gene involved in theGPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus to the fungus or to a plant or a part thereof.

15. Use of the DNA or the expression cassette of claim 9 or the vector of claim 11 or a dsRNA capable of inhibiting the expression of a gene involved in GPI anchor biosynthesis pathway, preferably a dsRNA capable of inhibiting the expression of the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably a dsRNA capable of inhibiting the expression of the GPI12, GAA1, GPI8 and/or GWT1 gene(s), in a fungus
for inactivating a fungus, while contacting a plant or a part thereof;
for protecting a plant against an infection by a fungus; or
for inhibiting the expression of a gene involved in the GPI anchor biosynthesis pathway, preferably the GPI1, GPI2, GPI3, GPI15, ERI1, GPI19, GPI12, GWT1, GPI14, PBN1, GPI18, MCD4, GPI10, SMP3, GPI13, GPI11, GPI7, GAA1, GPI8, GPI16, GPI17, GAB1, and/or BST1 gene(s), more preferably the GPI12, GAA1, GPI8 and/or GWT1 gene(s), in a fungus.
